# EUROPEAN PATENT SPECIFICATION

(11) **EP 3 383 996 B1**
(45) Date of publication and mention of the grant of the patent: **16.10.2024**
(21) Application number: 16871591.0
(22) Date of filing: 02.12.2016
(51) Int. Cl.: C12M 3/00, C12N 5/22, G01N 33/483, G01N 33/50, B01L 99/00, B81B 7/00

(54) **DEVICES FOR SIMULATING A FUNCTION OF A LIVER TISSUE AND METHODS OF USE AND MANUFACTURING THEREOF**
VORRICHTUNGEN ZUR SIMULATION EINER FUNKTION EINER LEBER SOWIE VERFAHREN ZUR VERWENDUNG UND HERSTELLUNG DAVON
DISPOSITIFS DE SIMULATION D'UNE FONCTION D'UN TISSU HÉPATIQUE ET LEURS PROCÉDÉS D'UTILISATION ET DE FABRICATION

(30) Priority: 04.12.2015 US 201562263378 P
(43) Date of publication of application: 10.10.2018
(73) Proprietor: President and Fellows of Harvard College, Cambridge, MA 02138 (US)
(72) Inventor: INGBER, Donald, E., Boston, Massachusetts 02118 (US); HAMILTON, Geraldine, Cambridge, MA 02138 (US); JANG, Kyung-Jin, Cambridge, MA 02138 (US); HANEY, Suzzette, Cambridge, Massachusetts 02138 (US); PATEL, Payal, Cambridge, MA 02138 (US); HERLAND, Anna, 583 36 Linkoping (SE); RESNIKOFF, Joshua, Somerville, Massachusetts 02143 (US)
(74) Representative: Haseltine Lake Kempner LLP
(86) International application number: PCT/US2016/064661
(87) International publication number: WO 2017/096192

(56) References cited:
- WO-A1-2012/032646
- WO-A2-2010/009307
- WO-A2-99/47922
- US-A1- 2008 220 516
- US-A1- 2013 309 677
- US-A1- 2013 309 677
- US-A1- 2014 342 445
- US-B2- 8 968 543
- RENNERT, K ET AL.: "A microfluidically perfused three dimensional human liver model.", BIOMATERIALS, vol. 71, 25 August 2015 (2015-08-25), pages 119 - 131, XP029269412
- BALE, SS ET AL.: "A novel low-volume two-chamber microfabricated platform for evaluating drug 1 metabolism and toxicity.", TECHNOLOGY (SINGAP WORLD SCI)., vol. 3, no. 4, 21 April 2015 (2015-04-21), pages 3, 11, XP055387364

## Description

### FIELD OF INVENTION

Various aspects described herein relate generally to microfluidic devices and methods of use and manufacturing thereof. In some embodiments, the microfluidic devices can be used for culture and/or support of living cells such as mammalian cells, and/or for simulating a function of a tissue, *e.g*., a liver tissue. Endothelial cell culture media for long-term culture of endothelial cells are also provided herein.

### BACKGROUND

Conventional *in vitro* hepatic model systems, *e.g.*, 2-dimensional monolayers of primary hepatocytes, are limited by their inability to maintain normal phenotypic characteristics over time in culture, including stable expression of clearance and metabolic pathways (Phase I and Phase II metabolism). In addition, one of the main drawbacks of the existing cell culture system is its limitation in maintaining a long-term viability of the cells. WO 2012/032646 A1 provides for a cell culture device and a cell culture method.

### SUMMARY

The present invention is as defined in the appended claims. The present invention provides for a method of culturing cells, comprising: a) providing a microfluidic device comprising a membrane, said membrane comprising a first surface and a second surface; b) seeding viable human hepatocytes on said first surface and viable human liver sinusoidal endothelial cells on said second surface; and c) culturing said seeded cells under flow conditions such that said cells remain viable for at least 14 days, wherein, prior to step b), said first surface of said membrane is coated with a layer of fibronectin, collagen I, and collagen IV.

Aspects described herein stem from, at least in part, design of devices that allow for a controlled and physiologically realistic co-culture of liver sinusoidal endothelial cells in one chamber with hepatocytes in other chamber(s) to establish hepatic function *in vitro.* In one embodiment, the chambers are aligned (*e.g*., vertically) with each other with one or more membranes separating them from each other ("liver-on-a-chip"). The liver-on-a-chip devices have been developed and optimized based on the basic design of an organ-on-a-chip as described in the U.S. Patent No. 8,647,861, and the International Patent App. No. PCT/US2014/071611. In some aspects, the inventors have optimized the design of the liver-on-a chip devices and culture conditions to provide long-term hepatic culture with physiologically relevant hepatic function (*e.g*., albumin and/or urea secretion, and/or CYP 450 metabolic capacity) for a human model. Described herein, for reference, are also devices and culture conditions for different animal models, e.g., rats and dogs.. Further, in some aspects, the inventors have formulated a beneficial endothelial cell culture medium for long-term culture, which is superior to the existing commercially available media. The novel endothelial cell culture media developed by the inventors allow co-cultures of endothelial and epithelial cells of liver to maintain functionality and viability for at least one month in a cell culture device, which is not at all possible using the commercial culture media. Described herein, for reference, is also novel endothelial cell culture media developed by the inventors to allow co-cultures of endothelial and epithelial cells of different tissue origins (*e.g*., lung, gut, kidney). Accordingly, embodiments of various aspects described herein relate to devices for simulating a function of a tissue and methods of using the same. Novel endothelial cell culture media for long-term culture and methods of using the same are also described herein.

Some aspects described herein relate to devices for simulating a function of a tissue. In one aspect, the device generally comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. In one aspect, the device generally comprises (i) a first structure defining a first chamber; (ii)a second structure defining a second chamber; and (iii) a membrane located at an interface region between the first chamber and the second chamber, the membrane including a first side facing toward the first chamber and a second side facing toward the second chamber, the first side comprising an extracellular matrix composition and hepatocytes, the second side comprising endothelial cells.

The first side of the membrane has an extracellular matrix composition disposed thereon, wherein the extracellular matrix (ECM) composition comprises an ECM coating layer and an ECM overlay composition over the ECM coating layer. The ECM coating layer can comprise fibronectin andr collagen I and collagen IV. The ECM overlay composition can comprise a protein mixture representing an extracellular microenvironment and optionally fibronectin. In some embodiments, the ECM overlay composition can form a gel layer. In some embodiments, the ECM overlay composition can be prepared in a concentration that is below gelling concentration of the respective ECM molecules.

In some embodiments, the ECM coating layer can further comprise laminin.

In some embodiments, the device can further comprise a monolayer of viable hepatocytes adhering on the ECM coating layer and overlaid by the ECM overlay composition. Culturing hepatocytes between the ECM coating layer and the ECM overlay composition can induce proper polarization in hepatocytes. Other cell types (e.g., non-parenchymal liver cells) including, but not limited to cholangiocytes (biliary endothelial cells) and/or liver fibroblasts, that are generally present in a liver in vivo can also be included between the ECM coating layer and the ECM overlay composition. The various cell types are derived from human. Also described herein are cell types derived from different mammalian sources, including, *e.g*., but not limited to rats, mice, and dogs. Thus, the device can be used to simulate a function of a liver tissue in different animal model of interest.

In some embodiments, said extracellular matrix composition comprises collagen. In some embodiments, said hepatocytes are adhered on the extracellular matrix composition. In some embodiments, the extracellular matrix comprises an overlay above said hepatocytes. In some embodiments, said overlay is a gel overlay. In some embodiments, said gel overlay comprises Matrigel. In some embodiments, said overlay is a coating.

In some embodiments, said first chamber has a height that is greater than said second chamber. In some embodiments, the height of said second chamber is 100 microns and the height of said first chamber is 200 microns or greater. In some embodiments, the first chamber is in fluidic communication with a fluidic channel. In some embodiments, the second chamber is in fluidic communication with a fluidic channel. In some embodiments, said endothelial cells are in contact with said second side of said membrane.

In some embodiments, the device can further comprise a monolayer of viable hepatocytes adhered on a coating or a surface comprising a first extracellular matrix composition. The viable hepatocytes can be derived from human. Also described herein, for reference, are viable hepatocytes from different mammalian sources, including, e.g., but not limited to rats, mice, and dogs. For human cells, a first extracellular matrix composition comprising collagen I coating produced good results. In the case of rat hepatocytes, a fibronectin mixture was found to work. In some embodiments, the hepatocytes can be further overlayed with a coating comprising a second extracellular matrix composition. For human cells, a Matrigel overlay (with no fibronectin) produced good results. In the case of rat hepatocytes, a fibronectin mixture was found to work. Thus, the device can be used to simulate a function of a liver tissue in different animal model of interest.

In some embodiments where human are used, the ECM overlay composition can comprise, essentially consist of, or consist of a protein mixture representing an extracellular microenvironment (e.g., MATRIGEL^{™}).

Also described herein, for reference, where dog hepatocytes are used the ECM overlay composition can comprise, a protein mixture representing an extracellular microenvironment (e.g., MATRIGEL^{™}). Also described herein for reference, where rat hepatocytes are used, the ECM overlay composition can comprise, essentially consist of, or consist of a protein mixture representing an extracellular microenvironment (e.g., MATRIGEL^{™}) and fibronectin.

In some embodiments, the device further comprises Kupffer cells. In some embodiments, the Kupffer cells are disposed in said second chamber. In some embodiments, the device further comprises Stellate cells. In some embodiments, said Stellate cells are disposed in said first chamber. In some embodiments, said Stellate cells are disposed in said second chamber.

In one aspect, described herein is a microfluidic device, comprising: a first structure defining a first microfluidic chamber having a height; a second structure defining a second microfluidic chamber having a height, wherein the height of the first chamber is greater than the height of the second chamber; and a membrane located at an interface region between the first chamber and the second chamber, the membrane including a first side facing toward the first chamber and a second side facing toward the second chamber, the first side comprising hepatocytes, the second side comprising endothelial cells.

In some embodiments, the height of said second chamber is 100 microns and the height of said first chamber is 200 microns or greater. In some embodiments, the endothelial cells comprise liver sinusoidal endothelial cells. In some embodiments, said hepatocytes are human hepatocytes. In some embodiments, the device further comprises a layer above said hepatocytes. In some embodiments, said layer is a protein layer. In some embodiments, said protein layer comprises a gel. In some embodiments, said protein layer comprises Matrigel. Also disclosed herein, for reference, are hepatocytes selected from the group consisting of dog hepatocytes or rat hepatocytes.

In some embodiments, the first microfluidic chamber is in fluidic communication with a microfluidic channel. In some embodiments, said second microfluidic chamber is in fluidic communication with a microfluidic channel.

In some embodiments, the device further comprises Kupffer cells. In some embodiments, said Kupffer cells are disposed in said second chamber. In some embodiments, the device further comprises Stellate cells. In some embodiments, said Stellate cells are disposed in said first chamber. In some embodiments, said Stellate cells are disposed in said second chamber.

In one aspect, described herein is a method for culturing cells, comprising: a) providing a fluidic device comprising a first structure defining a top chamber, a second structure defining a bottom chamber, and a membrane located at an interface region between the top chamber and the bottom chamber, the membrane including a top side facing toward the top chamber and a bottom side facing toward the bottom chamber; b) seeding hepatocytes in said top chamber and endothelial cells in said bottom chamber; and c) perfusing at least one of the top chamber or bottom chamber.

In some embodiments, said seeding of hepatocytes comprises seeding said hepatocytes on said top surface of said membrane. In some embodiments, said seeding of endothelial cells comprises seeding said endothelial cells on said bottom surface of said membrane. In some embodiments, said hepatocytes are human hepatocytes. In some embodiments, said endothelial cells comprise liver sinusoidal endothelial cells. Also described herein, for reference, are hepatocytes selected from the group consisting of dog hepatocytes and rat hepatocytes.

In some embodiments, the method further comprises plasma treating at least a portion of said fluidic device. In some embodiments, the method further comprises coating at least a region of said membrane with at least one extracellular matrix protein. In some embodiments, the method further comprises overlaying said hepatocytes with a protein overlay. In some embodiments, said protein overlay comprises a gel overlay. In some embodiments, said protein overlay comprises extracellular matrix proteins. In some embodiments, said protein overlay comprises Matrigel. In some embodiments, said protein overlay is adapted to form a gel.

In some embodiments, the height of said top chamber is greater than the height of said bottom chamber. In some embodiments, the height of said second channel is 100 microns and the height of said first channel is 200 microns or greater. In some embodiments, said perfusing generates a shear force of less than 0.1 dyne/cm² in said top chamber. In some embodiments, the top chamber is in fluidic communication with a fluidic channel. In some embodiments, the bottom chamber is in fluidic communication with a fluidic channel.

In some embodiments, the method further comprises seeding Kupffer Cells. In some embodiments, said Kupffer Cells are seeded in said bottom chamber. In some embodiments, said Kuppfer Cells are co-seeded with said endothelial cells. In some embodiments, the method further comprises seeding Stellate Cells. In some embodiments, said Stellate Cells are seeded in said top chamber. In some embodiments, said Stellate Cells are seeded in said. In some embodiments, seeded cells remain viable for at least 14 days.

In some embodiments, the method further comprises assessing the level of activity of one or more cellular enzymes. In some embodiments, said cellular enzymes is a CYP450 enzyme. In some embodiments, said assessing the level of activity comprises contacting said hepatocytes with an agent, and measuring one or both of the rate of production of a metabolite and the rate of disappearance of said agent. In some embodiments, the method further comprises d) measuring the level of one or more secreted factors. In some embodiments, said secreted factor is a transaminase. In some embodiments, said secreted factor is a lactose dehydrogenase. In some embodiments, said secreted factor is a cytokine. In some embodiments, said secreted factor is selected from the group consisting of albumin and urea. In some embodiments, the method further comprises d) assessing the amount of one or more cellular proteins. In some embodiments, the method further comprises d) measuring the RNA expression level of one or more RNA species. In some embodiments, the method further comprises d) assessing the level of one or more cellular proteins. In some embodiments, said cellular protein is albumin. In some embodiments, prior to step d), said seeded viable human hepatocytes are exposed to an agent.. In some embodiments, said agent is a drug candidate. In some embodiments, said flow conditions comprise perfusing said cells with media. Also described herein, for reference, is where prior to step d), said seeded viable dog hepatocytes are exposed to an agent. Also described herein, for reference, is where prior to step d), said seeded viable rat hepatocytes are exposed to an agent

In one aspect, described herein is a method of culturing cells, comprising: a) providing a microfluidic device comprising a membrane, said membrane comprising a top surface and a bottom surface; b) seeding viable hepatocytes on said top surface and viable liver sinusoidal endothelial cells on said bottom surface; c) culturing said seeded cells under flow conditions with a fluid such that said cells remain viable; and d) disposing a test compound into the fluid. In some embodiments, said viable hepatocytes are human hepatocytes. Also described herein, for reference, is where said viable hepatocytes are selected from dog hepatocytes and rat hepatocytes. In some embodiments, said hepatocytes are cultured under a gel overlay. In some embodiments, said gel overlay comprises extracellular matrix proteins. In some embodiments, said gel overlay comprises Matrigel. In some embodiments, said membrane is positioned between a first microfluidic channel having a height and a second microfluidic chamber having a height, wherein the height of the first chamber is greater than the height of the second chamber. In some embodiments, the height of said second channel is 100 microns and the height of said first channel is 200 microns or greater. In some embodiments, wherein at least a portion of said microfluidic device is plasma treated. In some embodiments, the method further comprises e) assessing the toxicity of said test compound. In some embodiments, the method further comprises e) assessing the clearance of said test compound. In some embodiments, said assessing the clearance comprises measuring the disappearance of said test compound. In some embodiments, the method further comprises e) assessing the induction or inhibition of liver enzymes by said test compound. In some embodiments, the method further comprises e) assessing metabolites from said test compound. In some embodiments, said assessing of metabolites is done by mass spectroscopy.

Another aspect described herein relates to a device for simulating a function of a liver tissue. The device comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side comprises an extracellular matrix composition and hepatocytes adhered on the extracellular matrix composition. In some embodiments, the extracellular matrix composition comprises fibronectin, collagen I and collagen IV.

In some embodiments, the first side can further comprise one or more of other cell types (e.g., non-parenchymal liver cells) including, but not limited to cholangiocytes (biliary endothelial cells) and/or liver fibroblasts, that are generally present in a liver in vivo.

In some embodiments of this aspect and other aspects described herein, the second side of the membrane can comprise a monolayer of liver sinusoidal endothelial cells adhered thereon. In some embodiments of this aspect and other aspects described herein, the second side of the membrane can comprise macrophagic kupffer cells, hepatic stellate cells, and/or liver fibroblasts.

It is not aware that there are any commercially available *in vitro* co-culture models of dog hepatocytes and dog liver sinusoidal endothelial cells. The inventors have successfully created a co-culture microfluidic device to simulate a function of a dog liver tissue. Accordingly, also described herein for reference is a device for simulating a function of a dog liver tissue. The device comprises: (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side of the membrane has dog hepatocytes adhered thereon and the second side has dog liver sinusoidal endothelial cells adhered thereon.

In some embodiments of various aspects described herein, the devices can comprise a flowing culture medium in the first chamber, wherein the flowing culture medium generates a shear stress that simulates physiological or pathological shear stress applied to cells cultured in the first chamber. In some embodiments, the flowing culture medium can generate a shear stress of no more than 0.05 dyne/cm². In some embodiments, the flowing culture medium can generate a shear stress of no more than 0.02 dyne/cm². In some embodiments, the flowing culture medium can generate a shear stress that is greater than 0.05 dyne/cm², e.g., up to about 5 dynes/cm². The inventors have discovered that addition of low shear stress improved liver cell morphology and long-term viability over static cultures.

In some embodiments of various aspects described herein, the hepatocytes growing in the devices described herein can display at least one or a combination of two or more of the following characteristics: (i) cuboidal morphology; (ii) formation of a bile canaliculus network; (iii) albumin secretion over a period of time; (iv) the presence of drug-metabolism function of at least one CYP450 enzyme and/or Phase II enzyme sustained over a period of time; (v) at least a 2-fold increase in activity of CYP450 drug metabolizing enzyme in the presence of an agent that induces CYP450 drug metabolizing enzyme; (vi) secretion of urea; (vii) level of alanine transaminase (ALT) activity and/or expression; (viii) level of aspartate transaminase (AST) activity and/or expression; and (ix) maintenance of glutathione level.

In some embodiments of various aspects described herein, the hepatocytes growing in the devices described herein can display at least one or a combination of two or more of the following characteristics: (i) cuboidal morphology; (ii) formation of a bile canaliculus network; (iii) albumin secretion over a period of time; (iv) the presence of drug-metabolism function of at least one CYP450 enzyme and/or Phase II enzyme sustained over a period of time; and (iv) at least a 2-fold increase in activity of CYP450 drug metabolizing enzyme in the presence of an agent that induces CYP450 drug metabolizing enzyme.

The heights of the first chamber and the second chamber can vary to suit the needs of desired applications (e.g., to provide a low shear stress, and/or to accommodate cell size). The first chamber and the second chamber can have the same height or different heights. In some embodiments, a height ratio of the first chamber to the second chamber can range from 1:1 to about 20:1. In some embodiments, a height ratio of the first chamber to the second chamber can range from 1:1 to about 10:1.

In some embodiments, the height of the first chamber can range from about 100 µm to about 50 mm, or about 100 µm to about 5 mm. In one embodiment, the height of the first chamber can be about 150 µm. In one embodiment, the height of the first chamber can be about 1 mm.

In some embodiments, the height of the second chamber can range from about 20 µm to about 1 mm, or about 50 µm to about 500 µm. In one embodiment, the height of the second chamber can be about 150 µm. In one embodiment, the height of the second chamber can be about 100 µm.

The width of the first chamber and/or the second chamber can vary with desired cell growth surface area. In some embodiments, the width of the first chamber and/or the second chamber can range from about 100 µm to about 200 mm, or about 100 µm to about 50 mm, or about 100 µm to about 5 mm. In one embodiment, the width of the first chamber and/or the second chamber can be about 1 mm.

The membrane separating the first chamber and the second chamber in some embodiments of the devices described herein can be rigid or at least partially flexible. In some embodiments, the membrane can be rigid. In some embodiments, at least a portion of the membrane where the cells are cultured thereon can be flexible. In some embodiments, the membrane can be configured to deform in a manner (*e.g*., stretching, retracting, compressing, twisting and/or waving) that simulates a physiological strain experienced by the cells in its native microenvironment. In these embodiments, the membrane can be at least partially flexible.

The membrane can be of any thickness. In some embodiments, the membrane can have a thickness of about 1 µm to about 300 µm, or about 1 µm to about 100 µm, or about 100 nm to about 50 µm. In some embodiments, the membrane can have a thickness of about 10 µm to about 80 µm. In one embodiment, the devices described herein (e.g., a liver-on-a-chip) can have a 50 µm thick membrane.

The membrane can be non-porous or porous. In some embodiments where at least a portion of the membrane is porous, the pores can have a diameter of about 0.1 µm to about 15 µm. In one embodiment, the pores can have a diameter of about 7 µm.

While the first chamber and the second chamber can be in any geometry or three-dimensional structure, in some embodiments, the first chamber and the second chamber can be configured to be form channels.

Not only have the inventors created a device for simulating a function of a liver tissue, the inventors have also developed a beneficial endothelial cell culture medium for long term culture of endothelial cells. In some instances, the endothelial cell culture medium can be used for co-culture or even long-term co-culture of endothelial cells and epithelial cells from different tissue origins. Accordingly, in some aspects, described herein are methods of maintaining long-term viability of endothelial cells. In one aspect, a method of maintaining long-term viability of endothelial cells comprises contacting a population of endothelial cells with an endothelial cell culture medium comprising: (a) a basal endothelial medium; and (b) a supplement cocktail essentially consisting of hEGF, hydrocortisone, VEGF, hFGF-beta, R3-IGF-1, ascorbic acid, heparin, and serum, and the concentration of FBS is about 0.3% to about 1%. Using the endothelial cell culture medium, the endothelial cells can be maintained as a viable monolayer for at least two weeks or longer.

In another aspect, a method of maintaining long-term viability of endothelial cells comprises contacting a population of endothelial cells with an endothelial cell culture medium comprising: (a) a basal endothelial medium; and (b) a supplement cocktail essentially consisting of Vitamin C (50ug/mL), insulin, transferrin, selenium, dexamethasone (1uM), glutamax(1%) and pen-strep (1%), and the concentration of FBS is about 0.3% to about 10%. Using the endothelial cell culture medium, the endothelial cells can be maintained as a viable monolayer for at least two weeks or longer. In some embodiments, the basal endothelial medium can be a mixture of DMEM and F 12.

In some embodiments, the endothelial cell culture medium can further comprise glutamine and/or GlutaMAX^{™} dipeptide.

An endothelial cell culture medium that provides longer viability of endothelial cells than existing media is also described herein. The endothelial cell culture medium comprises (a) a basal medium; and (b) a supplement cocktail essentially consisting of hEGF, hydrocortisone, VEGF, hFGF-beta, R3-IGF-1, ascorbic acid, heparin, and serum, and the concentration of FBS is about 0.3% to about 1%. In some embodiments, the basal medium can be a mixture of DMEM and F12.

Similarly, the inventors have also developed a beneficial epithelial cell culture medium for long term culture of epithelial cells. Accordingly, in one aspect, described herein is a method of maintaining long-term viability of epithelial cells (e.g. but not limited to hepatocytes). The method comprises contacting a population of epithelial cells with an epithelial cell culture medium comprising: (a) a basal epithelial medium; and (b) a supplement cocktail essentially consisting of Vitamin C (50ug/mL), insulin, transferrin, selenium, dexamethasone (1uM), glutamax (1%) and pen-strep (1%), and the concentration of FBS is about 0.3% to about 10%. In some embodiments, the basal epithelial medium is Williams E medium. Using the epithelial cell culture medium, the hepatocytes can be maintained as a viable monolayer for at least two weeks or longer.

Kits for cell culture are also described herein. In one aspect, the kit comprises (a) a cell culture device; and (b) an endothelial cell culture medium according to one or more embodiments described herein.

In some embodiments, the cell culture device provided in the kit can be a device for simulating a function of a tissue, which comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. In some embodiments, the first side or the second side of the membrane can comprise endothelial cells adhered thereon. In some embodiments, the first side or the second side of the membrane can comprise tissue-specific cells adhered thereon.. In some embodiments, tissue-specific cells can be cells derived from a liver. Also described herein, for reference, tissue-specific cells can be cells derived from any tissue or organ of interest, including, *e.g*., but not limited to a lung, a liver, a kidney, a skin, an eye, a brain, a blood-brain-barrier, a heart, a gastrointestinal tract, airways, a reproductive organ, and a combination of two or more thereof

In some embodiments, the basal medium and the supplement cocktail provided in the kit can be pre-mixed to form the endothelial cell culture medium. In some embodiments, the basal medium and the supplement cocktail provided in the kit can be pre-mixed to form the epithelial cell culture medium.

In some embodiments, the basal medium and the supplement cocktail provided in the kit can be separately packaged. For example, the basal medium and the supplement cocktail can be packaged independently, *e.g*., as powder or liquid.

In some embodiments, the kit can further comprise at least one vial of cells. In one embodiment, the cells can be endothelial cells. In one embodiment, the cells can be tissue-specific cells.

In one embodiment, the present invention contemplates a device, comprising: a first structure defining a first chamber; a second structure defining a second chamber; and a membrane located at an interface region between the first chamber and the second chamber to separate the first chamber from the second chamber, the membrane including a first side facing toward the first chamber and a second side facing toward the second chamber, the first side comprising an extracellular matrix composition and hepatocytes adhered on the extracellular matrix composition, the second side comprising liver endothelial cells, wherein the extracellular matrix composition comprises collagen (i.e. collagen I, collagen IV and fibronectin). In one embodiment, the liver endothelial cells comprise liver sinusoidal endothelial cells. In one embodiment, said viable hepatocytes are human hepatocytes. Also described herein, for reference, is where said viable hepatocytes are dog hepatocytes and rat hepatocytes.

In one embodiment, the present invention contemplates a method of culturing cells, comprising: a) providing a microfluidic device comprising a membrane, said membrane comprising a top surface and a bottom surface; b) seeding viable human hepatocytes on said top surface and viable human liver sinusoidal endothelial cells on said bottom surface; and c) culturing said seeded cells under flow conditions such that said cells remain viable for at least 14 days. In one embodiment, said human hepatocytes are primary human hepatocytes that were previously cryopreserved. In a further embodiment, the method further comprises d) assessing viability by measuring the level of activity and/or leakage of one or more cellular enzymes. In one embodiment, said human hepatocytes are primary human hepatocytes that were previously cryopreserved. In a further embodiment, the method further comprises d) assessing the level of activity and/or leakage of one or more cellular enzymes. In one embodiment, said cellular enzyme is a CYP450 enzyme. In one embodiment, said cellular enzyme is a transaminase. In a further embodiment, the method further comprises e) assessing viability by measuring the level of expression of one or more cellular proteins and/or their corresponding messenger RNA. In a further embodiment, the method further comprises assessing the level of expression of one or more cellular proteins and/or their corresponding messenger RNA. In one embodiment, prior to step b), said top surface of said membrane is treated with at least one extracellular matrix protein. In one embodiment, after step b), said viable human hepatocytes are covered with at least one extracellular matrix protein (i.e. collagen I, collagen IV and fibronectin). In one embodiment, said viable human hepatocytes are covered with a Matrigel overlay.

Also described herein, for reference, is a method of culturing cells, comprising: a) providing a microfluidic device comprising a membrane, said membrane comprising a top surface and a bottom surface; b) seeding viable dog hepatocytes on said top surface and viable dog liver sinusoidal endothelial cells on said bottom surface; and c) culturing said seeded cells under flow conditions such that said cells remain viable for at least 14 days. Dog hepatocytes were found to be more difficult to seed that those of humans or rats. In one instance, said dog hepatocytes are primary dog cryopreserved hepatocytes. In one instance, the method further comprises d) assessing viability by measuring the level of activity and/or leakage of one or more cellular enzymes. In one instance, said cellular enzyme is a CYP450 enzyme. In one instance, said cellular enzyme is a transaminase. In a further instance, the method further comprises e) assessing viability by measuring the level of expression of one or more cellular proteins and/or their corresponding messenger RNA. In one instance, prior to step b), said top surface of said membrane is treated with at least one extracellular matrix protein. In one instance, after step b), said viable dog hepatocytes are covered with at least one extracellular matrix protein. In one instance, said viable dog hepatocytes are covered with a Matrigel overlay.

Also described herein, for reference, is a method of culturing cells, comprising: a) providing a microfluidic device comprising a membrane, said membrane comprising a top surface and a bottom surface; b) seeding viable rat hepatocytes on said top surface and rat liver sinusoidal endothelial cells on said bottom surface; c) culturing said seeded cells under flow conditions such that said cells remain viable for at least 14 days. Also described herein, for reference is a method of culturing cells, comprising: a) providing a microfluidic device comprising a membrane, said membrane comprising a top surface and a bottom surface; b) seeding viable rat hepatocytes on said top surface and rat liver sinusoidal endothelial cells on said bottom surface; c) culturing said seeded cells under flow conditions such that said cells remain viable for at least 28 days. In one instance, the rat hepatocytes are primary rat cryopreserved hepatocytes. In one instance, prior to step b), said top surface of said membrane is treated with at least one extracellular matrix protein. In one instance, after step b), said viable rat hepatocytes are covered with at least one extracellular matrix protein . In one instance, said viable rat hepatocytes are covered with a Matrigel overlay.

### BRIEF DESCRIPTION OF THE DRAWINGS

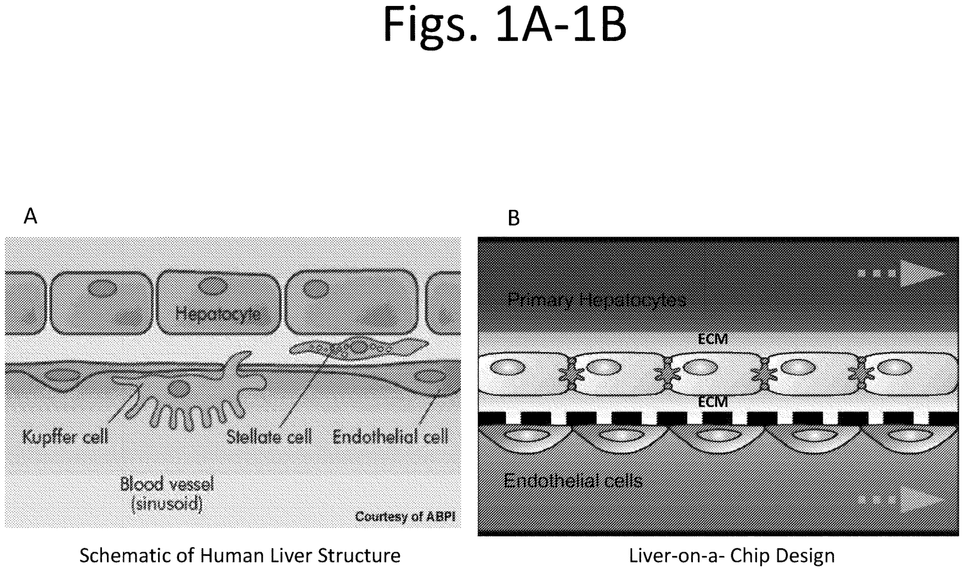

**Figs. 1A-1B** are schematic diagrams showing a cross-sectional view of a human liver structure (**Fig. 1A**) and of a liver-on-a- chip device according to one embodiment described herein (**Fig**. **1B**), respectively. The device is designed to mimic liver architecture and to support normal hepatocyte polarization for optimal hepatocyte viability and function.

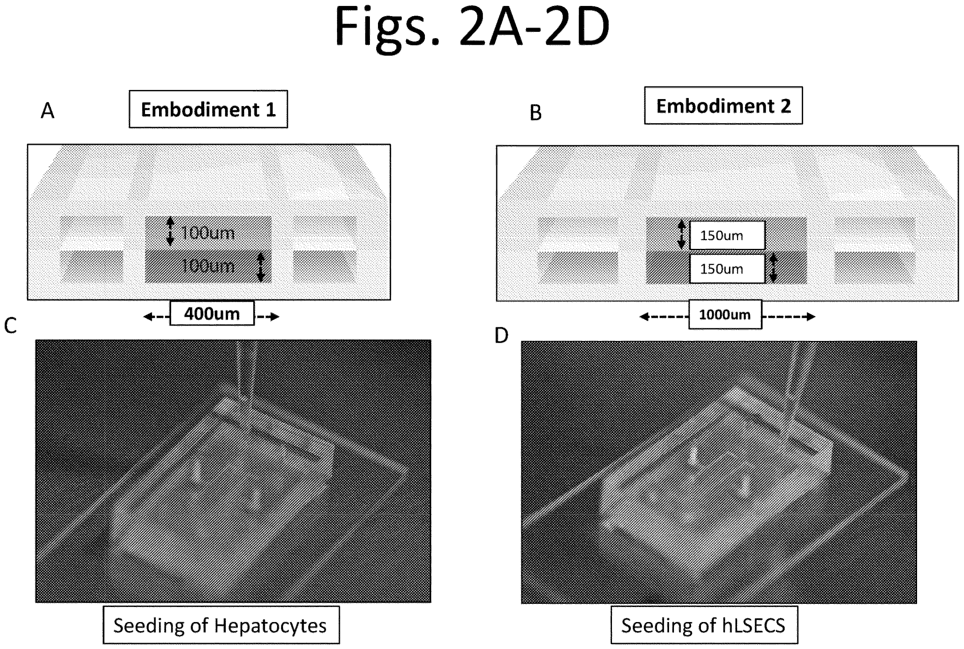

**Figs. 2A-2D** are images showing different embodiments of the devices described herein and seeding of hepatocytes and human liver sinusoidal endothelial cells (hLSECs) through different ports on the devices. **Fig. 2A** is a schematic diagram showing one embodiment of a liver-on-a-chip device. **Fig. 2B** is a schematic diagram showing another embodiment of a liver-on-a-chip device. **Fig. 2C** is a picture showing seeding of hepatocytes into the first chamber through a first port. **Fig. 2D** is a picture showing seeding of hLSECs into the second chamber through a second port.

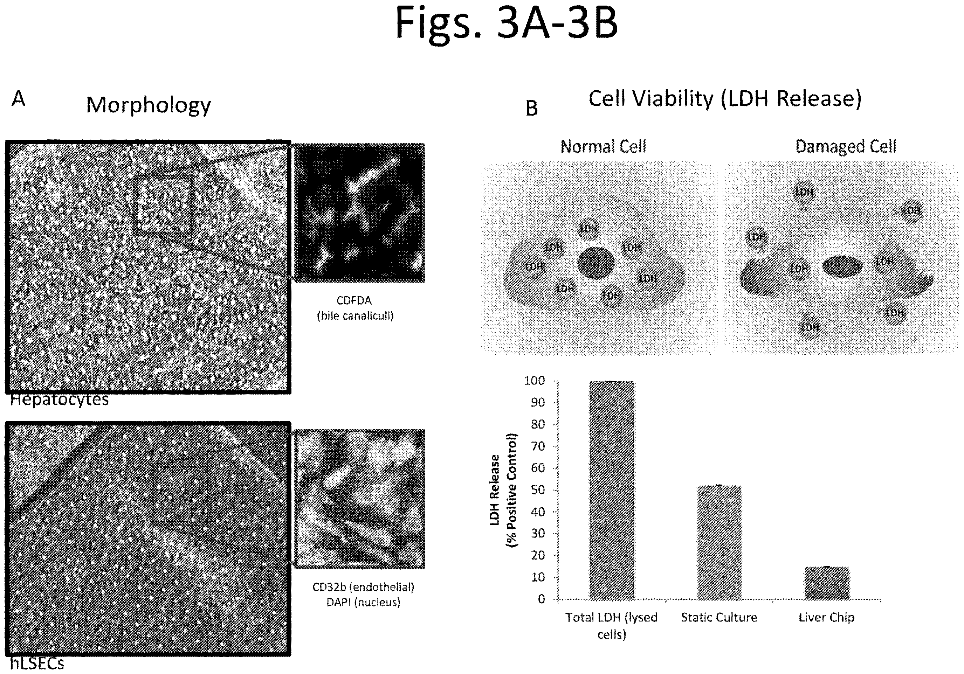

**Figs. 3A-3B** are experimental data showing human hepatocytes and hLSECs cultured under flow (e.g., at a shear stress of about 3.5 × 10⁻⁴ dyne/cm² to about 3.5 × 10⁻² dyne/cm²) in liver-on-a-chip device according to one embodiment described herein. **Fig. 3A** is a set of images showing hepatocytes (top panel) and hLSECs (bottom panel) at Day 13 of culture. The insets are fluorescent images showing (top panel) a live stain for CDFDA (a marker that is excreted into the bile via efflux transporters) to show formation of bile canaliculi; and (bottom panel) a stain for CD32b and DAPI to show viability of endothelial cells. **Fig. 3B** includes a bar graph showing that hepatocytes (on a chip that contains both hepatocytes and liver sinusoidal endothelial cells) released significantly lower levels of lactate dehydrogenase (LDH) than cells in a static culture, indicating that subjecting the cells to a low shear stress (*e.g*., a fluid flow) can facilitate maintenance of cell viability.

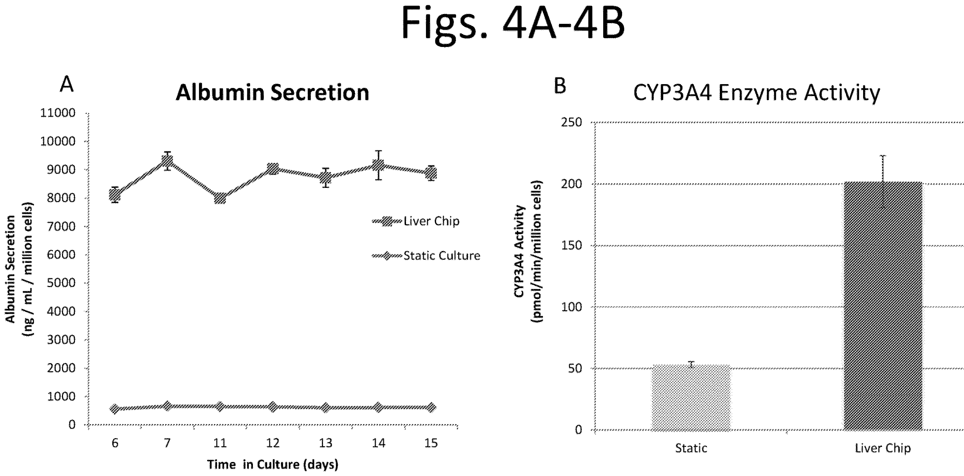

**Figs. 4A-4B** are data graphs showing hepatic function of a liver-on-a-chip device (also referred to as "liver chip") according to one embodiment described herein. **Fig. 4A** is a line graph showing that albumin secretion was significantly higher in the liver chip than in conventional static culture, and the high albumin secretion was maintained in the liver chip for at least 2 weeks or longer, indicating improved viability of hepatocyte-hLSEC co-culture in the liver chip than in the static culture. **Fig. 4B** is a bar graph showing that the liver chip's basal CYP3A4 enzyme activity level was at least 4 times greater than the level in static culture.

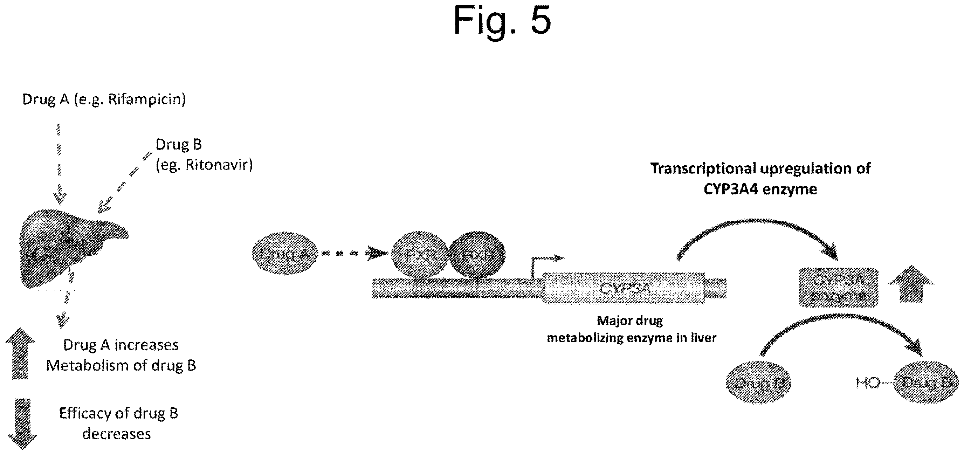

**Fig. 5** is a schematic illustration of functional relevance of liver CYP450 enzyme induction. When a subject takes both Drug A and Drug B together and both drugs interact with a CYP3A4 enzyme, Drug A can increase the metabolism of Drug B or decrease the efficacy of drug B. For example, Drug A can bind the nuclear receptor PXR in the hepatocytes and cause upregulation of the CYP3A4 enzyme, which will in turn increase metabolite formation of Drug B and thus reduce the level of Drug B in circulation and its efficacy. In addition, the increased metabolite formation of Drug B can increase toxic metabolites. In other circumstances, Drug A can lead to inhibition of a liver enzyme such as a CYP450 enzyme. Such circumstances can also lead to drug-drug interaction, and it is often beneficial to identify them.

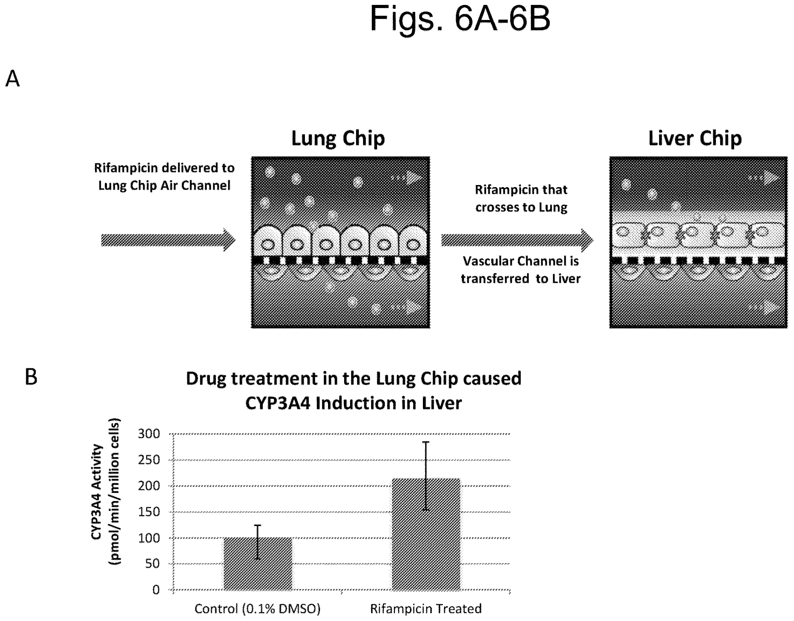

**Figs. 6A-6B** include experimental data showing drug treatment in a lung-on-a-chip device can cause CYP3A4 induction in a liver-on-a- chip device. **Fig. 6A** is a schematic diagram showing physiological coupling between a lung chip (*e.g.,* as described in the U.S. Patent No. 8,647,861) and a liver chip. Rifampicin was introduced to the "air" channel" with lung epithelial cells of the lung chip. The rifampicin delivered across the lung epithelial cells and endothelial cells to the "vascular" channel of the lung chip was then directed to hepatocytes in the liver chip. **Fig. 6B** is a bar graph showing that a ~2 fold increase in CYP3A4 drug metabolizing enzyme activity was detected in the liver chip when rifampicin was added in the lung chip.

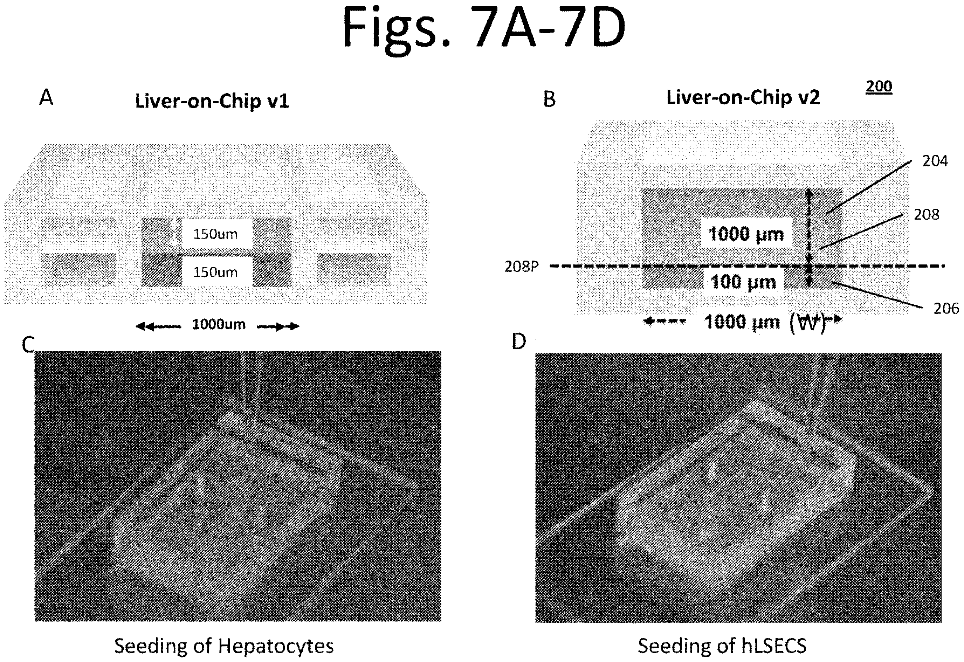

**Figs. 7A-7D** are images showing different embodiments of the devices described herein and seeding of hepatocytes and human liver sinusoidal endothelial cells (hLSECs) through different ports on the devices. **Fig. 7A** is a schematic diagram showing one embodiment of a liver-on-a-chip device. **Fig. 7B** is a schematic diagram showing another embodiment of a liver-on-a-chip device. **Fig. 7C** is a picture showing seeding of hepatocytes into the first chamber through a first port. **Fig. 7D** is a picture showing seeding of hLSECs into the second chamber through a second port.

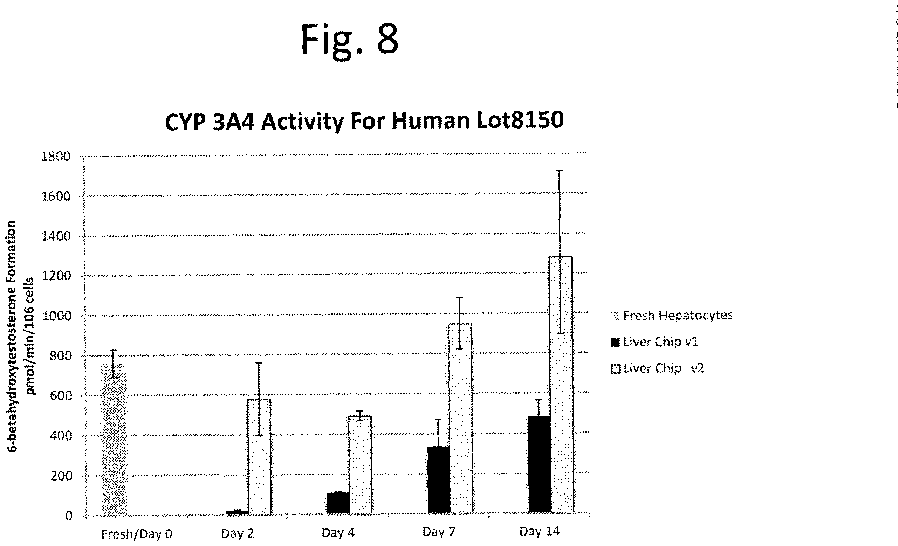

**Fig. 8** is a bar graph showing that basal CYP3A4 activity levels steadily increased in the liver chip shown in **Fig. 7A** (Liver chip v1), while the liver chip shown in **Fig. 7B** (Liver chip v2) showed comparable activity levels to fresh hepatocytes and the basal CYP3A4 activity was maintained in Liver chip v2 for at least about 2 weeks.

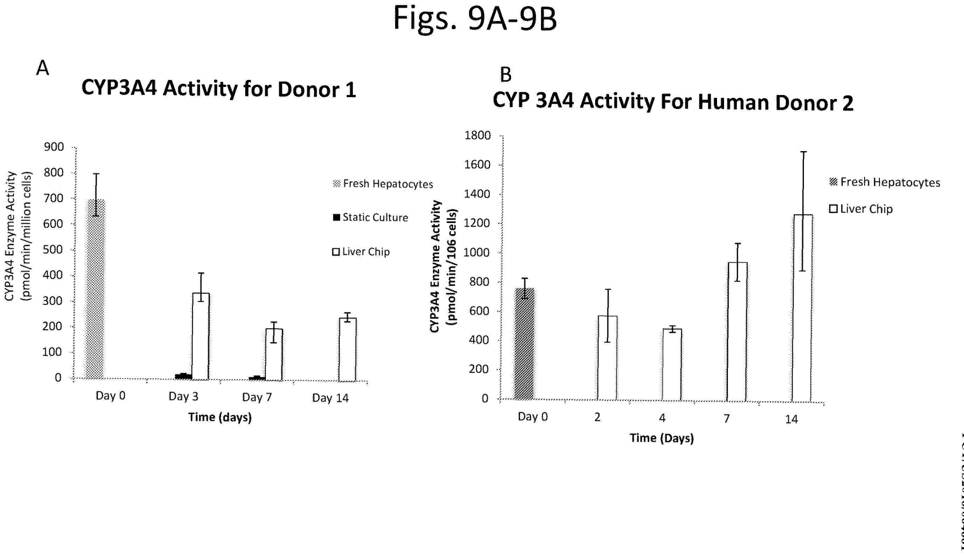

**Figs. 9A-9B** are bar graphs showing hepatic function of hepatocytes that were collected from human subjects cultured in the liver chip. Hepatocytes from Donor 1 (**Fig. 9A**) and Donor 2 (**Fig. 9B**) exhibited *in vivo*-relevant basal CYP3A4 levels for at least about 2 weeks when they were cultured in the liver chips, as compared to static cultures.

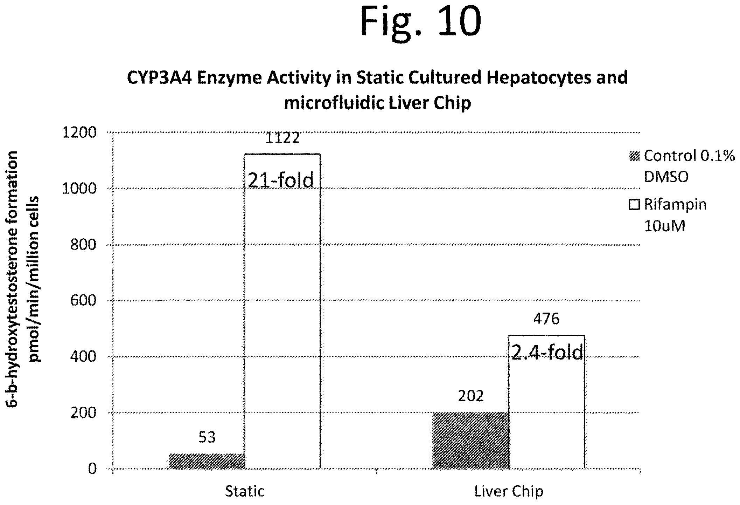

**Fig. 10** is a bar graph showing that basal levels of CYP3A4 activity were higher in the liver chips than in static Matrigel^{®} sandwich culture, the industry "gold standard." The CYP3A4 activity was reflected by measuring formation of 6-b-hydroxytestosterone from testosterone due to an increased CYP3A4 activity induced by rifampin.

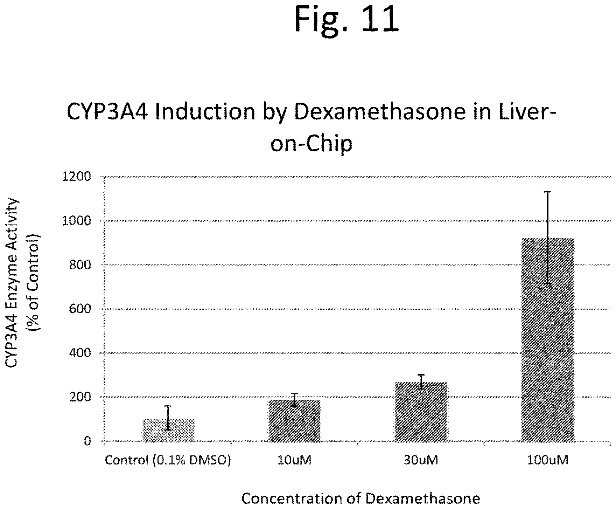

**Fig. 11** is a bar graph showing that induction of CY3A4 activity in the human liver chips increased with concentrations of dexamethasone.

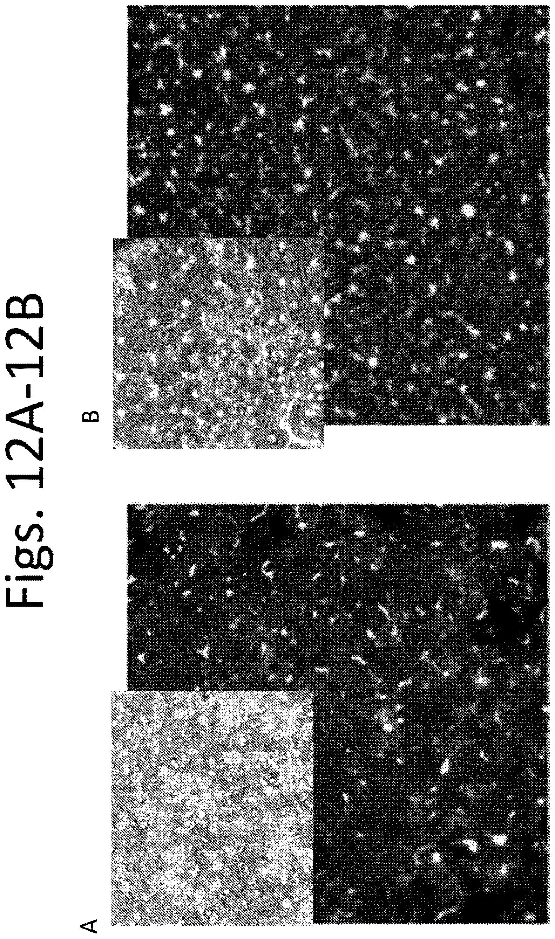

**Figs. 12A-12B** are fluorescent images comparing formation of bile canaliculi in the liver chips and static culture. A live stain for CDCFDA (a marker that is excreted into the bile via efflux transporters) was used. The stains showed increased bile canaliculi active transport by multidrug resistance-associated protein 2 (MRP2) in the liver chip (**Fig. 12B**), as compared to static culture (**Fig. 12A**). This shows increased formation of bile canaliculi in the liver chip, as compared to the static culture.

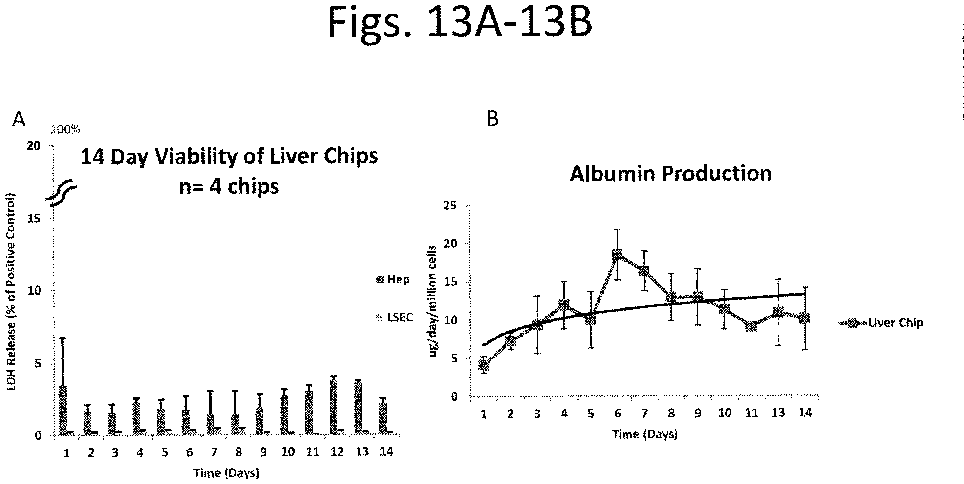

**Figs. 13A-13B** are data graphs showing that both hepatocytes and hLSEC monolayer maintained viable over a period of at least 2 weeks. **Fig. 13A** is a bar graph showing that both hepatocytes and hLSECs maintained low LDH release levels over a period of at least about 2 weeks. **Fig. 13B** is a line graph showing measurement of albumin production over time, indicating that albumin secretion was maintained in the liver chip for at least 2 weeks or longer.

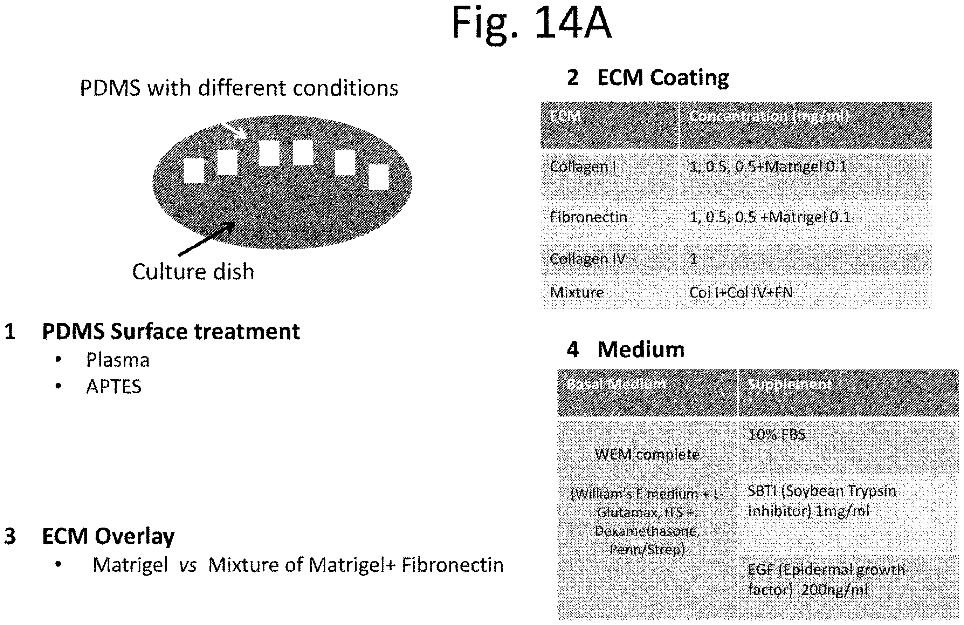

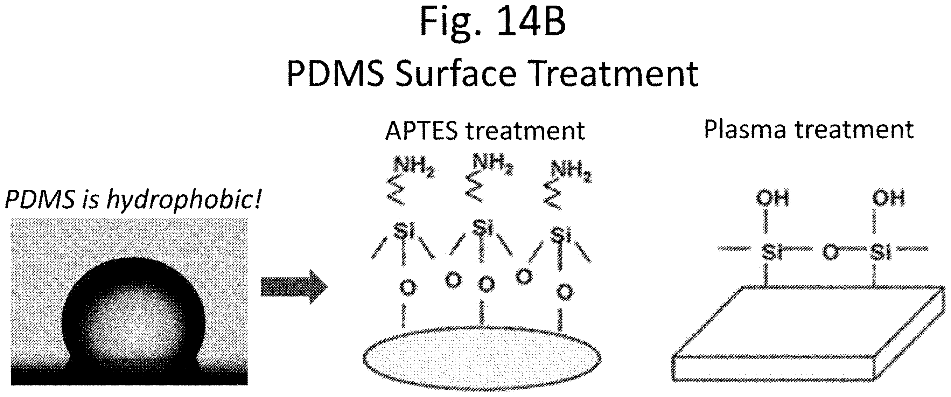

**Figs. 14A-14B** outline an exemplary optimization process to achieve long-term maintenance (*e.g*., viability and function) of a co-culture model of rat hepatocytes and rat LSECs (rLSECs) in a device as shown in **Fig. 7B****.** **Fig. 14A** shows four optimization parameters, namely (1) substrate surface treatment (*e.g*., PDMS surface treatment); (2) extracellular matrix (ECM) coating; (3) ECM overlay; and (4) culture medium composition. **Fig. 14B** is a schematic diagram showing exemplary surface treatment methods, *e.g.*, but not limited to APTES treatment and/or plasma treatment.

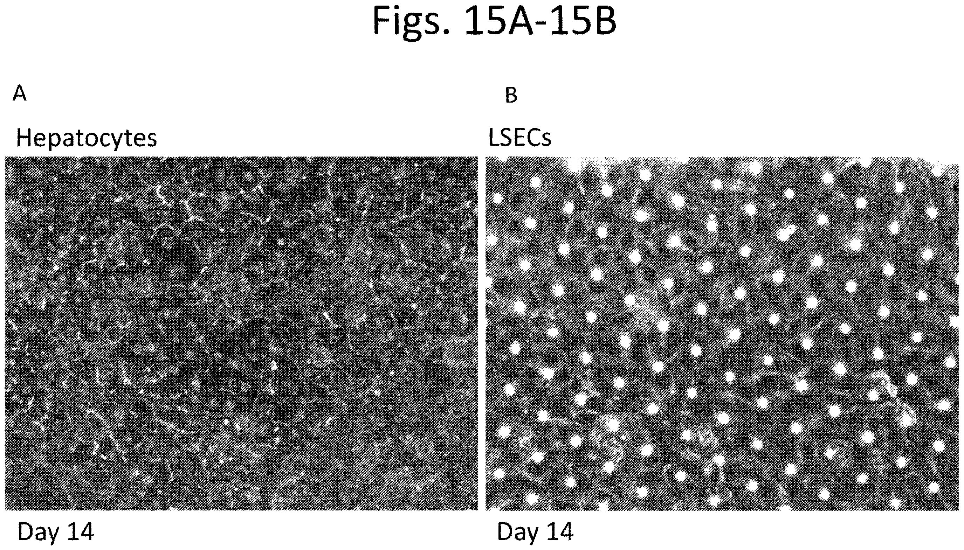

**Figs. 15A-15B** are phase contrast images showing a viable 2-week co-culture of rat hepatocytes (**Fig. 14A**) and rLSECs (**Fig. 14B**) in a liver-on-a-chip device that was optimized according to **Fig. 14A****.** Prior to culturing hepatocytes, the PDMS surface was plasma-treated, followed by an ECM coating of fibronectin (higher than 0.5 mg/mL) or an ECM coating mixture of fibronectin, collagen IV and collagen I. The ECM coating was further overlaid with a mixture of Matrigel^{®} and fibronectin. The WEM complete basal medium supplemented with about 10% FBS was used for the culture.

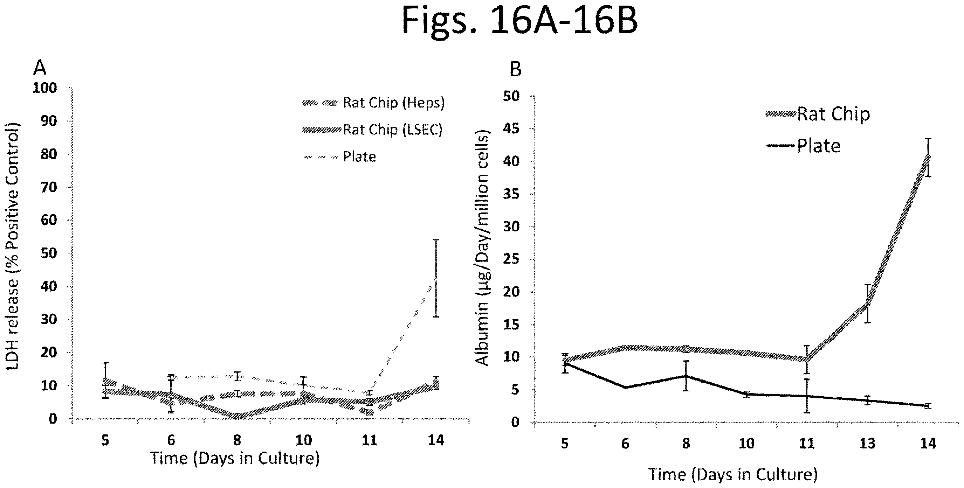

**Figs. 16A-16B** are line graphs showing improved viability of rat hepatocytes and rLSECs in the rat liver chips than in static cultures (*e.g*., plate culture). **Fig. 16A** shows that unlike static culture (*e.g*., plate culture), both rat hepatocytes and rLSECs maintained low LDH release levels over a period of at least about 2 weeks. **Fig. 16B** showed measurement of albumin production over time, indicating that albumin secretion in the rat liver chip was maintained or increased over at least 2 weeks.

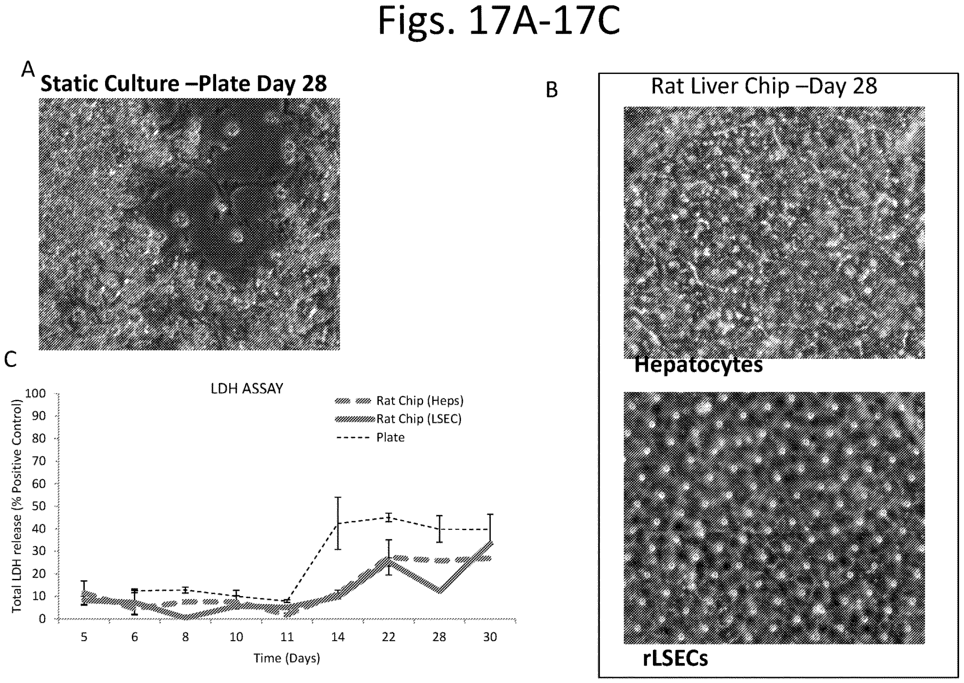

**Figs. 17A-17C** are experimental data showing a long-term co-culture (4 weeks) of rat hepatocytes and rLSECs in a rat liver chip. **Fig. 17A** is a phase contrast image showing cell morphology of a static plate culture after 4 weeks. **Fig. 17B** is a set of phase contrast images showing cell morphology of rat hepatocytes (top panel) and rLSECs (bottom panel) cultured in the rat liver chip after 4 weeks. **Fig. 17C** is a line graph showing improved viability of rat hepatocytes and rLSECs in the liver chip over a period of at least 4 weeks than in a static culture, as evidenced by a lower LDH release level measured in the liver chip.

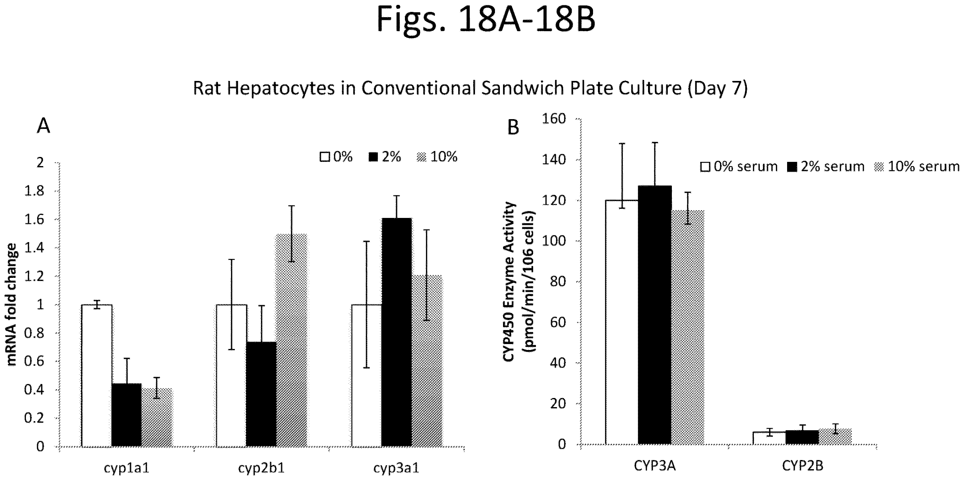

**Figs. 18A-18B** are bar graphs showing effects of serum on CYP450 mRNA expression and enzyme activity of rat hepatocytes. **Fig. 18A** shows that serum inhibited mRNA expression of cyp 1a1, but no significant effect on cyp2b1 and cyp3a1. **Fig. 18B** shows that the activity of CYP3A and CYP2B are serum-independent, as different concentrations of serum showed no effect on CYP3A and CYP2B activity.

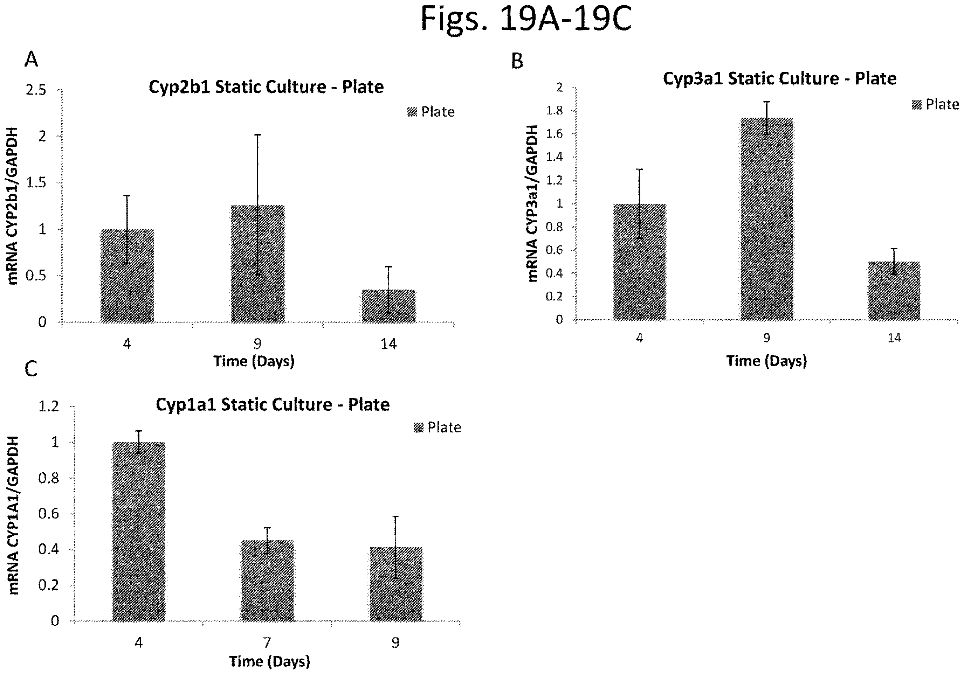

**Figs. 19A-19C** are bar graphs showing a time course of CYP450 mRNA expression in rat hepatocytes. The mRNA expression of cyp2b1 (**Fig. 19A**), cyp3a1 (**Fig. 19B**), cyp1a1 (**Fig. 19C**) in rat hepatocytes declined over time.

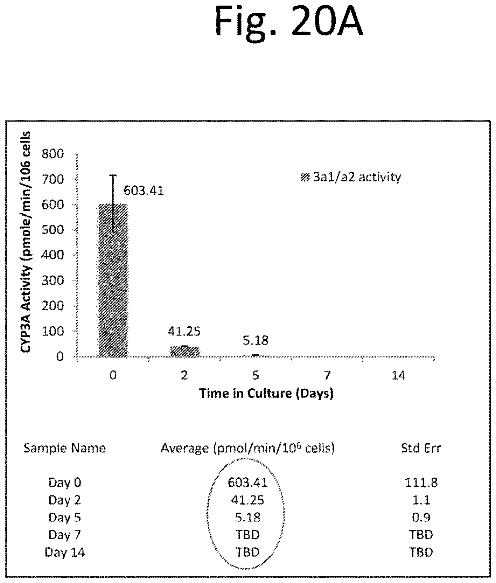

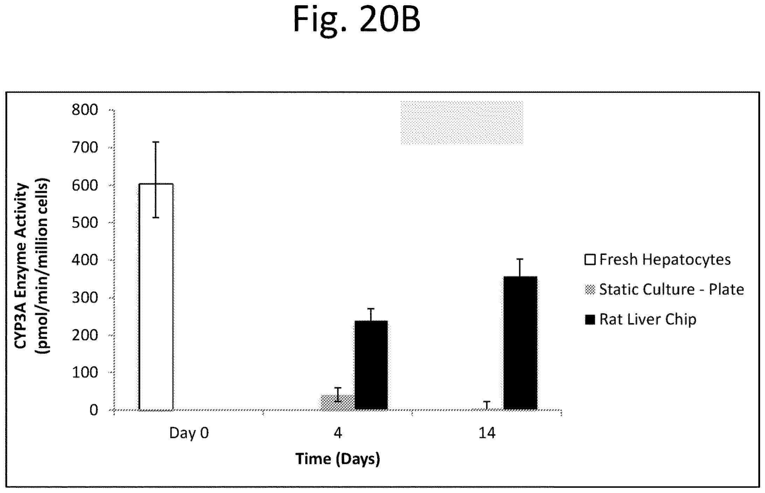

**Figs. 20A-20B** are bar graphs showing a time course of CYP3A activity in rat hepatocytes cultured in static cultures and liver chips. **Fig. 20A** shows that CYP3A1/A2 enzyme activity in rat hepatocytes declined over time in static cultures (*e.g*., conventional sandwich plate culture). **Fig. 20B** shows improved CYP3A4 activity in rat liver chips over static culture and maintenance of the CYP3A4 activity in the liver chip for at least two weeks.

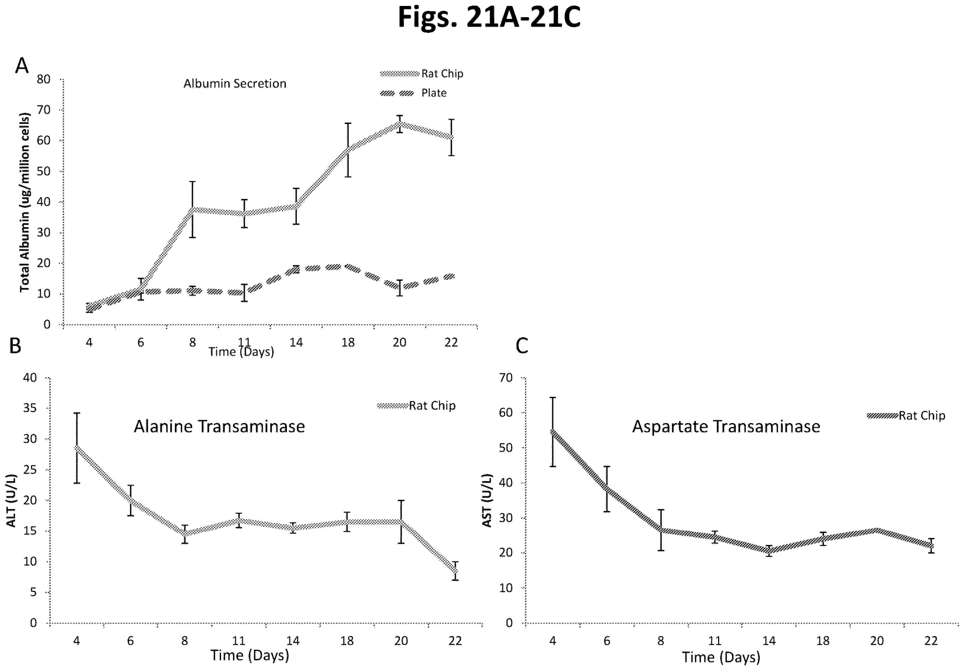

**Figs. 21A-21C** are line graphs showing liver specific functions measured in a rat liver chip. **Fig. 21A** shows high levels of albumin secretion in the rat liver chip, whereas the static plate shows low levels of albumin, indicating that the rat liver chips had improved hepatocyte viability over the static cultures. **Fig. 21B** shows a time course of alanine transaminase (ALT) activity measured in the liver chip. **Fig. 21C** shows a time course of aspartate transaminase (AST) measured in the liver chip. The ALT and AST activity levels measured in the liver chips were within the corresponding normal ranges in rat and human *in vivo* as shown in Table 3 in the Examples.

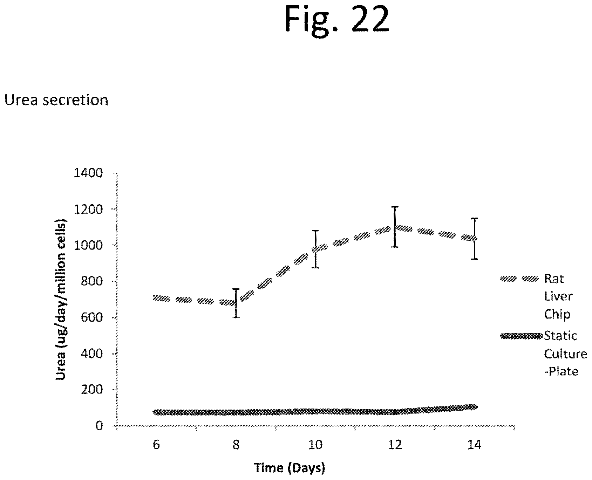

**Fig. 22** is a line graph showing urea synthesis function measured in a rat liver chip. Urea levels were maintained in the rat liver chips for at least 2 weeks and the levels were higher than static cultures (*e.g*., plates).

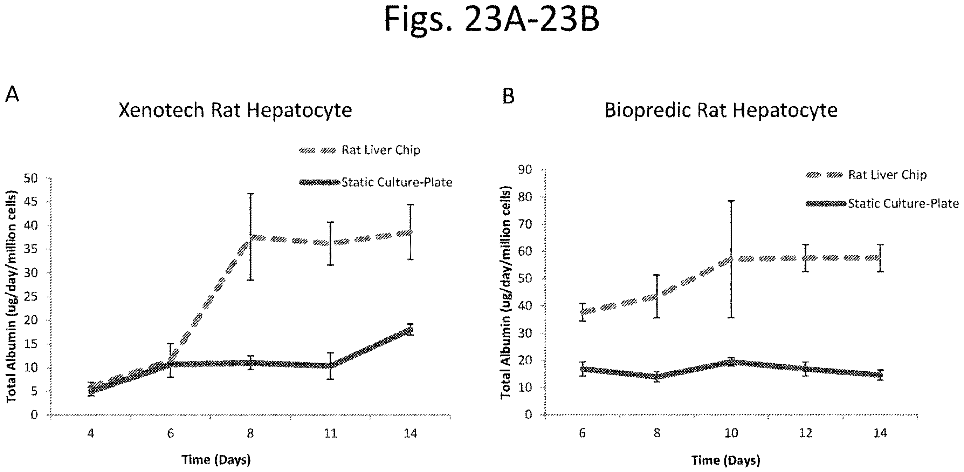

**Figs. 23A-23B** are line graphs showing hepatic function of rat hepatocytes obtained from different vendors. **Fig. 23A** shows albumin secretion of rat hepatocytes from Xenotech cultured in rat liver chips or in static culture plates. **Fig. 23B** shows albumin secretion of rat hepatocytes from Biopredic cultured in rat liver chips or in static culture plates.

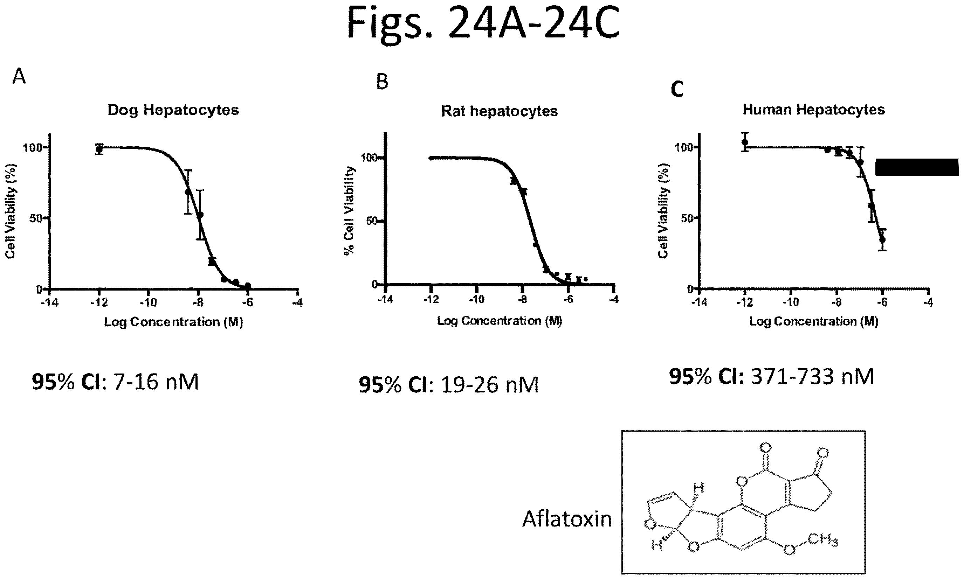

**Figs. 24A-24C** are dose response curves generated using hepatocytes of different species: dog (**Fig. 24A**), rat (**Fig. 24B**), and human (**Fig. 24C**). The figures show potency of aflatoxin B1 on different species as determined in the static plate cultures.

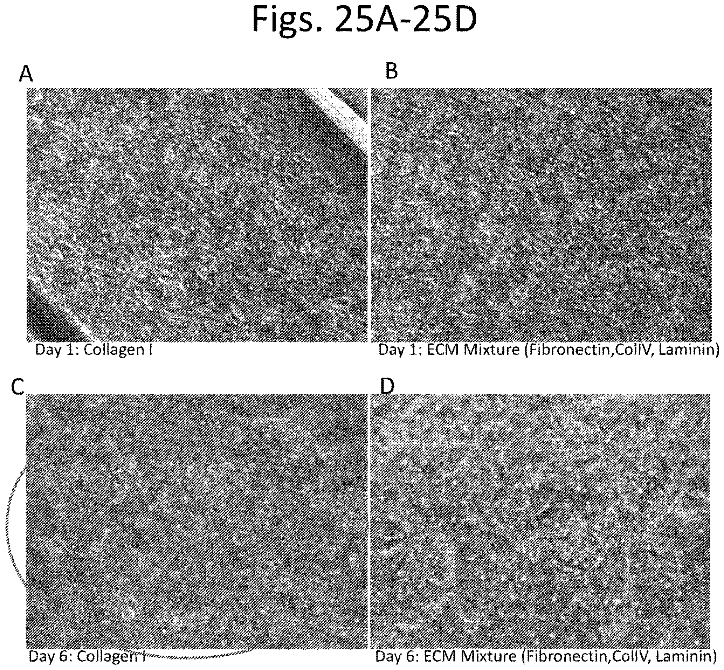

**Figs. 25A-25D** are phase contrast images showing cell morphology of dog hepatocytes growing on a surface with various ECM coatings. An ECM coating of collagen I was used in **Figs. 25A** and **25C**, while an ECM mixture coating comprising fibronectin, collagen IV, and laminin was used in **Figs. 25B** and **25D. Figs. 25A -25B** correspond to 1-day cultures; and **Figs. 25C-25D** correspond to 6-day cultures. The figures show that an ECM coating of collagen I provided improved viability and growth of dog hepatocytes.

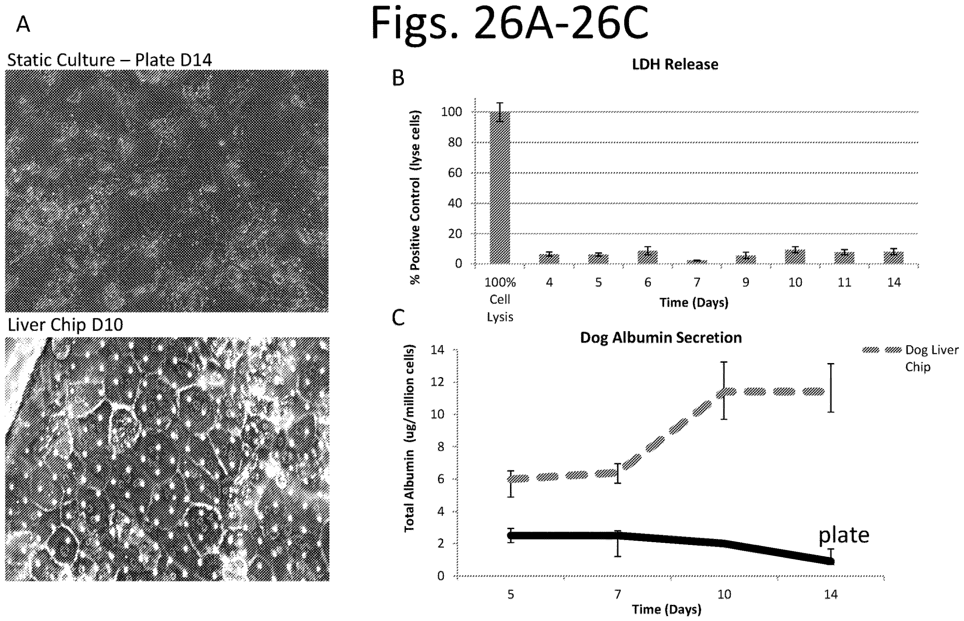

**Figs. 26A-26C** are experimental data showing cell viability and function of dog hepatocytes and dLSECs cultured in a dog liver chip. **Fig. 26A** is a set of phase contrast images showing cell morphology of dog hepatocytes in static cultures (top panel) and liver chips (bottom panel). **Fig. 26B** is a bar graph showing dog hepatocytes maintained low LDH release levels over a period of at least about 2 weeks, indicating viability and health of the hepatocytes monolayer being maintained in the dog liver chip over a period of at least 2 weeks. **Fig. 26C** is a line graph showing measurement of albumin production over time, indicating that the dog liver chip remained viable and maintained a high level of albumin secretion for at least about 2 weeks, as compared to static plate cultures.

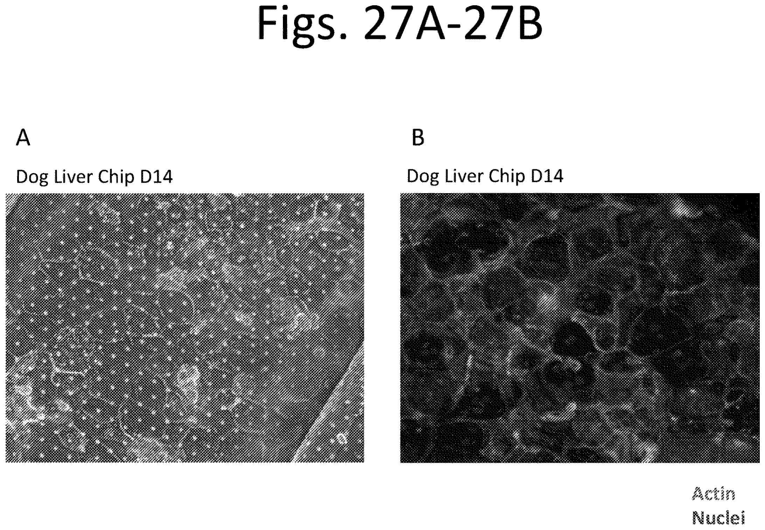

**Figs. 27A-27B** are microscopic images showing cell morphology of dog hepatocytes cultured in the liver chips after 2 weeks. **Fig. 27A** is a phase contrast image showing cuboidal morphology of dog hepatocytes. **Fig. 27B** is a fluorescent image showing F-actin staining of pericanalicular distribution in the hepatocyte monolayer cultured in the dog liver chips.

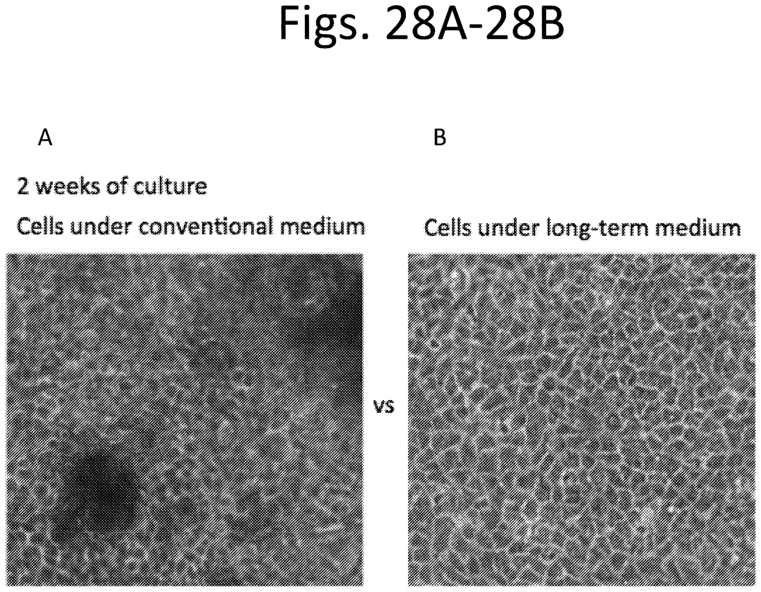

**Figs. 28A-28B** are brightfield images showing endothelial cells cultured for 2 weeks under an existing commercial culture medium (**Fig. 28A**) and a novel endothelial cell culture medium according to one embodiment described herein (**Fig. 28B**).

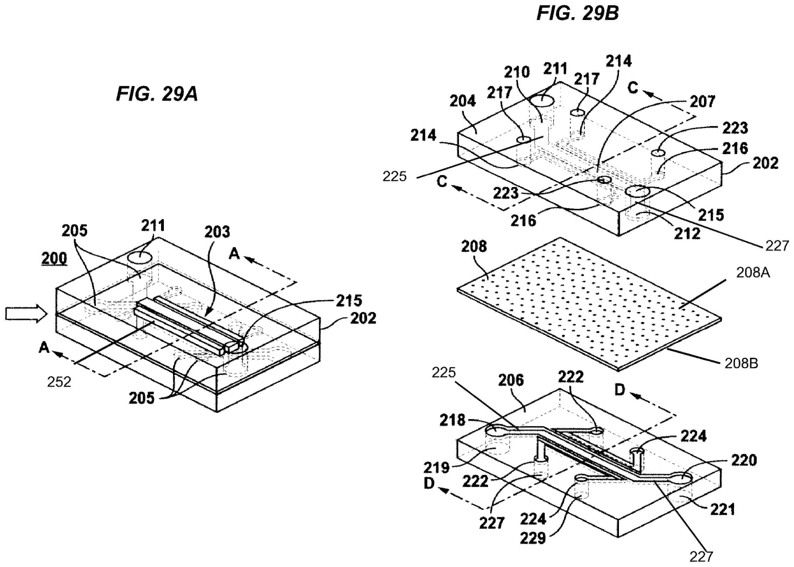

**Fig. 29A** illustrates a perspective view of a device in accordance with an embodiment. **Fig.** 29B illustrates an exploded view of the device in accordance with an embodiment.

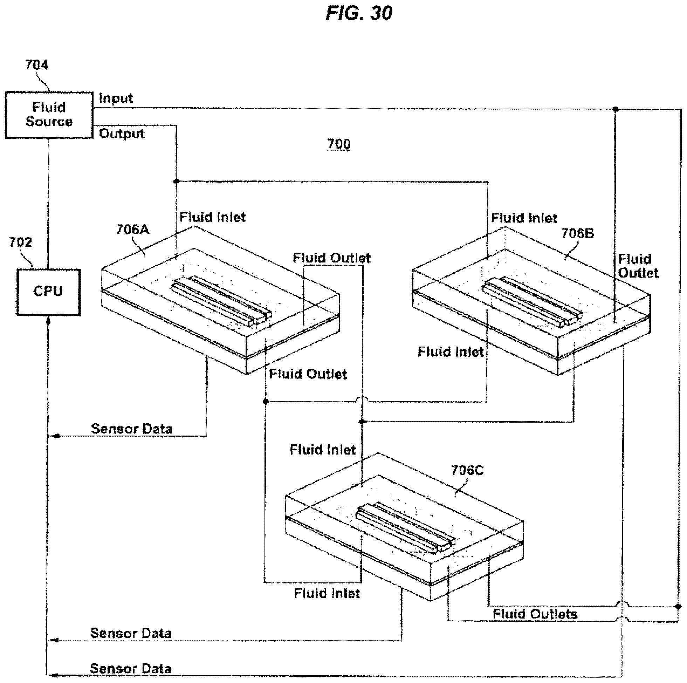

**Fig. 30** illustrates a system diagram employing at least one device described herein, which can be fluidically connected to another device described herein, an art-recognized organ-on-a-chip device, and/or to fluid sources.

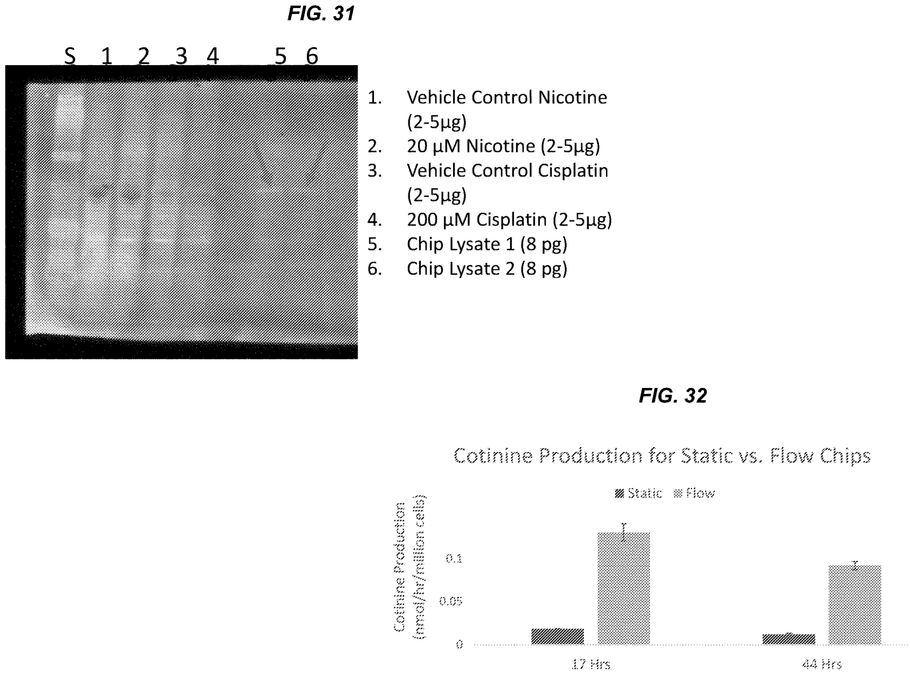

**Fig. 31** depicts analysis of protein levels demonstrating that CYP2A6 is expressed in liver cells grown on chips as described herein.
**Fig. 32** depicts a graph of cotinine production, comparing the rate of nitoctine to cotinine production in static vs. flow conditions. Error bars are standard deviations and represent averages of 2 chips

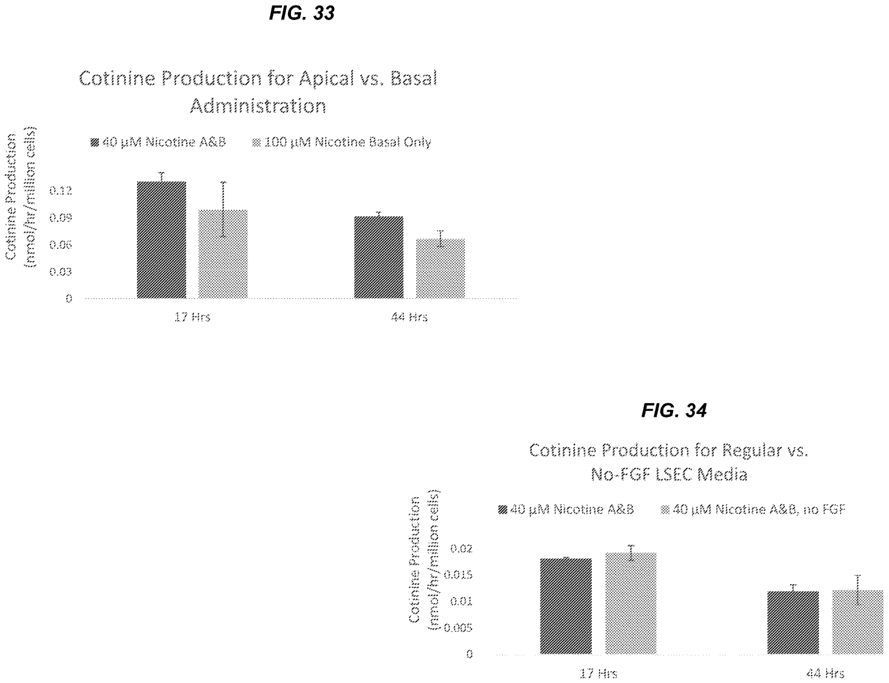

**Fig. 33** depicts a graph of nicotine to cotinine production in chips as described herein, comparing apical and basal administration of flow.
**Fig. 34** depicts a graph of nicotine to cotinine production in chips as described herein, comparing the effect of FGF in static chips.

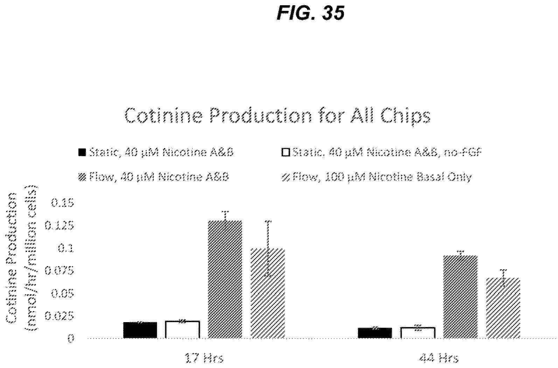

**Fig. 35** depicts a graph of nicotine to cotinine production in chips as described herein, under the indicated conditions. Error bars are standard deviations & represent averages between 2 chips

### DETAILED DESCRIPTION OF THE INVENTION

Aspects described herein stem from, at least in part, design of devices that allow for a controlled and physiologically realistic co-culture of liver sinusoidal endothelial cells in one chamber with hepatocytes in other chamber(s) to establish hepatic function *in vitro.* In one embodiment, the chambers are aligned (*e.g*., vertically) with each other with one or more membranes separating them from each other ("liver-on-a-chip"). The liver-on-a-chip devices have been developed and optimized based on the basic design of an organ-on-a-chip as described in the U.S. Patent No. 8,647,861, and the International Patent App. No. PCT/US2014/071611. In some aspects, the inventors have optimized the design of the liver-on-a chip devices and culture conditions to provide long-term hepatic culture with physiologically relevant hepatic function (*e.g*., albumin and/or urea secretion, and/or CYP 450 metabolic capacity) for different animal models, *e.g*., human. Described herein, for reference, are also devices and culture conditions for different animal models, e.g., rats and dogs Further, in some aspects, the inventors have formulated a universal endothelial cell culture medium for long-term culture, which performs superior to the existing commercially available media. The novel endothelial cell culture media developed by the inventors allow co-cultures of endothelial and epithelial cells of liver to maintain functionality and viability for at least one month (e.g. 28 - 30 days) in a cell culture device, which is otherwise impossible using the commercial culture media. Described herein, for reference, also is novel endothelial cell culture media developed by the inventors allow co-culture of endothelial and epithelial cells of different tissue origin (e.g., lung, gut, kidney). Accordingly, embodiments of various aspects described herein relate to devices for simulating a function of a tissue and methods of using the same. Novel endothelial cell culture media for long-term culture and methods of using the same are also described herein.

Those of ordinary skill in the art will realize that the following description is illustrative only and is not intended to be in any way limiting. Other embodiments will readily suggest themselves to such skilled persons having the benefit of this disclosure. Reference will now be made in detail to implementations of the example embodiments as illustrated in the accompanying drawings. The same reference indicators will be used throughout the drawings and the following description to refer to the same or like items. It is understood that the phrase "an embodiment" encompasses more than one embodiment and is thus not limited to only one embodiment for brevity's sake.

### Exemplary devices for simulating a function of a tissue

Some aspects described herein relate to devices for simulating a function of a tissue. In one aspect, the device generally comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side of the membrane has an extracellular matrix composition disposed thereon, wherein the extracellular matrix (ECM) composition comprises an ECM coating layer and an ECM overlay composition over the ECM coating layer.

As used herein, the terms "extracellular matrix" and "ECM" refer to a natural or artificial composition comprising, essentially consisting of, or consisting of extracellular molecules generally secreted by cells that provide structural and/or biochemical support for self and/or surrounding cells. In some embodiments, the terms "extracellular matrix" and "ECM" can refer to a composition comprising a solubilized basement membrane extracted from a tissue. The composition and structure of ECMs can vary depending on the source of the tissue. For example, sarcoma, small intestine submucosa (SIS), urinary bladder matrix (UBM) and liver stroma ECM each differ in their overall structure and composition due to the unique cellular niche needed for each tissue. Examples of ECM molecules include, but are not limited to, collagen, fibronectin, laminin, vitronectin, tenascin, entactin, thrombospondin, elastin, gelatin, fibrillin, merosin, anchorin, chondronectin, link protein, bone sialoprotein, osteocalcin, osteopontin, epinectin, hyaluronectin, undulin, epiligrin, and Kalinin, glycosaminoglycans, proteoglycans, chemoattractants, cytokines, growth factors, and any combinations thereof. Examples of commercially available extracellular matrices that can be used to prepare the ECM coating layer and/or ECM overlay composition include, for example, but not limited to an ECM composition by Sigma-Aldrich or Matrigel^{™} Matrix by Becton, Dickinson & Company. Matrigel^{™} matrix is a reconstituted basement membrane isolated from mouse Engelbreth-Holm-Swarm (EHS) tumor. Matrigel^{™} matrix comprises predominantly laminin, collagen IV, entactin and heparan sulfate proteoglycans. Matrigel^{™} matrix can also comprise a number of growth factors, including, *e.g.,* but are not limited to EGF, IGF-1, PDGF, TGF-β, VEGF, bFGF, and any combinations thereof. In some embodiments, the terms "extracellular matrix" and "ECM" also encompass a purified composition comprising a specific type of ECM molecules.

As used herein, the term "ECM coating layer" refers to a layer comprising at least one type or a mixture of extracellular matrix (ECM) molecules adsorbed or bound or coupled to a cell culture surface, *e.g.,* a membrane surface. The ECM coating layer can be formed on a membrane surface by depositing a solution of an ECM coating solution on the surface and allowing the ECM molecules to adsorb or bound to the surface, and/or patterning or "stamping" ECM molecules directly onto the surface, *e.g*., in a specific pattern or arrangement.

As used herein, the term "ECM overlay composition" refers to a composition comprising at least one type or a mixture of extracellular matrix molecules. In some embodiments, the ECM overlay composition can be placed on top of cells (including, but not limited to hepatocytes, cholangiocytes (biliary endothelial cells) and/or liver fibroblasts) that are adhered on an ECM coating layer as described herein. The ECM overlay composition can be placed on top of cells (including, but not limited to hepatocytes, cholangiocytes (biliary endothelial cells) and/or liver fibroblasts) by depositing an ECM solution on top of the cells. The ECM overlay composition can have a similar composition as the ECM coating layer or have a different composition from the ECM coating layer.

In some embodiments, the ECM overlay composition can be a gel layer (and more typically, a very thin coating of gel) comprising at least one type or a mixture of extracellular matrix molecules. The ECM gel layer or coating can be placed on top of the ECM coating layer by depositing an ECM gel solution on top of the ECM coating layer and allowing the ECM gel solution to form a gel layer.

In some embodiments, the ECM overlay or gel composition can be provided in a concentration that is below gelling concentration of the respective extracellular matrix molecule(s). Accordingly, the total concentration of the ECM overlay composition can range from about 10 µg/ml to about 10 mg/ml, or about 50 µg/ml to about 10 mg/ml, or about 100 µg/ml to about 10 mg/ml, or about 100 µg/ml to about 3 mg/ml. In one embodiment, the total concentration of the ECM overlay composition can be about 250 µg/ml.

The composition and concentration(s) of extracellular matrix component(s) in the ECM coating solution and/or ECM overlay or gel solution can vary with and be optimized for cells of different tissue types and/or of different species (*e.g*., human cells vs. rat cells or dog cells) using methods known in the art. For example, as shown in the Reference Example 3, the inventors cultured rat hepatocytes on surfaces treated with different compositions of an ECM coating layer and/or ECM overlay composition or ECM gel layer, and then monitored cell morphology and/or growth to determine an optimal ECM coating and/or ECM overlay composition that allows for consistent performance of the cell culture surface in maintaining viability and function of the hepatocytes. The inventors have determined that an ECM coating layer formed from (i) an ECM coating solution containing about 0.5 mg/mL fibronectin, about 0.4 mg/mL of collagen IV, and about 0.1 mg/mL of collagen I; or (ii) an ECM coating solution containing higher than 0.5 mg/mL fibronectin provided an optimal condition for cell attachment/growth of rat hepatocytes on a membrane surface. The inventors have also determined that an ECM coating of matrigel (250 ug/mL) provides good results for liver cells on chips.

In some instances, the ECM coating layer can comprise, essentially consist of, or consist of fibronectin and/or collagen I, or a functional fragment or variant thereof.

In some instances, the ECM coating layer can comprise, essentially consist of, or consist of collagen IV, or functional fragments or variants thereof.

In some embodiments, the ECM coating layer can comprise, essentially consist of, or consist of fibronectin, collagen IV, and collagen I, or functional fragments or variants thereof. In some embodiments, the fibronectin component can be present at about 30% (w/w) to about 70% (w/w) of the extracellular matrix components. In one embodiment, the fibronectin component can be present at about 50% (w/w) of the extracellular matrix components. In some embodiments, the collagen IV component can be present at about 20% (w/w) to about 60% (w/w) of the extracellular matrix components. In one embodiment, the collagen IV component can be present at about 40% (w/w) of the extracellular matrix components. In some embodiments, the collagen I component can be present at about 1% (w/w) to about 20% (w/w) of the extracellular matrix components. In one embodiment, the collagen I component can be present at about 10% (w/w) of the extracellular matrix components.

In some embodiments, the fibronectin component can be present at about 0.5 mg/mL to about 1 mg/mL of the extracellular matrix components. In some embodiments, the collagen IV component can be present at about 1 mg/mL of the extracellular matrix components. In some embodiments, the collagen I component can be present at about 0.5 mg/mL to about 1 mg/mL of the extracellular matrix components.

In some embodiments, the ECM coating layer can comprise laminin or a functional fragment thereof. In some embodiments, the laminin component can be present at about 0.5% (w/w) to about 30% (w/w) of the extracellular matrix components. In some embodiments, the laminin can be present at about 100 µg/mL of the extracellular matrix components.

In some embodiments, the total extracellular matrix components can be applied to a membrane surface (*e.g*., the first side of the membrane) in a concentration of between about 0.05 mg/mL to about 5 mg/mL. In some embodiments, the total concentration of the extracellular matrix components applied to the membrane surface (*e.g*., the first side of the membrane) can range from 0.1 mg/mL to about 5 mg/mL. In some embodiments, the total concentration of the extracellular matrix components applied to the membrane surface (*e.g*., the first side of the membrane) can range from 1 mg/mL to about 3 mg/mL.

The inventors have also determined that an ECM overlay composition formed from an ECM overlay solution containing about 20 µg/mL to about 1 mg/mL fibronectin, and about 100 µg/mL to about 10 mg/ml basement membrane-derived proteins (*e.g*., Matrigel^{™}), alone in combination with fibronectin, provided an optimal condition for cell attachment/growth of rat hepatocytes on a membrane surface.

The inventors have also determined that an ECM overlay composition or ECM gel coating or layer formed from an ECM gel solution containing no fibronectin, and about 250 mg/mL of basement membrane-derived proteins (*e.g*., Matrigel^{™}) provided an optimal condition for cell attachment/growth of rat hepatocytes on a membrane surface.

Accordingly, in some embodiments, the ECM overlay composition or gel layer can comprise, essentially consist of, or consist of a protein mixture gel derived from a basement membrane. In one embodiment, the protein mixture gel derived from a basement membrane comprises Matrigel^{™}.

In some embodiments, the ECM overlay composition can comprise, essentially consist of, or consist of a protein mixture derived from a basement membrane and/or collagen I. In some embodiments, the ECM overlay composition can comprise, essentially consist of, or consist of a protein mixture derived from a basement membrane and/or collagen I, and fibronectin. In one embodiment, the protein mixture derived from a basement membrane comprises Matrigel^{™}.

In some embodiments where the ECM overlay composition comprises, essentially consists of, or consists of a protein mixture derived from a basement membrane (*e.g.,* Matrigel^{™}) and/or collagen I, and fibronectin or a functional fragment or variant thereof. In some embodiments, the basement membrane-derived protein mixture and/or collagen I can be present at about 50 (w/w)% to about 99.9 (w/w)% of the total extracellular matrix components. In some embodiments, the fibronectin component can be present at about 0.1 (w/w)% to about 50 (w/w)% of the extracellular matrix components.

In some embodiments, the total extracellular matrix components can be applied over an ECM coating layer described herein to form an ECM overlay composition in a concentration of between about 50µg/mL to about 10 mg/mL. In some embodiments, the total concentration of the extracellular matrix components applied over the ECM coating layer can range from 100 µg/mL to about 10 mg/mL. In some embodiments, the total concentration of the extracellular matrix components applied over the ECM coating layer can be about 250 µg/mL.

In some embodiments where human are used, the ECM overlay composition can comprise, essentially consist of, or consist of a protein mixture derived from a basement membrane (*e.g*., Matrigel^{™}). Also described herein for reference, where dog hepatocutes are used, the ECM overlay composition can comprise, essentially consist of, or consist of a protein mixture derived from a basement membrane (*e.g*., Matrigel^{™}). Also described herein for reference where rat hepatocytes are used, the ECM overlay composition can comprise, essentially consist of, or consist of a protein mixture derived from a basement membrane (*e.g*., Matrigel^{™}) and fibronectin.

Accordingly, described herein is also a device for simulating a function of a tissue, wherein the device comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side of the membrane has an extracellular matrix composition disposed thereon. The extracellular matrix (ECM) composition comprises (a) an ECM coating layer comprising fibronectin and collagen I and collagen IV; and (b) an ECM overlay composition over the ECM coating layer, the ECM overlay composition comprising a mixture of basement membrane-derived proteins and optionally fibronectin.

In some embodiments, the ECM coating layer can further comprise collagen IV.

In some embodiments of various aspects described herein, the device can further comprise a monolayer, or a multi-layered or three-dimensional structure, comprising at least one type of tissue-specific cells adhered on the ECM coating layer and/or on the ECM composition. Appropriate tissue-specific cells can be selected depending on the organization and/or function of a tissue to be modeled. For example, tissue-specific cells are generally parenchymal cells (*e.g*., epithelial cells) derived from a tissue or an organ which is a liver. Also described herein, for reference, is where tissue-specific cells derived from a tissue or organ which is lung, a kidney, a skin, an eye, a brain, a blood-brain-barrier, a heart, a gastrointestinal tract, airways, a reproductive organ, and a combination of two or more thereof.

In some embodiments, the devices described herein are optimized to simulate a function of a liver tissue. In some embodiments, the device can further comprise hepatocytes adhered on the extracellular matrix composition. The viable hepatocytes can be derived from humans. Also disclosed herein, for reference, viable hepatocytes can be derived from different mammalian sources, including, e.g., but not limited torats, mice, and dogs. Thus, the devices can be used to simulate a function of a liver tissue in different animal model of interest.

In some embodiments, the device can further comprise hepatocytes adhered on the ECM coating layer and overlaid by the ECM overlay layer. Other cell types (e.g., non-parenchymal liver cells) including, but not limited to cholangiocytes (biliary endothelial cells) and/or liver fibroblasts, that are generally present in a liver in vivo can also be included between the ECM coating layer and the ECM overlay layer. Cell types are derived from human. Also described herein, for reference, are cell types derived from different mammalian sources, including, *e.g*., but not limited to rats, mice, and dogs. Thus, the devices can be used to simulate a function of a liver tissue in different animal model of interest.

As used herein, the term "monolayer" refers to a single layer of cells on a growth surface, on which no more than 10% (*e.g.,* 10%, 9%, 8%, 7%, 6%, 5%, 4%, 3%, 2%, 1%, or 0%) of the cells are growing on top of one another, and at least about 90% or more (e.g., at least about 95%, at least 98%, at least 99%, and up to 100%) of the cells are growing on the same growth surface. In some embodiments, all of the cells are growing side-by side, and can be touching each other on the same growth surface. The condition of the cell monolayer can be assessed by any methods known in the art, *e.g*., microscopy, and/or immunostaining for cell-cell adhesion markers. In some embodiments where the cell monolayer comprises a hepatocyte monolayer, the condition of the hepatocyte monolayer can be assessed by presence of cuboidal cell morphology and/or staining for any art-recognized markers in hepatocytes (e.g., staining for bile caniculi, mrp2 transporter or CDFDA, albumin, and/or HNF4). In some embodiments, the hepatocyte monolayer can further comprise cholangiocytes (biliary endothelial cells) and/or liver fibroblasts. In some embodiments where the cell monolayer comprises an endothelial cell monolayer, the condition of the endothelial cell monolayer can be assessed by staining for any art-recognized cell-cell adhesion markers in endothelial cells, *e.g*., but not limited to VE-cadherin. In some embodiments, the endothelial cell monolayer can further comprise macrophagic kupffer cells, hepatic stellate cells, and/or liver fibroblasts.

In some embodiments of various aspects described herein, the device can further comprise a monolayer of endothelial cells adhered on the second side of the membrane. The endothelial cells can be derived from vascular endothelial cells and/or lymphatic endothelial cells. In some embodiments, the endothelial cells can be derived from the same tissue origin as the tissue-specific cells cultured in the first chamber. In some embodiments, the endothelial cell monolayer can further comprise macrophagic kupffer cells, hepatic stellate cells, and/or liver fibroblasts.

### Exemplary liver-on-a-chip devices

The inventors have developed liver-on-a-chip devices based on the basic design of an organ-on-a-chip as described in the U.S. Patent No. 8,647,861, and the International Patent App. No. PCT/US2014/071611, and also optimized culture conditions to provide long-term hepatic culture with physiologically relevant hepatic function (*e.g*., albumin and/or urea secretion, and/or CYP 450 metabolic capacity) for a human model. Also described herein for reference are devices and optimized culture conditions for different animal models, *e.g*., rats, and dogs.

Accordingly, some aspects described herein relate to devices for simulating a function of a liver tissue (also referred to as "liver-on-a-chip device"). The liver-on-a-chip devices described herein can be used to simulate at least one or more (*e.g*., 1, 2, 3, 4, 5 or more) phenotypes and/or functions of a liver tissue. For examples, in some embodiments, the liver-on-a-chip devices described herein can simulate at least one or more of the phenotypes and/or functions of a liver tissue selected from the group consisting of (i) cuboidal morphology; (ii) a substantially constant level of albumin secretion over a period of time (*e.g*., at least 2 weeks, at least 4 weeks or longer); (iii) a substantially constant level of urea synthesis over a period of time (*e.g*., at least 2 weeks, at least 4 weeks or longer); (iv) at least a 2-fold increase (including, *e.g*., at least 3-fold increase or higher) in activity of CYP450 drug metabolizing enzyme in the presence of an agent that induces CYP450 drug metabolizing enzyme; (v) formation of a bile canaliculus network and active transport by multidrug resistance-associated protein 2 (MRP2); (vi) a level of alanine transaminase (ALT) activity and/or expression; (vii) level of aspartate transaminase (AST) activity and/or expression; (viii) maintenance of glutathione level, and a combination of two or more thereof. Methods for characterizing these phenotypes and/or functions of a liver tissue are known in the art. For example, to measure CYP450 (cytochrome P450) activity, one can perform mass spectroscopy analysis of specific metabolite(s) of an agent/compound exposed to liver cells in the chamber, and/or a luminescent assay that provides a luminogenic substrate for CYP enzyme to act on, which in turn produces a detectable luciferin product (e.g., P450-Glo^{™} assays from Promega). Immunocytochemistry, proteome and/or transcriptome analysis can be used to reveal differences in drug transporting proteins as well as drug metabolizing enzymes. Bile canalicular network formation can be detected, for example, by staining, with 5-(and-6)-carboxy-2',7'-dichloro- fluorescein diacetate (CDFDA, Invitrogen).

In one aspect, the liver-on-a-chip device comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side comprises an extracellular matrix composition and hepatocytes adhered on the extracellular matrix composition. In some embodiments, the extracellular matrix composition comprises fibronectin, collagen I and collagen IV, or functional fragments or variants thereof. In some instances, Matrigel is the preferred ECM. In the case of the human liver cells, collagen I may be used.

In some embodiments, the extracellular matrix composition comprises an ECM coating layer that comprise, essentially consist of, or consist of fibronectin, collagen I, and collagen IV. In some embodiments, the fibronectin component can be present at about 30% (w/w) to about 70% (w/w) of the extracellular matrix components. In one embodiment, the fibronectin component can be present at about 50% (w/w) of the extracellular matrix components. In some embodiments, the collagen IV component can be present at about 20% (w/w) to about 60% (w/w) of the extracellular matrix components. In one embodiment, the collagen IV component can be present at about 40% (w/w) of the extracellular matrix components. In some embodiments, the collagen I component can be present at about 1% (w/w) to about 20% (w/w) of the extracellular matrix components. In one embodiment, the collagen I component can be present at about 10% (w/w) of the extracellular matrix components.

In some embodiments, the total extracellular matrix components can be applied to a membrane surface (*e.g*., the first side of the membrane) in a concentration of between about 0.05 mg/mL to about 5 mg/mL. In some embodiments, the total concentration of the extracellular matrix components applied to the membrane surface (*e.g*., the first side of the membrane) can range from 0.1 mg/mL to about 5 mg/mL. In some embodiments, the total concentration of the extracellular matrix components applied to the membrane surface (*e.g*., the first side of the membrane) can range from 1 mg/mL to about 3 mg/mL.

In one embodiment, the ECM coating solution can comprise, essentially consist of, or consist of fibronectin of about 0.5 mg/mL, collagen IV of about 0.4 mg/mL, and collagen I of about 0.1 mg/mL.

In some embodiments, the extracellular matrix composition can further comprise an ECM overlay composition described herein. In some embodiments, the ECM overlay composition can comprise, essentially consist of, or consist of a protein mixture derived from a basement membrane and/or collagen I. In some embodiments, the ECM overlay composition can comprise, essentially consist of, or consist of a protein mixture derived from a basement membrane and/or collagen I, and fibronectin or a functional fragment or variant thereof. In one embodiment, the protein mixture derived from a basement membrane comprises Matrigel^{™}.

In some embodiments where the ECM overlay composition comprises, essentially consists of, or consists of a protein mixture derived from a basement membrane (*e.g*., MatrigelTM) and/or collagen I, and fibronectin or a functional fragment or variant thereof. In some embodiments, the basement membrane-derived protein mixture and/or collagen I can be present at about 50% to about 99.9% of the total extracellular matrix components. In some embodiments, the fibronectin component can be present at about 0.1% to about 50% of the total extracellular matrix components.

In some embodiments, the total extracellular matrix components can be applied over the ECM coating layer described herein to form an ECM overlay composition in a concentration of between about 50 µg/mL to about 10 mg/mL. In some embodiments, the total concentration of the extracellular matrix components applied over the ECM coating layer can range from 100 µg/mL to about 10 mg/mL. In some embodiments, the total concentration of the extracellular matrix components applied over the ECM coating layer can be about 250 µg/mL.

In some embodiments of this aspect and other aspects described herein, the second side of the membrane can comprise a monolayer of endothelial cells adhered thereon. In some embodiments, the monolayer of endothelial cells can comprise a monolayer of liver sinusoidal endothelial cells adhered thereon. Sinusoidal endothelial cells are generally derived from sinusoidal blood vessels with fenestrated, discontinuous endothelium, and can be identified by the presence of at least one or more markers, including, *e.g*., but not limited to CD31, CD32, and/or CD32b. Other art-recognized markers for sinusoidal endothelial cells, e.g., Stab-1, Stab-2, L-SIGN, MRC1, VE-Cadherin, and a combination of two or more thereof, can also be used. In some examples, for reference, other endothelial cells can also be used.

In some embodiments, the second side of the membrane and/or the second chamber comprising endothelial cells can further comprise macrophagic kupffer cells, hepatic stellate cells, and/or liver fibroblasts.

The inventors have also successfully created a co-culture microfluidic device to simulate a function of a dog liver tissue. Described herein, for reference, is a device for simulating a function of a dog liver tissue. The device comprises: (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. The first side of the membrane has dog hepatocytes adhered thereon and the second side has dog liver sinusoidal endothelial cells adhered thereon.

In some instances, the first side of the membrane can comprise an extracellular matrix composition. In some instances, the extracellular matrix can comprise an ECM coating of collagen I. The collagen I concentration can range from about 50 µg/ml to about 500 µg/mL. In one instances, the collagen I concentration can be about 200 µg/ml. In some instances, the extracellular matrix can comprise an ECM coating of collagen I (approximately 100-200 ug/mL). In other instances, it can comprise a Matrigel overlay (e.g., 250ug/mL).

In some instances, the extracellular matrix can further comprise an ECM overlay composition as described herein where dog hepatocytes are cultured between the ECM coating layer and the ECM overlay composition.

In some instances, the first side of the membrane can further comprise other cell types (e.g., non-parenchymal liver cells) including, but not limited to cholangiocytes (biliary endothelial cells) and/or liver fibroblasts, that are generally present in a liver in vivo cultured between the ECM coating layer and the ECM overlay layer.

In some instances, the second side of the membrane can further comprise macrophagic kupffer cells, hepatic stellate cells, and/or liver fibroblasts.

In some embodiments of various aspects described herein, the devices can comprise a flowing culture medium in the first chamber and/or the second chamber, wherein the flowing culture medium generates a shear stress. The shear stress can be a physiological or pathological shear stress, or a shear stress that does not adversely affect cell viability. In some embodiments, culture media flowing in the first chamber and the second chamber can generate different shear stresses on respective cells cultured therein. In some embodiments, the culture medium flowing in the first chamber in which hepatocytes are cultured can generate a shear stress of more than 5 dyne/cm², no more than 4 dyne/cm², no more than 3 dyne/cm², no more than 2 dyne/cm², no more than 1 dyne/cm², no more than 0.5 dyne/cm², no more than 0.1 dyne/cm², no more than 0.05 dyne/cm², no more than 0.04 dyne/cm², no more than 0.03 dyne/cm², no more than 0.02 dyne/cm², no more than 0.01 dyne/cm². The inventors have discovered that addition of low shear stress improved liver cell morphology and long-term viability over static cultures. In some embodiments where cells, e.g., endothelial cells, in the second chamber can tolerate higher shear stress, the culture medium flowing in the second chamber can be greater than 5 dyne/cm² or higher.

In some embodiments, the device can comprise a first flowing culture medium in the first chamber and a second flowing culture medium in the second chamber. The shear stress created by the first flowing culture medium in the first chamber can be less than that created in the second chamber by the second flowing culture medium. For example, the shear stress created by the first flowing culture medium in the first chamber can be no more than 60%, including, *e.g*., no more than 50%, no more than 40%, no more than 30%, no more than 20%, no more than 10%, of the shear stress generated by the second flowing culture medium in the second chamber. In one embodiment, the shear stress created by the first flowing culture medium in the first chamber can be about 50% of the shear stress created in the second chamber. In one embodiment, the shear stress created in the first chamber can be about 0.025 dyne/cm². In one embodiment, the shear stress created in the second chamber can be about 0.05 dyne/cm².

Based on the viscosity of the culture medium and/or dimensions of the chambers, one of skill in the art can determine appropriate flow rates of culture medium through the chambers to achieve desired shear stress. In some embodiments, the flow rate of the culture medium through the first chamber can range from about 5 µL/hr to about 50 µL/hr. In some embodiments, the flow rate of the culture medium through the second chamber can range from about 15 µL/hr to about 150 µL/hr.

***Chamber-defining first structure and second structure:*** In accordance with various aspects of certain embodiments described herein, the first structure defines a first chamber, and the second structure defines a second chamber. While the first chamber and the second chamber can be in any geometry or three-dimensional structure, in some embodiments, the first chamber and the second chamber can be configured to be form channels. **Figs. 2A-2B**, 7A-7B, and **29A-29B** illustrate a perspective view of the devices in accordance with some embodiments described herein. For example, as shown in **Figs. 29A-29B**, the device 200 can include a body 202 comprising a first structure 204 and a second structure 206 in accordance with an embodiment. The body 202 can be made of an elastomeric material, although the body can be alternatively made of a non-elastomeric material, or a combination of elastomeric and non-elastomeric materials. It should be noted that the microchannel design 203 is only exemplary and not limited to the configuration shown in **Figs. 29A-29B****.** While operating chambers 252 (*e.g*., as a pneumatics means to actuate the membrane 208, see the International Appl. No. PCT/US2009/050830 for further details of the operating chambers) are shown in **Figs. 29A-29B**, they are not required in all of the embodiments described herein. In some embodiments, the devices do not comprise operating chambers on either side of the first chamber and the second chamber. For example, **Fig. 7B** shows a device that does not have an operating channel on either side of the first chamber and the second chamber. In other embodiments, the devices described herein can be configured to provide other means to actuate the membrane, *e.g*., as described in the International Pat. Appl. No. PCT/US2014/071570.

In some embodiments, various organ chip devices described in the International Patent Application Nos. PCT/US2009/050830; PCT/US2012/026934; PCT/US2012/068725; PCT/US2012/068766; PCT/US2014/071611; and PCT/US2014/071570, can be modified to form the devices described herein. For example, the organ chip devices described in those patent applications can be modified in accordance with the devices described herein.

The first structure 204 and/or second structure 206 can be fabricated from a rigid material, an elastomeric material, or a combination thereof. As used herein, the term "rigid" refers to a material that is stiff and does not bend easily, or maintains very close to its original form after pressure has been applied to it. The term "elastomeric" as used herein refers to a material or a composite material that is not rigid as defined herein. An elastomeric material is generally moldable and curable, and has an elastic property that enables the material to at least partially deform (*e.g*., stretching, expanding, contracting, retracting, compressing, twisting, and/or bending) when subjected to a mechanical force or pressure and partially or completely resume its original form or position in the absence of the mechanical force or pressure. In some embodiments, the term "elastomeric" can also refer to a material that is flexible/stretchable but does not resume its original form or position after pressure has been applied to it and removed thereafter. The terms "elastomeric" and "flexible" are interchangeably used herein.

In some embodiments, the material used to make the first structure and/or second structure or at least the portion of the first structure 204 and/or second structure 206 that is in contact with a gaseous and/or liquid fluid can comprise a biocompatible polymer or polymer blend, including but not limited to, polydimethylsiloxane (PDMS), polyurethane, polyimide, styrene-ethylene-butylene-styrene (SEBS), polypropylene, polycarbonate, cyclic polyolefin polymer/copolymer (COP/COC), or any combinations thereof. As used herein, the term "biocompatible" refers to any material that does not deteriorate appreciably and does not induce a significant immune response or deleterious tissue reaction, *e.g.,* toxic reaction or significant irritation, over time when implanted into or placed adjacent to the biological tissue of a subject, or induce blood clotting or coagulation when it comes in contact with blood.

Additionally or alternatively, at least a portion of the first structure 204 and/or second structure 206 can be made of non-flexible or rigid materials like glass, silicon, hard plastic, metal, or any combinations thereof.

The membrane 208 can be made of the same material as the first structure 204 and/or second structure 206 or a material that is different from the first structure 204 and/or second structure 206 of the devices described herein. In some embodiments, the membrane 208 can be made of a rigid material. In some embodiments, the membrane is a thermoplastic rigid material. Examples of rigid materials that can be used for fabrication of the membrane include, but are not limited to, polyester, polycarbonate or a combination thereof. In some embodiments, the membrane 208 can comprise a flexible material, *e.g*., but not limited to PDMS. Additional information about the membrane is further described below.

In some embodiments, the first structure and/or second structure of the device and/or the membrane can comprise or is composed of an extracellular matrix polymer, gel, and/or scaffold. Any extracellular matrix can be used herein, including, but not limited to, silk, chitosan, elastin, collagen, proteoglycans, hyaluronic acid, collagen, fibrin, and any combinations thereof.

The device in **Fig. 29A** can comprise a plurality of access ports 205. In addition, the branched configuration 203 can comprise a tissue-tissue interface simulation region (membrane 208 in **Fig. 29B**) where cell behavior and/or passage of gases, chemicals, molecules, particulates and cells are monitored. **Fig. 29B** illustrates an exploded view of the device in accordance with an embodiment. In one embodiment, the body 202 of the device 200 comprises a first outer body portion (first structure) 204, a second outer body portion (second structure) 206 and an intermediary membrane 208 configured to be mounted between the first and second outer body portions 204, 206 when the portions 204, 206 are mounted to one another to form the overall body.

The first outer body portion or first structure 204 can have a thickness of any dimension, depending, in part, on the height of the first chamber 204. In some embodiments, the thickness of the first outer body portion or first structure 204 can be about 1 mm to about 100 mm, or about 2 mm to about 75 mm, or about 3 mm to about 50 mm, or about 3 mm to about 25 mm. In some embodiments, the first outer body portion or first structure 204 can have a thickness that is more than the height of the first chamber by no more than 5 mm, no more than 4 mm, no more than 3 mm, no more than 2 mm, no more than 1 mm, no more than 500 microns, no more than 400 microns, no more than 300 microns, no more than 200 microns, no more than 100 microns, no more than 70 microns or less. In some embodiments, it is desirable to keep the first outer body portion or first structure 204 as thin as possible such that cells on the membrane can be visualized or detected by microscopic, spectroscopic, and/or electrical sensing methods.

The second outer body portion or second structure 206 can have a thickness of any dimension, depending, in part, on the height of the second chamber 206. In some embodiments, the thickness of the second outer body portion or second structure 206 can be about 50 µm to about 10 mm, or about 75 µm to about 8 mm, or about 100 µm to about 5 mm, or about 200 µm to about 2.5 mm. In one embodiment, the thickness of the second outer body portion or second structure 206 can be about 1 mm to about 1.5 mm. In one embodiment, the thickness of the second outer body portion or second structure 206 can be about 0.2 mm to about 0.5 mm. In some embodiments, the second outer first structure and/or second structure portion 206 can have a thickness that is more than the height of the second chamber by no more than 5mm, no more than 4 mm, no more than 3 mm, no more than 2 mm, no more than 1 mm, no more than 500 microns, no more than 400 microns, no more than 300 microns, no more than 200 microns, no more than 100 microns, no more than 70 microns or less. In some embodiments, it is desirable to keep the second outer body portion or second structure 206 as thin as possible such that cells on the membrane can be visualized or detected by microscopic, spectroscopic, and/or electrical sensing methods.

In some embodiments, the first chamber and the second chamber can each independently comprise a channel. The channel(s) can be substantially linear or they can be non-linear. In some embodiments, the channels are not limited to straight or linear channels and can comprise curved, angled, or otherwise non-linear channels. It is to be further understood that a first portion of a channel can be straight, and a second portion of the same channel can be curved, angled, or otherwise non-linear. Without wishing to be bound by a theory, a non-linear channel can increase the ratio of culture area to device area, thereby providing a larger surface area for cells to grow. This can also allow for a higher amount or density of cells in the channel.

**Fig. 29B** illustrates an exploded view of the device in accordance with an embodiment. As shown in **Fig. 29B****,** the first outer body portion or first structure 204 includes one or more inlet fluid ports 210 in communication with one or more corresponding inlet apertures 211 located on an outer surface of the first structure 204. The device 200 can be connected to a fluid source via the inlet aperture 211 in which fluid travels from the fluid source into the device 200 through the inlet fluid port 210.

Additionally, the first outer body portion or first structure 204 can include one or more outlet fluid ports 212 in communication with one or more corresponding outlet apertures 215 on the outer surface of the first structure 204. In some embodiments, a fluid passing through the device 200 can exit the device to a fluid collector or other appropriate component via the corresponding outlet aperture 215. It should be noted that the device 200 can be set up such that the fluid port 210 is an outlet and fluid port 212 is an inlet.

In some embodiments, as shown in **Fig. 29B****,** the device 200 can comprise an inlet channel 225 connecting an inlet fluid port 210 to the first chamber 204. The inlet channels and inlet ports can be used to introduce cells, agents (*e.g*., but not limited to, stimulants, drug candidate, particulates), air flow, and/or cell culture media into the first chamber 204.

The device 200 can also comprise an outlet channel 227 connecting an outlet fluid port 212 to the first chamber 204. The outlet channels and outlet ports can also be used to introduce cells, agents (*e.g*., but not limited to, stimulants, drug candidate, particulates), air flow, and/or cell culture media into the first chamber 204.

Although the inlet and outlet apertures 211, 215 are shown on the top surface of the first structure 204 and are located perpendicular to the inlet and outlet channels 225, 227, one or more of the apertures 211, 215 can be located on one or more lateral surfaces of the first structure and/or second structure such that at least one of the inlet and outlet apertures 211, 215 can be in-plane with the inlet and/or outlet channels 225, 227, respectively, and/or be oriented at an angle from the plane of the inlet and/or outlet channels 225, 227.

In another embodiment, the fluid passing between the inlet and outlet fluid ports can be shared between the first chamber 204 and second chamber 206. In either embodiment, characteristics of the fluid flow, such as flow rate, fluid type and/or composition, and the like, passing through the first chamber 204 can be controllable independently of fluid flow characteristics through the second chamber 206 and vice versa.

In some embodiments, while not necessary, the first structure 204 can include one or more pressure inlet ports 214 and one or more pressure outlet ports 216 in which the inlet ports 214 are in communication with corresponding apertures 217 located on the outer surface of the device 200. Although the inlet and outlet apertures are shown on the top surface of the first structure 204, one or more of the apertures can alternatively be located on one or more lateral sides of the first structure and/or second structure. In operation, one or more pressure tubes (not shown) connected to an external force source (*e.g*., pressure source) can provide positive or negative pressure to the device via the apertures 217. Additionally, pressure tubes (not shown) can be connected to the device 200 to remove the pressurized fluid from the outlet port 216 via the apertures 223. It should be noted that the device 200 can be set up such that the pressure port 214 is an outlet and pressure port 216 is an inlet. It should be noted that although the pressure apertures 217, 223 are shown on the top surface of the first structure 204, one or more of the pressure apertures 217, 223 can be located on one or more side surfaces of the first structure 204.

Referring to **Fig. 29B****,** in some embodiments, the second structure 206 can include one or more inlet fluid ports 218 and one or more outlet fluid ports 220. As shown in **Fig. 29B****,** the inlet fluid port 218 is in communication with aperture 219 and outlet fluid port 220 is in communication with aperture 221, whereby the apertures 219 and 221 are located on the outer surface of the second structure 206. Although the inlet and outlet apertures are shown on the surface of the second structure, one or more of the apertures can be alternatively located on one or more lateral sides of the second structure.

As with the first outer body portion or first structure 204 described above, one or more fluid tubes connected to a fluid source can be coupled to the aperture 219 to provide fluid to the device 200 via port 218. Additionally, fluid can exit the device 200 via the outlet port 220 and outlet aperture 221 to a fluid reservoir/collector or other component. It should be noted that the device 200 can be set up such that the fluid port 218 is an outlet and fluid port 220 is an inlet.

In some embodiments, the second outer body portion and/or second structure 206 can include one or more pressure inlet ports 222 and one or more pressure outlet ports 224. In some embodiments, the pressure inlet ports 222 can be in communication with apertures 227 and pressure outlet ports 224 are in communication with apertures 229, whereby apertures 227 and 229 are located on the outer surface of the second structure 206. Although the inlet and outlet apertures are shown on the bottom surface of the second structure 206, one or more of the apertures can be alternatively located on one or more lateral sides of the second structure. Pressure tubes connected to an external force source (*e.g*., pressure source) can be engaged with ports 222 and 224 via corresponding apertures 227 and 229. It should be noted that the device 200 can be set up such that the pressure port 222 is an outlet and fluid port 224 is an inlet.

In some embodiments where the operating channels (*e.g*., 252 shown in **Fig. 29A**) are not mandatory, the first structure 204 does not require any pressure inlet port 214, pressure outlet port 216. Similarly, the second structure 206 does not require any pressure inlet port 222 or pressure outlet port 224.

In an embodiment, the membrane 208 is mounted between the first structure 204 and the second structure 206, whereby the membrane 208 is located within the first structure 204 and/or second structure 206 of the device 200 (see, *e.g.*, **Fig. 7B**). In an embodiment, the membrane 208 is a made of a material having a plurality of pores or apertures therethrough, whereby molecules, cells, fluid or any media is capable of passing through the membrane 208 via one or more pores in the membrane 208. As discussed in more detail below, the membrane 208 in one embodiment can be made of a material which allows the membrane 208 to undergo stress and/or strain in response to an external force (*e.g*., cyclic stretching or pressure). In one embodiment, the membrane 208 can be made of a material which allows the membrane 208 to undergo stress and/or strain in response to pressure differentials present between the first chamber 204, the second chamber 206 and the operating channels 252. Alternatively, the membrane 208 is relatively inelastic or rigid in which the membrane 208 undergoes minimal or no movement.

In some embodiments where the device simulates a function of a liver tissue, the membrane can be rigid.

The first chamber 204 and/or the second chamber 206 can have a length suited to the need of an application (*e.g*., a physiological system to be modeled), desirable size of the device, and/or desirable size of the view of field. In some embodiments, the first chamber 204 and/or the second chamber 206 can have a length of about 0.5 cm to about 10 cm. In one embodiment, the first chamber 204 and/or the second chamber 206 can have a length of about 1 cm to about 3 cm. In one embodiment, the first chamber 204 and/or the second chamber 206 can have a length of about 2 cm.

The width of the first chamber and/or the second chamber can vary with desired cell growth surface area. The first chamber 204 and the second chamber 206 can each have a range of width dimension (shown as W in **Fig. 7B**) between 100 microns and 50 mm, or between 200 microns and 10 mm, or between 200 microns and 1500 microns, or between 400 microns and 1 mm, or between 50 microns and 2mm, or between 100 microns and 5 mm. In some embodiments, the first chamber 204 and the second chamber 206 can each have a width of about 500 microns to about 2 mm. In some embodiments, the first chamber 204 and the second chamber 206 can each have a width of about 1 mm.

In some embodiments, the widths of the first chamber and the second chamber can be configured to be different, with the centers of the chambers aligned or not aligned. In some embodiments, the channel heights, widths, and/or cross sections can vary along the length of the devices described herein.

The heights of the first chamber and the second chamber can vary to suit the needs of desired applications (*e.g*., to provide a low shear stress, and/or to accommodate cell size). The first chamber and the second chamber of the devices described herein can have the same heights or different heights. In some embodiments, the height of the second chamber 206 can be substantially the same as the height of the first chamber 204.

In some embodiments, the height of at least a length portion of the first chamber 204 (*e.g*., the length portion where the cells are designated to grow) can be substantially greater than the height of the second chamber 206 within the same length portion. For example, the height ratio of the first chamber to the second chamber can be greater than 1:1, including, for example, greater than 1.1:1, 1.5:1, 2:1, 2.5:1, 3:1, 3.5:1, 4:1, 4.5:1, 5:1, 6:1, 7:1, 8:1, 9:1, 10:1, 11:1, 12:1, 13:1, 14:1, 15:1, 16:1, 17:1, 18:1, 19:1,20:1, 25:1, 30:1, 35:1, 40:1, 45:1, 50:1. In some embodiments, the height ratio of the first chamber to the second chamber can range from 1.1:1 to about 50:1, or from about 2.5:1 to about 50:1, or from 2.5 to about 25:1, or from about 2.5:1 to about 15:1. In one embodiment, the height ratio of the first chamber to the second chamber ranges from about 1:1 to about 20:1. In one embodiment, the height ratio of the first chamber to the second chamber ranges from about 1:1 to about 15:1. In one embodiment, the height ratio of the first chamber to the second chamber can be about 10:1.

The height of the first chamber 204 can be of any dimension, *e.g*., sufficient to accommodate cell height and/or to permit a low shear flow. For example, in some embodiments, the height of the first chamber can range from about 100 µm to about 50 mm, about 200 µm to about 10 mm, about 500 µm to about 5 mm, or about 750 µm to about 2 mm. In one embodiment, the height of the first chamber can be about 150 µm. In one embodiment, the height of the first chamber can be about 1 mm.

The height of the second chamber 206 can be of any dimension provided that the flow rate and/or shear stress of a medium flowing in the second chamber can be maintained within a physiological range, or does not cause any adverse effect to the cells. In some embodiments, the height of the second chamber can range from 20 µm to about 1 mm, or about 50 µm to about 500 µm, or about 75 µm to about 400 µm, or about 100 µm to about 300 µm. In one embodiment, the height of the second chamber can be about 150 µm. In one embodiment, the height of the second chamber can be about 100 µm.

The first chamber and/or the second chamber can have a uniform height along the length of the first chamber and/or the second chamber, respectively. Alternatively, the first chamber and/or the second chamber can each independently have a varying height along the length of the first chamber and/or the second chamber, respectively. For example, a length portion of the first chamber can be substantially taller than the same length portion of the second chamber, while the rest of the first chamber can have a height comparable to or even smaller than the height of the second chamber.

In some embodiments, the first structure and/or second structure of the devices described herein can be further adapted to provide mechanical modulation of the membrane. Mechanical modulation of the membrane can include any movement of the membrane that is parallel to and/or perpendicular to the force/pressure applied to the membrane, including, but are not limited to, stretching, bending, compressing, vibrating, contracting, waving, or any combinations thereof. Different designs and/or approaches to provide mechanical modulation of the membrane between two chambers have been described, *e.g*., in the International Patent App. Nos. PCT/US2009/050830, and PCT/US2014/071570, and can be adapted herein to modulate the membrane in the devices described herein.

In some embodiments, the devices described herein can be placed in or secured to a cartridge. In accordance with some embodiments of some aspects described herein, the device can be integrated into a cartridge and form a monolithic part. Some examples of a cartridge are described in the International Patent App. No. PCT/US2014/047694. The cartridge can be placed into and removed from a cartridge holder that can establish fluidic connections upon or after placement and optionally seal the fluidic connections upon removal. In some embodiments, the cartridge can be incorporated or integrated with at least one sensor, which can be placed in direct or indirect contact with a fluid flowing through a specific portion of the cartridge during operation. In some embodiments, the cartridge can be incorporated or integrated with at least one electric or electronic circuit, for example, in the form of a printed circuit board or flexible circuit. In accordance with some embodiments of some aspects described herein, the cartridge can comprise a gasketing embossment to provide fluidic routing.

In some embodiments, the cartridge and/or the device described herein can comprise a barcode. The barcode can be unique to types and/or status of the cells present on the membrane. Thus, the barcode can be used as an identifier of each device adapted to mimic function of at least a portion of a specific tissue and/or a specific tissue-specific condition. Prior to operation, the barcode of the cartridge can be read by an instrument so that the cartridge can be placed and/or aligned in a cartridge holder for proper fluidic connections and/or proper association of the data obtained during operation of each device. In some embodiments, data obtained from each device include, but are not limited to, cell response, immune cell recruitment, intracellular protein expression, gene expression, cytokine/chemokine expression, cell morphology, functional data such as effectiveness of an endothelium as a barrier, concentration change of an agent that is introduced into the device, or any combinations thereof.

In some embodiments, the device can be connected to the cartridge by an interconnect adapter that connects some or all of the inlet and outlet ports of the device to microfluidic channels or ports on the cartridge. Some examples interconnect adapters are disclosed in U.S. Provisional Application No. 61/839,702, filed on June 26, 2013, and the International Patent Application No. PCT/US2014/044417 filed June 26, 2014. The interconnect adapter can include one or more nozzles having fluidic channels that can be received by ports of the device described herein. The interconnect adapter can also include nozzles having fluidic channels that can be received by ports of the cartridge.

In some embodiments, the interconnect adaptor can comprise a septum interconnector that can permit the ports of the device to establish transient fluidic connection during operation, and provide a sealing of the fluidic connections when not in use, thus minimizing contamination of the cells and the device. Some examples of a septum interconnector are described in U.S. Provisional Application No. 61/810,944 filed April 11, 2013.

***Membrane:*** The membrane 208 is oriented along a plane 208P parallel to the x-y plane between the first chamber 204 and the second chamber 206 as shown in **Fig. 7B****.** It should be noted that although one membrane 208 is shown in **Fig. 7B**, more than one membrane 208 can be configured in devices which comprise more than two chambers.

The membrane separating the first chamber and the second chamber in the devices described herein can be porous (*e.g.,* permeable or selectively permeable), non-porous (*e.g.,* non-permeable), rigid, flexible, elastic or any combinations thereof. Accordingly, the membrane 208 can have a porosity of about 0% to about 99%. As used herein, the term "porosity" is a measure of total void space (*e.g*., through-holes, openings, interstitial spaces, and/or hollow conduits) in a material, and is a fraction of volume of total voids over the total volume, as a percentage between 0 and 100% (or between 0 and 1). A membrane with substantially zero porosity is non-porous or non-permeable.

As used interchangeably herein, the terms "non-porous" and "non-permeable" refer to a material that does not allow any molecule or substance to pass through.

In some embodiments, the membrane can be porous and thus allow molecules, cells, particulates, chemicals and/or media to migrate or transfer between the first chamber 204 and the second chamber 206 via the membrane 208 from the first chamber 204 to the second chamber 206 or vice versa.

As used herein, the term "porous" generally refers to a material that is permeable or selectively permeable. The term "permeable" as used herein means a material that permits passage of a fluid (*e.g*., liquid or gas), a molecule, a whole living cell and/or at least a portion of a whole living cell, *e.g.,* for formation of cell-cell contacts. The term "selectively permeable" as used herein refers to a material that permits passage of one or more target group or species, but act as a barrier to non-target groups or species. For example, a selectively-permeable membrane can allow passage of a fluid (*e.g.*, liquid and/or gas), nutrients, wastes, cytokines, and/or chemokines from one side of the membrane to another side of the membrane, but does not allow whole living cells to pass therethrough. In some embodiments, a selectively-permeable membrane can allow certain cell types to pass therethrough but not other cell types.

The permeability of the membrane to individual matter/species can be determined based on a number of factors, including, *e.g.,* material property of the membrane (*e.g*., pore size, and/or porosity), interaction and/or affinity between the membrane material and individual species/matter, individual species size, concentration gradient of individual species between both sides of the membrane, elasticity of individual species, and/or any combinations thereof.

A porous membrane can have through-holes or pore apertures extending vertically and/or laterally between two surfaces 208A and 208B of the membrane (**Fig. 29B**), and/or a connected network of pores or void spaces (which can, for example, be openings, interstitial spaces or hollow conduits) throughout its volume. The porous nature of the membrane can be contributed by an inherent physical property of the selected membrane material, and/or introduction of conduits, apertures and/or holes into the membrane material.

In some embodiments, a membrane can be a porous scaffold or a mesh. In some embodiments, the porous scaffold or mesh can be made from at least one extracellular matrix polymer (*e.g*., but not limited to collagen, alginate, gelatin, fibrin, laminin, hydroxyapatite, hyaluronic acid, fibroin, and/or chitosan), and/or a biopolymer or biocompatible material (*e.g*., but not limited to, polydimethylsiloxane (PDMS), polyurethane, styrene-ethylene-butylene-styrene (SEBS), poly(hydroxyethylmethacrylate) (pHEMA), polyethylene glycol, polyvinyl alcohol and/or any biocompatible material described herein for fabrication of the device first structure and/or second structure) by any methods known in the art, including, *e.g*., but not limited to, electrospinning, cryogelation, evaporative casting, and/or 3D printing. See, *e.g*., Sun et al. (2012) "Direct-Write Assembly of 3D Silk/Hydroxyapatite Scaffolds for Bone Co-Cultures." Advanced Healthcare Materials, no. 1: 729-735; Shepherd et al. (2011) "3D Microperiodic Hydrogel Scaffolds for Robust Neuronal Cultures." Advanced Functional Materials 21: 47-54; and Barry III et al. (2009) "Direct-Write Assembly of 3D Hydrogel Scaffolds for Guided Cell Growth." Advanced Materials 21: 1-4, for examples of a 3D biopolymer scaffold or mesh that can be used as a membrane in the device described herein.

In some embodiments, a membrane can comprise an elastomeric portion fabricated from a styrenic block copolymer-comprising composition, *e.g*., as described in the International Pat. App. No. PCT/US2014/071611, can be adopted in the devices described herein. In some embodiments, the styrenic block copolymer-comprising composition can comprise SEBS and polypropylene.

In some embodiments, a membrane can be a hydrogel or a gel comprising an extracellular matrix polymer, and/or a biopolymer or biocompatible material. In some embodiments, the hydrogel or gel can be embedded with a conduit network, *e.g.,* to promote fluid and/or molecule transport. See, *e.g.*, Wu et al. (2011) "Omnidirectional Printing of 3D Microvascular Networks." Advanced Materials 23: H178-H183; and Wu et al. (2010) "Direct-write assembly of biomimetic microvascular networks for efficient fluid transport." Soft Matter 6: 739-742, for example methods of introducing a conduit network into a gel material.

In some embodiments, a porous membrane can be a solid biocompatible material or polymer that is inherently permeable to at least one matter/species (*e.g*., gas molecules) and/or permits formation of cell-cell contacts. In some embodiments, through-holes or apertures can be introduced into the solid biocompatible material or polymer, *e.g*., to enhance fluid/molecule transport and/or cell migration. In one embodiment, through-holes or apertures can be cut or etched through the solid biocompatible material such that the through-holes or apertures extend vertically and/or laterally between the two surfaces of the membrane 208A and 208B. It should also be noted that the pores can additionally or alternatively incorporate slits or other shaped apertures along at least a portion of the membrane 208 which allow cells, particulates, chemicals and/or fluids to pass through the membrane 208 from one section of the central channel to the other.

The pores of the membrane (including pore apertures extending through the membrane 208 from the top 208A to bottom 208B surfaces thereof and/or a connected network of void space within the membrane 208) can have a cross-section of any size and/or shape. For example, the pores can have a pentagonal, circular, hexagonal, square, elliptical, oval, diamond, and/or triangular shape.

The cross-section of the pores can have any width dimension provided that they permit desired molecules and/or cells to pass through the membrane. In some embodiments, the pore size of the membrane should be big enough to provide the cells sufficient access to nutrients present in a fluid medium flowing through the first chamber and/or the second chamber. In some embodiments, the pore size can be selected to permit passage of cells (*e.g*., immune cells and/or cancer cells) from one side of the membrane to the other. In some embodiments, the pore size can be selected to permit passage of nutrient molecules. In some embodiments, the width dimension of the pores can be selected to permit molecules, particulates and/or fluids to pass through the membrane 208 but prevent cells from passing through the membrane 208. In some embodiments, the width dimension of the pores can be selected to permit cells, molecules, particulates and/or fluids to pass through the membrane 208. Thus, the width dimension of the pores can be selected, in part, based on the sizes of the cells, molecules, and/or particulates of interest. In some embodiments, the width dimension of the pores (*e.g*., diameter of circular pores) can be in the range of 0.01 microns and 20 microns, or in one embodiment, approximately 0.1-15 microns, or approximately 1-10 microns. In one embodiment, the pores have a width of about 7 microns.

In an embodiment, the porous membrane 208 can be designed or surface patterned to include micro and/or nanoscopic patterns therein such as grooves and ridges, whereby any parameter or characteristic of the patterns can be designed to desired sizes, shapes, thicknesses, filling materials, and the like.

The membrane 208 can have any thickness to suit the needs of a target application. In some embodiments, the membrane can be configured to deform in a manner (*e.g*., stretching, retracting, compressing, twisting and/or waving) that simulates a physiological strain experienced by the cells in its native microenvironment. In these embodiments, a thinner membrane can provide more flexibility. In some embodiments, the membrane can be configured to provide a supporting structure to permit growth of a defined layer of cells thereon. Thus, in some embodiments, a thicker membrane can provide a greater mechanical support. In some embodiments, the thickness of the membrane 208 can range between 70 nanometers and 100 µm, or between 1 µm and 100 µm, or between 10 and 100 µm. In one embodiment, the thickness of the membrane 208 can range between 10 µm and 80 µm. In one embodiment, the thickness of the membrane 208 can range between 30 µm and 80 µm. In one embodiment, the thickness of the membrane 208 can be about 50 µm.

While the membrane 208 generally have a uniform thickness across the entire length or width, in some embodiments, the membrane 208 can be designed to include regions which have lesser or greater thicknesses than other regions in the membrane 208. The decreased thickness area(s) can run along the entire length or width of the membrane 208 or can alternatively be located at only certain locations of the membrane 208. The decreased thickness area can be present along the bottom surface of the membrane 208 (i.e. facing second chamber 206), or additionally/alternatively be on the opposing surface of the membrane 208 (i.e. facing second chamber 204). It should also be noted that at least portions of the membrane 208 can have one or more larger thickness areas relative to the rest of the membrane, and capable of having the same alternatives as the decreased thickness areas described above.

In some embodiments, the membrane can be coated with substances such as various cell adhesion promoting substances or ECM proteins, such as fibronectin, laminin, various collagen types, glycoproteins, vitronectin, elastins, fibrin, proteoglycans, heparin sulfate, chondroitin sulfate, keratin sulfate, hyaluronic acid, fibroin, chitosan, or any combinations thereof. In some embodiments, one or more cell adhesion molecules can be coated on one surface of the membrane 208 whereas another cell adhesion molecule can be applied to the opposing surface of the membrane 208, or both surfaces can be coated with the same cell adhesion molecules. In some embodiments, the ECMs, which can be ECMs produced by cells, such as primary cells or embryonic stem cells, and other compositions of matter are produced in a serum-free environment.

In an embodiment, one can coat the membrane with a cell adhesion factor and/or a positively-charged molecule that are bound to the membrane to improve cell attachment and stabilize cell growth. The positively charged molecule can be selected from the group consisting of polylysine, chitosan, poly(ethyleneimine) or acrylics polymerized from acrylamide or methacrylamide and incorporating positively-charged groups in the form of primary, secondary or tertiary amines, or quaternary salts. The cell adhesion factor can be added to the membrane and is fibronectin, laminin, various collagen types, glycoproteins, vitronectin, elastins, fibrin, proteoglycans, heparin sulfate, chondroitin sulfate, keratin sulfate, hyaluronic acid, tenascin, antibodies, aptamers, or fragments or analogs having a cell binding domain thereof. The positively-charged molecule and/or the cell adhesion factor can be covalently bound to the membrane. In another embodiment, the positively-charged molecule and/or the cell adhesion factor are covalently bound to one another and either the positively-charged molecule or the cell adhesion factor is covalently bound to the membrane. Also, the positively-charged molecule or the cell adhesion factor or both can be provided in the form of a stable coating non-covalently bound to the membrane.

In an embodiment, the cell attachment-promoting substances, matrix-forming formulations, and other compositions of matter are sterilized to prevent unwanted contamination. Sterilization can be accomplished, for example, by ultraviolet light, filtration, gas plasma, ozone, ethylene oxide, and/or heat. Antibiotics can also be added, particularly during incubation, to prevent the growth of bacteria, fungi and other undesired micro-organisms. Such antibiotics include, by way of non-limiting example, gentamicin, streptomycin, penicillin, amphotericin and ciprofloxacin.

In some embodiments, the membrane and/or other components of the devices described herein can be treated using gas plasma, charged particles, ultraviolet light, ozone, or any combinations thereof.

Using the devices described herein, one can study biotransformation, absorption, as well as drug clearance, metabolism, delivery, and toxicity. The activation of xenobiotics can also be studied. The bioavailability and transport of chemical and biological agents across epithelial layers as in a tissue or organ, *e.g*., lung, and endothelial layers as in blood vessels, and across the liver for drug metabolism can also be studied. The acute basal toxicity, acute local toxicity or acute organ-specific toxicity, teratogenicity, genotoxicity, carcinogenicity, and mutagenicity, of chemical agents can also be studied. Effects of infectious biological agents, biological weapons, harmful chemical agents and chemical weapons can also be detected and studied. Infectious diseases and the efficacy of chemical and biological agents to treat these diseases, as well as optimal dosage ranges for these agents, can be studied. The response of organs *in vivo* to chemical and biological agents, and the pharmacokinetics and pharmacodynamics of these agents can be detected and studied using the devices described herein. The impact of genetic content on response to the agents can be studied. The amount of protein and gene expression in response to chemical or biological agents can be determined. Changes in metabolism in response to chemical or biological agents can be studied as well using devices described herein.

In some embodiments, the devices described herein (e.g., a Liver-on-a-Chip) can be used to assess the clearance of a test compound. For clearance studies, the disappearance of a test compound can be measured (e.g. using mass spec) in the media of the top chamber, bottom chamber, or both chambers (divided by a membrane comprising liver cells).

For example, in accordance to one aspect of the invention, a Liver-on-Chip drug-metabolizing performance can be measured by i) disposing a substrate compound with known liver metabolites in the media of the top chamber, bottom chamber, or both chambers; and ii) measuring the amount of generated metabolite in the media of the top chamber, bottom chamber or both chambers (e.g. using mass spec). As is known in the art, the choice of the substrate and measured metabolite can help provide information on specific liver drug-metabolism enzymes (e.g. CYP450 isoforms, Phase II enzymes, etc.)

In some embodiments, the devices described herein (e.g., a Liver-on-a-Chip) can be used to assess the induction or inhibition potential of a test compound. For induction or inhibition studies a variety of tests are contemplated. For example, induction of CYP3A4 activity in the liver is one of main causes of drug-drug interactions, which is a mechanism to defend against exposure to drugs and toxin, but can also lead to unwanted side-effects (toxicity) or change the efficacy of a drug. A reliable and practical CYP3A induction assay with human hepatocytes in a 96-well format has been reported, where various 96-well plates with different basement membrane were evaluated using prototypical inducers, rifampicin, phenytoin, and carbamazepine. See Drug Metab. Dispo. (2010) Nov;38(1 1): 1912-6.

According to one aspect of the invention, the induction or inhibition potential of a test compound at a test concentration can be evaluated by i) disposing the test compound in the media of the top chamber, bottom chamber or both chambers at the test concentration; ii) exposing the device for a selected period of time; and iii) assessing the induction or inhibition of liver enzymes by comparing liver performance to a measurement performed before the test compound was applied, to a measurement performed on a Liver-on-Chip that was subjected to a lower concentration of test compound (or no test compound at all), or both. In some embodiments, the liver performance measurement can comprise an RNA expression level. In some embodiments, the liver performance measurement comprises assessing drug-metabolizing capacity.

In some embodiments, the devices described herein (e.g., a Liver-on-a-Chip) can be used to identify in vivo metabolites of a test compound or agent, and optionally the in vivo ratio of these metabolites. According to one aspect of the invention, in vivo metabolites can be identified by i) disposing a test compound or agent in the media of the top chamber, bottom chamber, or both chambers; and ii) measuring the concentration of metabolites in the media of the top chamber, bottom chamber, or both chambers. In some embodiments, the measuring of the concentration of metabolites comprises mass spectroscopy.

In some embodiments, the devices described herein (e.g., a Liver-on-a-Chip) can be used to identify the toxicity of a test compound or agent at a test concentration. According to one aspect of the disclosure, toxicity can be evaluated by i) disposing a test compound in the media of the top chamber, bottom chamber, or both chambers; and ii) measuring one or more toxicity endpoints selected from the list of leakage of cellular enzymes (e.g., lactose dehydrogenase, alanine aminotransferase, aspartate aminotransferase) or material (e.g., adenosine triphosphate), variation in RNA expression, inhibition of drug-metabolism capacity, reduction of intracellular ATP (adenosine triphosphate), cell death, apoptosis, and cell membrane degradation.

### Exemplary methods of making the devices described herein

Embodiments of various devices comprising a first chamber, a second chamber, and a membrane can enable us to leverage the control of microfluidic technology for device fabrication. In some embodiments, the devices described herein can be manufactured using any conventional fabrication methods, including, *e.g*., injection molding, embossing, etching, casting, machining, stamping, lamination, photolithography, or any combinations thereof. Soft lithography techniques are described in "Soft Lithography in Biology and Biochemistry," by Whitesides, et al., published Annual Review, Biomed Engineering, 3.335-3.373 (2001), as well as "An Ultra-Thin PDMS Membrane As A Bio/Micro-Nano Interface: Fabrication And Characterization", by Thangawng et al., Biomed Microdevices, vol. 9, num. 4, 2007, p. 587-95.

Without wishing to be limiting, in some embodiments, the devices described herein can be produced as a monolithic device or as individual components (*e.g.,* a first structure comprising a first chamber, a second structure comprising a second chamber, and a membrane), which can then be assembled together to form a device described herein. Each individual component can be produced by a conventional manufacturing method such as injection molding, extrusion, casting, lamination, embossing, compression molding, solvent casting, an additive manufacturing method (*e.g*., 3D printing), or any combinations thereof.

Once the first and second structures 204, 206 are formed and removed from their respective molds, the access ports can be made to access the chambers.

The membrane 208 can be engineered for a variety of purposes, some discussed above.

In some embodiments, the membrane 208 can be sandwiched between the first structure and the second structure, *e.g*., using an appropriate adhesive or epoxy, physical clamping and/or plasma bond between the two PDMS surfaces, in order to form a fluidic seal between the membrane with the first structure and the second structure.

After forming the body of the devices described herein, the first side of the membrane can be coated with an ECM composition according to one or more embodiments described herein. After formation of the ECM composition, tissue specific cells, *e.g*., hepatocytes, can be grown thereon.

In some embodiments, at least one layer of cells comprising blood vessel-associated cells (*e.g*., endothelial cells) can be cultured on the second side of the membrane. In some instances, fibroblasts and smooth muscle cells can be cultured on the second side of the membrane.

### Exemplary methods of using the devices and systems described herein

The ability of the devices described herein to recapitulate a physiological microenvironment and/or function can provide an *in vitro* model versatile for various applications, *e.g.,* but not limited to, modeling a liver-specific physiological condition (*e.g*., but not limited to normal and disease states) and/or identification of therapeutic agents. Accordingly, methods of using the devices are also described herein. In one aspect, the method comprises: (a) providing at least one device according to one or more embodiments described herein; and (b) flowing a first fluid through the first chamber.

In some embodiments, the method can further comprise flowing the second chamber with a second fluid.

In some embodiments, the method can further comprise detecting response of the endothelial cells and/or tissue specific cells (*e.g*., hepatocytes) in the device and/or detecting at least one component (*e.g.,* a cytokine or molecule secreted or consumed by the cells in the device) present in an output fluid from the device. Methods to detect different types of cell response are known in the art, including, *e.g*., but not limited to cell labeling, immunostaining, optical or microscopic imaging (*e.g*., immunofluorescence microscopy and/or scanning electron microscopy), gene expression analysis, cytokine/chemokine secretion analysis, metabolite analysis, polymerase chain reaction, immunoassays, ELISA, gene arrays, and any combinations thereof.

In some embodiments, the devices described herein can be used to create an *in vitro* model that mimics a liver-specific condition. As used herein, the term "liver-specific condition" refers to any condition that can be diagnosed in a liver *in vivo.* The condition can occur naturally in the liver *in vivo* (including, *e.g*., a normal healthy condition, or a condition induced or caused by a congenital defect), or induced or caused by a condition-inducing agent or stimulant (*e.g*., including, but not limited to an environmental agent such as alcohol).

In some embodiments, liver-specific cells such as hepatocytes can be derived from established cells lines and/or collected from a subject, *e.g*., by a biopsy, and/or differentiated from stem cells. In some embodiments, the stem cells (*e.g*., induced pluripotent stem cells) can be generated by differentiating somatic cells (*e.g*., skin fibroblasts) collected from a subject.

In some embodiments, disease-specific cells can be obtained from one or more patients diagnosed with the specific disease. For example, hepatocytes from an acquired liver condition or disorder at different stages of the liver condition or disorder can be isolated from the liver of a patient diagnosed with the acquired liver condition or disorder at a specific stage. By way of example only, in some embodiments, hepatocytes from non-alcoholic fatty liver disease (NAFLD) and/or nonalcoholic steatohepatitis (NASH) at different stages (e.g., from mild steatosis to cirrhosis) can be isolated from the liver of a patient diagnosed with NAFLD and/or NASH. These disease-specific cells can then be cultured in a device described herein to model the disease-specific liver condition or disorder.

In some embodiments where the availability of disease-specific liver cells is low, non-disease liver cells can be manipulated in vitro, e.g., by genetic methods such as gene silencing/knock down, to acquire at least one or more phenotypes of a liver disease or disorder.

By way of example only, hepatitis (e.g., hepatitis C virus infection) can be modeled in a device described herein by infecting hepatocytes or induced pluripotent stem cell-derived hepatocytes from a host of interest with a hepatitis virus from a patient carrier. See, e.g., Schwartz et al., PNAS (2012) 109: 2544-2548, for additional information about modeling hepatitis C virus infection using induced pluripotent stem cells.

As another example, malaria infection can be modeled in a device described herein by infecting hepatocytes or induced pluripotent stem cell-derived hepatocytes from a host of interest with *P*. *falciparum* and/or *P. vivax* sporozoites. See, e.g., March et al., Cell Host Microbe (2013) 14: 104-115, for additional information about modeling malaria infection in a liver cell culture.

In some embodiments, disease-specific cells can be induced pluripotent stem (iPS) cell-derived hepatocytes. In some embodiments, the iPS cells can be derived from differentiated somatic cells (e.g., skin fibroblasts) obtained from a patient diagnosed with a genetically caused liver pathology (including, but not limited to hemochromatosis, alpha1-antitrypsin deficiency, familial hypercholesterolemia, and/or glycogen storage disease type 1a). In some embodiments, the iPS cells can be genetically engineered to have one or more mutations associated with a genetically caused liver pathology in a patient. Accordingly, the iPS cell-derived hepatocytes can then be cultured in a device described herein to model a disease-specific liver condition or disorder.

In other embodiments, the liver-specific cells (*e.g*., normal liver-specific cells) can be contacted with a condition-inducing agent described herein that is capable of inducing the liver-specific cells to acquire at least one characteristic associated with the liver-specific condition. For example, hepatocarcinoma or liver metastasizes can be modeled in a device described herein by introducing tumor cells (primary tumor cells or established lines) into the device cultured with hepatocytes. In another example, drug induced liver injury can be introduced to liver cells by contacting the liver cells with a known or novel compound or agent that causes injury to liver cells. Viral and bacterial infections can also be modeled in a device described herein, e.g., by stimulating hepatocytes and/or associated liver cells with a viral or bacterial-mimicking stimuli such as TNFα, lipopolysaccharides, and/or polyinosinic:polycytidylic acid.

In some embodiments, a liver-specific condition, *e.g*., a disease-specific condition can be created by genetically modifying normal healthy cells, *e.g.,* by silencing one or more genes, or over-expressing one or more genes. Methods of gene silencing include, but are not limited to, RNA interference (*e.g*., but not limited to small interfering RNA (siRNA), microRNA (miRNA), and/or short hairpin RNA (shRNA)), antisense oligonucleotides, ribozymes, triplex forming oligonucleotides, and the like.

In some embodiments where the devices described herein are used to create a disease-specific model, the devices can further comprise normal healthy cells (*e.g*., obtained from one or more healthy donors) cultured in a separate chamber, *e.g.*, to create a baseline for comparison.

In some embodiments, the device can comprise both healthy and disease-specific cells. In some embodiments, the device can include only disease-specific cells.

In some embodiments, the devices described herein can be used to determine an effect of a test agent (*e.g.*, toxicity) on the cells cultured in the devices described herein. Accordingly, in some embodiments, the method can further comprise contacting the liver-specific cells (*e.g*., hepatocytes, alone or in combination with cholangiocytes (biliary endothelial cells) and/or liver fibroblasts) and/or endothelial cell layer (optionally further comprising macrophagic kupffer cells, hepatic stellate cells, and/or liver fibroblasts) with a test agent. Non-limiting examples of the test agents include proteins, peptides, single or double-stranded nucleic acids, antigens, nanoparticles, environmental toxins or pollutant, naturally occurring particles including, *e.g*., pollen, chemical or bioweapons, small molecules, drugs or drug candidates, chemicals or particles used in cosmetic products, aerosols, vaccine or vaccine candidates, pro-inflammatory agents, viruses, bacteria, unicellular organisms, cytokines, and any combinations thereof.

Effects of the test agent on the cells can be determined by measuring response of the cells to the test agent, the fluid exiting the first chamber, the fluid exiting the second chamber, or any combinations thereof; and comparing the measured response with the cells not contacted with the test agent. Various methods to measure cell response are known in the art, including, but not limited to, cell labeling, immunostaining, optical or microscopic imaging (*e.g*., immunofluorescence microscopy and/or scanning electron microscopy), spectroscopy, gene expression analysis, cytokine/chemokine secretion analysis, metabolite analysis, polymerase chain reaction (PCR), immunoassays, ELISA, gene arrays, spectroscopy, immunostaining, electrochemical detection, polynucleotide detection, fluorescence anisotropy, fluorescence resonance energy transfer, electron transfer, enzyme assay, magnetism, electrical conductivity (*e.g*., trans-epithelial electrical resistance (TEER)), isoelectric focusing, chromatography, immunoprecipitation, immunoseparation, aptamer binding, filtration, electrophoresis, use of a CCD camera, mass spectroscopy, or any combination thereof. Detection, such as cell detection, can be carried out using light microscopy with phase contrast imaging and/or fluorescence microscopy based on the characteristic size, shape and refractile characteristics of specific cell types. Greater specificity can be obtained using optical imaging with fluorescent or cytochemical stains that are specific for individual cell types or microbes.

The cellular responses to the test agent, various environmental and/or external cues can be monitored using various systems that can be combined with the devices described herein. For example, one can monitor changes in pH using well known sensors. One can integrate force sensors into the membrane to measure changes in the mechanical properties of the cells. One can also sample cells, continuously or periodically for measurement of changes in gene transcription or changes in cellular biochemistry or structural organization. For example, one can measure reactive oxygen species (ROSs) that are a sign of cellular stress. One can also subject the "tissue" grown on either side or both sides of the membrane to microscopic analysis, immunohistochemical analysis, *in situ* hybridization analysis, or typical pathological analysis using staining, such as hematoxylin and eosin staining. Samples for these analyses can be carried out in real-time, or taken after an experiment or by taking small biopsies at different stages during a study or an experiment.

In some embodiments, the exclusion of fluorescently labeled large molecules (*e.g*. dextrans of different weight or FITCs) can be quantitated to determine the permeability of the endothelium and thus assess the barrier function of the epithelium, *e.g*., in a tissue-specific condition. For example, flowing a fluid containing fluorescently labeled large molecules (*e.g*., but not limited to, inulin-FITC) into a first chamber cultured with differentiated epithelium can provide a non-invasive barrier measurement. As a functional tight junction barrier will prevent large molecules from passing through the epithelium from the first chamber to the second chamber, the absence of the detection of the fluorescently labeled large molecules in the second chamber is indicative of a functional barrier function of the epithelium.

In some embodiments, one can subject the cells grown on the membrane to other cells, such as immune system cells or bacterial cells, to antibodies or antibody-directed cells, for example to target specific cellular receptors. One can expose the cells to viruses or other particles. To assist in detection of movement of externally supplied substances, such as cells, viruses, particles or proteins, one can naturally label them using typical means such as radioactive or fluorescent labels.

In some embodiments where the liver-specific cells to be assayed are adapted to be condition-specific (*e.g*., disease-specific), exposure of the liver-specific cells to a test agent followed by determination of the effect of the test agent on the cells can facilitate identification of a therapeutic agent for treatment of the condition, or measurement of efficacy of a therapeutic agent. As used herein, the term "efficacy" generally refers to ability of a test agent to produce a desired therapeutic effect or outcome. Depending on the nature and/or type of the test agents, examples of desired effects or outcomes include, but are not limited to, therapeutic effect, cytotoxicity, cell growth, cell differentiation, improved or reduced cell function or phenotype (*e.g*., but not limited to, permeability of a cell layer, cell migration, expression and/or secretion of a protein or cytokine that can be affected by cell exposure to the test agent), and any combinations thereof. The term "therapeutic effect" as used herein refers to a consequence of treatment, the results of which are judged to be desirable and beneficial.

In some embodiments where the liver-specific cells are patient-specific, exposure of the patient-specific cells to a test agent, followed by determination of the effect of the test agent on the cells can facilitate identification of a personalized treatment for a subject.

In some embodiments where the tissue-specific cells are patient population-specific, exposure of the patient population-specific cells to a test agent, followed by determination of the effect of the test agent on the cells can facilitate identification of a treatment specified for that particular patient population. As used herein, the term "patient population-specific" refers to cells collected from a population of patients sharing at least one or more phenotypes and/or characteristics (*e.g*., but not limited to, specific gene mutation, ethnicity, gender, life styles, BMI, resistance to treatment, and any combinations thereof) other than the disease or disorder.

In accordance with some embodiments of various aspects described herein, the devices can be used to determine toxicity of a test agent upon exposure to the hepatocytes. For example, the toxicity of a test agent can be determined by measuring response of the hepatocytes and/or at least one component present in a fluid (*e.g.,* gaseous and/or liquid fluid) within the device or present in an output fluid (*e.g.,* gaseous and/or liquid fluid) from the device after exposure to the test agent. As used herein, the term "toxicity" refers to ability of a test agent to induce or cause any adverse and/or side effect on a cell and/or even cell death. For example, the toxicity of a test agent can be characterized by its ability to induce or cause an adverse effect on cell function and/or phenotype, including, but not limited to, alteration in cell metabolism, mutagenicity, carcinogenicity, teratogenicity, DNA damage, protein or membrane damage, cell energy depletion, mitochondrial damage, genotoxicity, apoptosis, cell death, cell rupture, and any combinations thereof.

In some embodiments, one or more devices described herein can be used in combination with a pharmacokinetic (PK) model, a pharmacodynamic (PD) model, or a PK-PD model to quantitatively analyze the effect of an agent to be tested. For example, a series of devices, each modeling a tissue, *e.g*., one for gut, one for liver, and another one for heart, can be connected to provide a microphysiological system that can be used to determine the fate of an agent administered into the system. The term "pharmacokinetics" is used herein in accordance with the art, and refers to the study of the action of agents, *e.g*., drugs, in the first structure and/or second structure, for example, the effect and duration of drug action, the rate at which they are absorbed, distributed, metabolized, and eliminated by the first structure and/or second structure etc. (*e.g.* the study of a concentration of an agent, *e.g.,* a drug, in the serum of a patient following its administration via a specific dose or therapeutic regimen). The term "pharmacodynamics" is used in accordance with the art, and refers to the study of the biochemical and physiological effects of an agent, *e.g.,* a drug, on a subject's first structure and/or second structure or on microorganisms such as viruses within or on the first structure and/or second structure, and the mechanisms of drug action and the relationship between drug concentration and effect (*e.g.* the study of a pathogen, *e.g*., a virus, present in a patient's plasma following one or more therapeutic regimens). Methods for PK-PD modeling and analysis are known in the art. See, *e.g*., Bonate, P.L. (2006). Pharmacokinetic-Pharmacodynamic Modeling and Simulation. New York, Springer Science & Business Media; Gabrielsson, J. and D. Weiner (2000); and Pharmacokinetic and Pharmacodynamic Data Analysis: Concepts and Applications. Stockholm, Swedish Pharmaceutical Press. For example, a PK model can be developed to model a microphysiological system comprising a plurality of the devices described herein, wherein each device can model a different tissue that can produce an effect (*e.g*., absorption, metabolism, distribution and/or excretion) on an agent to be administered. To construct a PK model for a device described herein, mass balance equations describing the flow in, flow out, and metabolism of an agent can be set up for each first chamber and second chamber. A PD model can be integrated into each device described herein, describing the kinetics of potential cell response (*e.g*., inflammation, cytokine release, ligand binding, cell membrane disruption, cell mutation and/or cell death) in each device that mimics a tissue or an organ. This *in vitro*/in silico system, combining one or more devices described herein with an integrated PK-PD modeling approach, can be used to predict drug toxicity in a more realistic manner than conventional *in vitro* systems. In some embodiments, one or more of the devices described herein can be used to quantify, estimate or gauge one or more physical-chemical, pharmacokinetic and/or pharmacodynamic parameters. Various physical-chemical, pharmacokinetic and pharmacodynamic parameters are known in the art, including, for example, the ones discussed in the aforementioned references. Exemplary physical-chemical, pharmacokinetic and pharmacodynamic parameters include, but are not limited to, permeability, logP, logD, volume of distribution, clearances (including intrinsic clearances), absorption rates, rates of metabolism, exchange rates, distribution rates and properties, excretion rates, IC50, binding coefficients, etc.

In some instances, for reference only, the devices described herein can be cultured with animal cells (*e.g*., but not limited to, pig cells, rabbit cells, dog cells, mouse cells, and/or rat cells) to determine response of the animal cells to an agent introduced into the devices described herein. The measured response of the animal cells in the devices can then be correlated with the actual response occurred *in vivo* when the agent is administered to a living animal (*e.g.,* a pig, a rabbit, a dog, a mouse, and/or a rat). By identifying the correlation between the *in vitro* and *in vivo* responses in one or more animal models, one can extrapolate or predict effect of the agent on a human subject *in vivo,* based on the measured responses of the human cells to the agent in the devices. Additionally or alternatively, a therapeutic dose of an agent for a human subject can be determined accordingly.

The devices described herein can have many different applications including, but not limited to, cell differentiation, formation of a stratified and/or three-dimensional tissue structure, development of a disease model in a tissue of interest, development of a mucosal immunity platform; studies on clearance of a particle or molecule; studies on immune cell response (*e.g*., trans-epithelial migration, maturation, activation, cell killing, and/or drainage); studies on various tissue-specific diseases such as liver, respiratory, intestinal, digestive, skin, cardiac, and/or ocular diseases; studies of mechanism of action of drugs, target identification and/or validation, identification of markers of disease; assessing pharmacokinetics and/or pharmacodynamics of various chemical or biological agents; assessing efficacy of therapeutics and/or vaccines; testing gene therapy vectors; drug and/or vaccine development; molecule or drug screening or drug discovery; determination of an appropriate treatment or drug for a specific patient population or individual patient; identification of a risk population to a disease or disorder; identification of a new drug target for a patient population that is non-responsive to a previously-administered treatment; studies of cell behavior in a physiologically-relevant model (including, e.g., stem cells and bone marrow cells); studies on biotransformation, absorption, clearance, metabolism, and activation of xenobiotics; studies on bioavailability and transport of chemical or biological agents across epithelial or endothelial layers; studies on transport of biological or chemical agents across the liver epithelial barrier; studies on acute basal toxicity of chemical agents; studies on acute local or acute organ-specific toxicity of chemical agents; studies on chronic basal toxicity of chemical agents; studies on chronic local or chronic organ-specific toxicity of chemical agents; studies on teratogenicity of chemical agents; studies on genotoxicity, carcinogenicity, and/or mutagenicity of chemical agents; detection of infectious biological agents and/or biological weapons; detection of harmful chemical agents and chemical weapons; studies on infectious diseases (*e.g*., bacterial, viral and/or fungal infections); assessing infectivity and/or virulence of a new strain; studies on the optimal dose range of a chemical and/or biological agent to treat a disease; prediction of the response of an organ *in vivo* exposed to a biological and/or chemical agent; studies concerning the impact of genetic content on response to agents; studies on gene transcription in response to chemical or biological agents; studies on protein expression in response to chemical or biological agents; studies on changes in metabolism in response to chemical or biological agents; as well as example uses described below. The devices described herein can also be used to screen on the cells, for an effect of the cells on the materials (for example, in a manner equivalent to tissue metabolism of a drug).

The devices described herein can also allow different growth factors, chemicals, gases and nutrients to be added to different cell types according to the needs of cells and their existence *in vivo.* Controlling the location of those factors or proteins can direct the process of specific cell remodeling and functioning, and also can provide the molecular cues to the whole system, resulting in such beneficial developments as neotissue, cell remodeling, enhanced secretion, and the like.

In yet another aspect, the devices described herein can be utilized as multi cell type cellular microarrays, such as microfluidic devices. Using the devices described herein, pattern integrity of cellular arrays can be maintained. These cellular microarrays can constitute the "lab-on-a-chip", particularly when multiplexed and automated. These miniaturized multi cell type cultures will facilitate the observation of cell dynamics with faster, less noisy assays, having built-in complexity that will allow cells to exhibit *in vivo*-like responses to the array.

In yet another aspect, the devices described herein can be utilized as biological sensors. Cell-based biosensors can provide more information than other biosensors because cells often have multifaceted physiological responses to stimuli, as well as novel mechanisms to amplify these responses. All cell types in the devices described herein can be used to monitor different aspects of an analyte at the same time; different cell type in the devices described herein can be used to monitor different analytes at the same time; or a mixture of both types of monitoring. Cells ranging from E. coli to cells of mammalian lines have been used as sensors for applications in environmental monitoring, toxin detection, and physiological monitoring.

In some embodiments, the devices described herein can be utilized in an overall system incorporating sensors, computers, displays and other computing equipment utilizing software, data components, process steps and/or data structures.

**Fig. 30** illustrates a schematic of a system having at least one device in accordance with an embodiment described herein, *e.g.*, 706A, fluidically connected to another device described herein, *e.g.*, 706B, and/or any cell culture device known in the art, *e.g.,* an art-recognized organ-on-a-chip, *e.g.*, 706C. As shown in **Fig. 30**, the system 700 includes one or more CPUs 702 coupled to one or more fluid sources 704 and external force sources (*e.g*., pressure sources) (not shown), whereby the preceding are coupled to three devices 706A, 706B, and 706C. It should be noted that although three devices 706 are shown in this embodiment, fewer or greater than three devices 706 can be used. In the system 700, two of the three devices (i.e. 706A and 706B) are connected in parallel with respect to the fluid source 704 and devices 706A and 706C are connected in serial fashion with respect to the fluid source 704. It should be noted that the shown configuration is only one example and any other types of connection patterns can be utilized depending on the application. In some embodiments, a system can be the one described in the International Patent Application No. PCT/US 12/68725, entitled "Integrated human organ-on-chip microphysiological systems," where one or more devices described herein can be fluidically connected to form the system.

In the example shown, fluid from the fluid source 704 is provided directly to the fluid inlets of devices 706A and 706B. As the fluid passes through device 706A, it is output directly into the fluid inlet port of devices 706B and 706C. Additionally, the fluid outlet from device 706B is combined with the output from device 706A into device 706C. With multiple devices operating, it is possible to monitor, using sensor data, how the cells in the fluid or membrane behave after the fluid has been passed through another controlled environment. This system thus allows multiple independent "stages" to be set up, where cell behavior in each stage can be monitored under simulated physiological conditions and controlled using the devices 706. One or more devices are connected serially can provide use in studying chemical communication between cells. For example, one cell type can secrete protein A in response to being exposed to a particular fluid, whereby the fluid, containing the secreted protein A, exits one device and then is exposed to another cell type specifically patterned in another device, whereby the interaction of the fluid with protein A with the other cells in the other device can be monitored (*e.g*. paracrine signaling). For the parallel configuration, one or more devices connected in parallel can be advantageous in increasing the efficiency of analyzing cell behavior across multiple devices at once instead of analyzing the cell behavior through individual devices separately.

For example, as illustrated in **Fig. 6A**, an organ-on-a-chip device (*e.g*., as described in the U.S. Patent No. 8,647,861, and in the International Patent App. No. PCT/US2014/071611) can be coupled to a liver-on-a- chip device described herein. A drug introduced into the organ-on-a-chip device can exit the device via a culture medium exiting the organ-on-a-chip device and be directed to the liver-on-a-chip device where hepatocytes can be exposed to the drug. Thus, drug metabolism can be determined, *e.g*., by measuring the activity of CYP450 drug metabolizing enzyme and/or the level of metabolites converted from the drug.

The drug(s) can be introduced into the devices as aerosols and/or by culture medium. By way of example only, as shown in **Fig. 6A**, an aerosol drug can be introduced to the "air" channel" containing lung epithelial cells on one side of the membrane. The other side of the membrane facing the "vascular" channel can be lined with endothelial cells. The aerosol drug diffuses to "vascular" channel across the lung epithelial cells and endothelial cells. The drug-containing fluid flowing through the "vascular" channel can then be directed to hepatocytes in the liver-on-a-chip device, where the activity of CYP3A4 drug metabolizing enzyme activity induced by the aerosol drug can be measured.

### Endothelial cell culture media and methods of using the same

Not only have the inventors created a device for simulating a function of a liver tissue, the inventors have also developed a novel endothelial cell culture medium for long term culture of endothelial cells and/or more importantly for long term co-culture of endothelial cells and epithelial cells from different tissue origins. Accordingly, in one aspect, an endothelial cell culture medium that provides longer viability of endothelial cells than existing media (*e.g*., by at least about 2 weeks or longer, including, *e.g*., at least about 3 weeks, at least about 4 weeks or longer) is described herein. The endothelial cell culture medium comprises (a) a basal medium; and (b) a supplement cocktail essentially consisting of hEGF, hydrocortisone, VEGF, hFGF-beta, R3-IGF-1, ascorbic acid, heparin, and serum, wherein the concentration of the serum is about 0.3% to about 1%. In some embodiments, the basal medium can be a mixture of DMEM and F12.

In some embodiments, the serum can comprise, essentially consist of, or consist of FBS.

As used herein, the term "medium" or "media", when used in reference to endothelial cell culture media, refers to a basal medium or media for culturing cells and containing nutrients that maintain cell viability and support proliferation. The cell culture basal medium can contain any of the following in an appropriate combination: salt(s), buffer(s), amino acids, glucose or other sugar(s), antibiotics, and other components such as peptide growth factors, etc. Cell culture media ordinarily used for particular cell types are known to those skilled in the art. Examples of cell culture medium include Minimum Essential Medium (MEM), Eagle's Medium, Dulbecco's Modified Eagle Medium (DMEM), Dulbecco's Modified Eagle Medium: Nutrient Mixture F-12 (DMEM F12), FIO Nutrient Mixture, Ham's FlO Nutrient Mix, Ham's F12 Nutrient Mixture, Medium 199, RPMI, RPMI 1640, reduced serum medium, basal medium (BME), DMEM/F12 (1:1), and the like, and combinations thereof. The cell culture basal medium or media can be modified by adding one or more factors or components to suit the need of different applications. For example, to make the endothelial cell culture media described herein, the cell culture basal medium or media can be added with, for example, hEGF, hydrocortisone, VEGF, hFGF-beta, R3-IGF-1, ascorbic acid, heparin, and serum.

In some embodiments, the endothelial cell culture medium can be in a form of powder (*e.g*., lyophilized powder). The powder can be reconstituted in an aqueous solution (*e.g*., water) upon use to reach the desired concentrations as described herein.

In one embodiment, the endothelial cell culture medium is DMEM/F12 basal medium supplemented with hEGF, hydrocortisone, VEGF, hFGF-beta, R3-IGF-1, ascorbic acid, heparin, and serum, wherein the concentration of FBS is about 0.3%. Antibiotics (*e.g.,* Penicillin-Streptomycin) and/or phenol red can be optionally added.

The concentrations of each individual component described are generally the working concentrations for the culturing methods of various aspects described herein. In some embodiments, the concentrations of each individual component in the endothelial cell culture media can be increased, *e.g.,* by 2-fold or more, including, *e.g.,* 3-fold, 4-fold, 5-fold, 6-fold, 7-fold, 8-fold, 9-fold, 10-fold, or higher to create a concentrated endothelial cell culture media. A user can dilute the concentrated endothelium cell culture media to the working concentrations with an aqueous solution (*e.g.,* sterilized water) upon use. Accordingly, concentrated endothelial cell culture media are also provided herein.

In another aspect, described herein is a method of maintaining long-term viability of endothelial cells. The method comprises contacting a population of endothelial cells with an endothelial cell culture medium comprising: (a) a basal medium; and (b) a supplement cocktail essentially consisting of hEGF, hydrocortisone, VEGF, hFGF-beta, R3-IGF-1, ascorbic acid, heparin, and serum, wherein the concentration of the serum is about 0.3% to about 1%. Using the endothelial cell culture medium, the endothelial cells can be maintained as a viable monolayer for at least two weeks or longer.

In some embodiments, the serum can comprise, essentially consist of, or consist of FBS.

Examples of cell culture basal media that can be used to formulate the endothelial cell culture media include, but are not limited to, Minimum Essential Medium (MEM), Eagle's Medium, Dulbecco's Modified Eagle Medium (DMEM), Dulbecco's Modified Eagle Medium: Nutrient Mixture F-12 (DMEM: F12), FlO Nutrient Mixture, Ham's FIO Nutrient Mix, Ham's F12 Nutrient Mixture, Medium 199, MCDB-131, RPMI, RPMI 1640, reduced serum medium, basal medium (BME), and the like, and combinations thereof. In some embodiments, the basal medium can be a mixture of DMEM and F12. In some embodiments, the DMEM and F12 are in volumetric ratio of about 1:1. In some embodiments, the basal medium can comprise MCDB-131 or a culture medium substantially similar to MCDB-131.

### Kits

Kits for cell culture are also described herein. In one aspect, the kit comprises (a) a cell culture device; and (b) an endothelial cell culture medium according to one or more embodiments described herein.

As used herein, the term "cell culture device" refers to a device or apparatus suitable for culturing cells. Examples of cell culture devices include, but are not limited to, Transwell systems, plates, microwells, bioreactors, microfluidic devices, and any combinations thereof. In some embodiments, the cell culture device provided in the kit can be a device for simulating a function of a tissue, which comprises (i) a first structure defining a first chamber; (ii) a second structure defining a second chamber; and (iii) a membrane located at an interface region between the first chamber and the second chamber to separate the first chamber from the second chamber, the membrane including a first side facing toward the first chamber and a second side facing toward the second chamber. In some embodiments, the first side or the second side of the membrane can comprise endothelial cells adhered thereon. In some embodiments, the first side or the second side of the membrane can comprise tissue-specific cells adhered thereon. Tissue-specific cells can be cells derived from liver. Also described herein, is tissue-specific cells derived from any tissue or organ of interest including a kidney, a skin, an eye, a brain, a blood-brain-barrier, a heart, a gastrointestinal tract, airways, a reproductive organ, and a combination of two or more thereof.

In some embodiments, the basal medium and the supplement cocktail provided in the kit can be pre-mixed to form the endothelial cell culture medium.

In some embodiments, the basal medium and the supplement cocktail provided in the kit can be separately packaged. For example, the basal medium and the supplement cocktail can be packaged independently, *e.g.,* as powder or liquid.

In some embodiments where the basal medium and/or the supplement cocktail are in a form of powder, *e.g.,* lyophilized powder, the powder can be reconstituted upon use. In some embodiments, the basal medium and/or the supplement cocktail can be in a form of liquid.

In some embodiments, the kit can further comprise at least one vial of cells. In one embodiment, the cells can be endothelial cells. In one embodiment, the cells can be tissue-specific cells.

### Some selected definitions

Unless defined otherwise, all technical and scientific terms used herein have the same meaning as commonly understood by one of ordinary skill in the art to which this invention belongs.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

Other than in the operating examples, or where otherwise indicated, all numbers expressing quantities of ingredients or reaction conditions used herein should be understood as modified in all instances by the term "about." The term "about" when used to described the present invention, in connection with percentages means ±5%.

In one aspect, the present invention relates to the herein described compositions, methods, and respective component(s) thereof, as essential to the invention, yet open to the inclusion of unspecified elements, essential or not ("comprising"). In some embodiments, other elements to be included in the description of the composition, method or respective component thereof are limited to those that do not materially affect the basic and novel characteristic(s) of the invention ("consisting essentially of"). This applies equally to steps within a described method as well as compositions and components therein. In other embodiments, the inventions, compositions, methods, and respective components thereof, described herein are intended to be exclusive of any element not deemed an essential element to the component, composition or method ("consisting of").

As used herein, the term "co-culture" refers to two or more different cell types being cultured in some embodiments of the devices described herein. The different cell types can be cultured in the same chamber (*e.g.,* first chamber or second chamber) and/or in different chambers (*e.g.,* one cell type in a first chamber and another cell type in a second chamber). For example, in some embodiments, the devices described herein can have hepatocytes in the first chamber and endothelial cells in the second chamber.

The term "statistically significant" or "significantly" refers to statistical significance and generally means a two standard deviation (2 SD) below normal, or lower, concentration of the marker. The term refers to statistical evidence that there is a difference. It is defined as the probability of making a decision to reject the null hypothesis when the null hypothesis is actually true. The decision is often made using the p-value.

A "marker" as used herein is used to describe the characteristics and/or phenotype of a cell. Markers can be used for selection of cells comprising characteristics of interests. Markers will vary with specific cells. Markers are characteristics, whether morphological, functional or biochemical (enzymatic) characteristics of the cell of a particular cell type, or molecules expressed by the cell type. In some embodiments, such markers are proteins, and possess an epitope for antibodies or other binding molecules available in the art, and thus can be monitored by FACs analysis, and immunocytochemistry. However, a marker may consist of any molecule found in a cell including, but not limited to, proteins (peptides and polypeptides), lipids, polysaccharides, nucleic acids and steroids. Examples of morphological characteristics or traits include, but are not limited to, shape, size, and nuclear to cytoplasmic ratio. Examples of functional characteristics or traits include, but are not limited to, the ability to adhere to particular substrates, ability to incorporate or exclude particular dyes, ability to filtrate particles, ability to migrate under particular conditions, and the ability to differentiate along particular lineages. Markers may be detected by any method available to one of skill in the art, including for example, detection of nucleic acid, *e.g.* mRNA, *e.g.* by quantitative PCR.

As used interchangeably herein, the term "substantially" means a proportion of at least about 60%, or preferably at least about 70% or at least about 80%, or at least about 90%, at least about 95%, at least about 97% or at least about 99% or more, or any integer between 70% and 100%. In some embodiments, the term "substantially " means a proportion of at least about 90%, at least about 95%, at least about 98%, at least about 99% or more, or any integer between 90% and 100%. In some embodiments, the term "substantially" can include 100%.

As used herein, the term "functional fragment or variant" when used in reference to an ECM molecule, *e.g.,* but not limited to fibronectin, collagen I, and/or collagen IV, refers to a fragment or variant of such ECM molecule that retains at least about 70% or more (including, *e.g.,* at least about 75%, at least about 80%, at least about 85%, at least about 90%, at least about 95%, at least about 97%, at least about 99%, or 100%) of the activity of the wild-type or parent ECM molecule, *e.g.,* to provide structural and/or biochemical support (*e.g.,* cell adhesion and/or proliferation) for cells.

All patents, patent applications, and publications identified for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

### EXAMPLES

The following examples illustrate some embodiments and aspects described herein. The following examples do not in any way limit the invention.

### Example 1. Human liver-on-a-chip device design 1 and characterization

Hepatocytes, unlike other cell types, do not simply adhere to a surface and proliferate upon thawing. To identify suitable cell sources, cryopreserved vials were evaluated for their adhesion ability, their ability to retain their *in vivo* like morphology, and/or their ability to form bile canaliculi network (for biliary clearance determination). Typically, in 2D static culture hepatocytes flatten out and lose the cobblestone morphology.

After identifying suitable cell sources of hepatocytes, the hepatocytes were cultured on a first side of the membrane (coated with collagen I at approx.. 100 ug/mL) facing a first chamber of the device, *e.g.,* as shown in **Fig. 2B** or **Fig. 7B****,** to permit growth of a confluent monolayer within the first chamber. **Fig. 3A** is a set of images showing hepatocytes (top panel) and hLSECs (bottom panel) from Day 13. It is noted that the cells were able to continue to grow for at least about 3 weeks or longer. One of the striking things observed is that this morphology was still observed after 3 weeks, which is not normally seen in static culture. In static culture the cells generally begin to deteriorate after about 1 week.

To assess whether the cells formed bile canaliculi, a live stain for using CDFDA (a marker that is excreted into the bile via efflux transporters) was performed. The stain (**Fig. 3A** top panel) showed that active transport occurred between the hepatocytes growing in the devices described herein.

Human liver sinusoidal endothelial cells (LSECs) were cultured on a second side of the membrane facing a second chamber of the device. The LSECs were stained for CD32b (an endothelial cell marker) that is normally expressed by human LSECs, and the image was shown in **Fig. 3A****,** bottom panel. A flow of cell culture medium was also introduced into the first chamber and the second chamber at different flow rates to subject the cells to appropriate shear stress. For example, the hepatocytes were exposed to a shear stress of about 0.025 dyne/cm², and the LSECs a shear stress of about 0.05 dynes/cm².

After the cells were determined to maintain proper morphology in the devices described herein, cell viability was assessed over time. Cell viability can be measured by any known methods in the art. For example, lactate dehydrogenase (LDH) levels can be measured to assess cell viability. LDH is an enzyme found in all cells. When a cell is damaged and the membrane is compromised or becomes non-intact (*e.g.,* with a break in the membrane), LDH is released into the culture media and thus the culture media can be collected and measured for LDH content. **Fig. 3B** shows that cells cultured in one embodiment of the liver-on-a-chip device described herein (also referred to as "liver chip" herein) released significantly lower levels of LDH than cells in static culture, indicating that subjecting the cells to a low shear stress (*e.g.,* a fluid flow) can facilitate maintenance of cell viability.

Next, the device was evaluated to determine if it could also exhibit or retain normal liver function. To assess the normal function of the liver chip, in one embodiment, albumin secretion (normally measured from blood of a subject) was measured from the culture medium flowing in the LSEC chamber. A reduced level in albumin secretion is indicative of liver injury. **Fig. 4A** shows that albumin secretion was maintained in the liver chip for at least 2 weeks or longer and was significantly higher than albumin secretion measured in conventional static culture.

It was next sought to determine function of the liver chip for drug metabolism. To this end, CYP3A4 was measured because it is the major P450 drug metabolizing enzyme in the liver. **Fig. 4B** shows that the liver chip's basal CYP3A4 enzyme activity levels were at least 4 times greater than the level in static culture. While these levels can vary greatly from donor to donor, the basal CYP3A4 level observed in the liver chip was comparable to what is observed *in vivo.*

Induction of CYP3A4 activity in the liver is one of main causes of drug-drug interactions, which is a mechanism to defend against exposure to drugs and toxin, but can also lead to unwanted side-effects (toxicity) or change the efficacy of a drug. **Fig. 5** illustrates that when taking two drugs that interact with the same enzyme (in this case CYP3A4), Drug A can increase the metabolism of Drug B or decrease the efficacy of drug B. For example, Drug A can bind the nuclear receptor PXR in the hepatocytes and cause upregulation of the CYP3A4 enzyme, which will in turn increase metabolite formation of Drug B and thus reduce the level of Drug B in circulation and its efficacy. In addition, the increased metabolite formation of Drug B can increase toxic metabolites.

Physiological coupling between two devices, each simulating a function of a different tissue, was also evaluated. In one embodiment, measurement of CYP3A4 activity was used to determine physiological coupling between different organ chips. **Fig. 6A** is a schematic diagram showing physiological coupling between a lung chip (*e.g.,* as described in the U.S. Patent No. 8,647,86) and a liver chip for a period of time (e.g., about 1 week). Rifampicin was administered to the "air" channel" at a dose of about 50 µM (*e.g.,* determined by mass spectrometry). In some embodiments, rifampicin can be administered to the "air" channel at a concentration of about 10 µM. The rifampicin level measured in the "vascular" channel across the lung epithelial cells and endothelial cells was about 1.4 µM, indicating that the lung endothelial cells formed a functional barrier. The rifampicin-containing fluid flowing through the "vascular" channel was then directed to the hepatocytes in the liver chip. A 2 fold increase in CYP3A4 drug metabolizing enzyme activity induced by rifampicin was detected, as compared to control without rifampicin **(****Fig. 6B****).** According to FDA guidelines, a 2-fold induction in CYP3A4 activity should be observed for drug-drug interaction.

### Example 2. Human liver-on-a-chip device design 2 and characterization

As hepatocytes are much more cuboidal than epithelial cells of other tissue, *e.g*., alveolar flat cell type, the height of the chamber in which the hepatocytes are cultured can be increased to accommodate the cell size as well as the width so that much more confluent monolayers of hepatocytes can be achieved. For example, in one embodiment, the hepatocytes can be cultured in the top chamber of the device shown in **Fig. 7B****.** As described in Example 1, CYP3A4 activity of the hepatocytes growing in the device of **Fig. 7B** (referred to as "Liver chip v2") was measured over a period of time, and compared to the cells grown in the device of **Fig. 7A** (referred to as "Liver chip v1"). **Fig. 8** shows that CYP3A4 activity levels steadily increased in Liver chip v1, while the Liver chip v2 showed comparable activity levels to fresh hepatocytes and the CYP3A4 activity was maintained in Liver chip v2 for at least about 2 weeks. Similarly, hepatocytes collected from human subjects (Donor 1 and Donor 2) exhibited *in vivo-*relevant CYP3A4 levels for at least about 2 weeks when they were cultured in the liver chips, as compared to static cultures **(****Figs. 9A-9B****).** Indeed, the CYP3A4 levels measured in the liver chip described herein was also magnitudes higher than the CYP3A4 activity measured in the existing human liver model (*e.g.,* 3D InSight^{™} human liver microtissues from InSphero).

To assess the drug-drug interaction in the liver chip, hepatocytes were cultured for about 2 days in the top chamber of the liver chip and then treated with rifampin and testosterone for about 3 days. Rifampin is known to induce the level of CYP3A4 activity in hepatocytes, which in turn increases the metabolism of testosterone to 6-b-hydroxytestosterone. Thus, CYP450 activity was reflected by measuring formation of 6-b-hydroxytestosterone turnover (from testosterone) and the readout was detected by mass spectrometry. A high level of 6-b-hydroxytestosterone is indicative of a high CYP3A4 activity. **Fig. 10** shows that basal levels of CYP3A4 activity were higher in the liver chip than in static Matrigel^{®} sandwich culture, the industry "gold standard." A robust induction of about 2-fold increase in the CYP3A4 activity in the liver chip (despite PDMS absorption) should enable drug-drug interaction studies. The liver chip was further validated by showing CYP3A4 enzyme activity response to dexamethasone (anti-inflammatory molecule) at various concentrations indicated in **Fig. 11. Fig. 11** shows that induction of CY3A4 activity in the human liver chip increased with concentrations of dexamethasone.

To assess whether the cells formed bile canaliculi, a live stain for using CDCFDA (a marker that is excreted into the bile via efflux transporters) was performed. The stains **(****Figs. 12A-12B****)** showed increased bile canaliculi active transport by multidrug resistance-associated protein 2 (MRP2) in the liver chip, as compared to static culture. Thus, increased formation of bile canaliculi in the liver chip was observed, as compared to the static culture.

The inventors have also showed that hepatocytes and hLSECs maintained viable and functional in the liver chip for at least about 2 weeks. Cell viability of hepatocytes and hLSECs over time were determined by measuring lactate dehydrogenase (LDH) levels every day. As discussed in Example 1, when a cell is damaged and the membrane is compromised or becomes non-intact (*e.g.,* with a break in the membrane), LDH is released into the culture media and thus the culture media can be collected and measured for LDH content. **Fig. 13A** shows that both hepatocytes and hLSECs maintained low LDH release levels over a course of at least about 2 weeks, indicating viability and health of the hepatocytes and hLSEC monolayer being maintained over a period of at least 2 weeks. The initial increase in LDH release by the hepatocytes as shown at Day 1 can be due to the stress of the liver chips being connected to pumps and cells being exposed to fluid flow. **Fig. 13B** showed measurement of albumin production over time, indicating that albumin secretion was maintained in the liver chip for at least 2 weeks or longer.

### Reference Example 3. Rat liver-on-a-chip device design and characterization

Similar to development of human liver-on-a-chip devices as described in Examples 1-2, suitable cell sources of rat hepatocytes and rat liver sinusoidal endothelial cells (rLSECs) were first identified. Cryopreserved vials were evaluated for their adhesion ability, their ability to retain their *in vivo* like morphology, and/or their ability to form bile canaliculi network (for biliary clearance determination). Rat hepatocytes from Xenotech Lot: XT1310140 and Biopredic Lot: HEP139031-T and rLSECs from Cell Biologies Lot: R1072 were selected to develop rat liver-on-a-chip devices. See **Figs. 23A-23B** for characterization of hepatic function of rat hepatocytes obtained from XenoTech and Biopredic.

In order to achieve long-term maintenance (*e.g.*, viability and function) of a co-culture model of rat hepatocytes and rLSECs in a device as shown in **Fig. 7B****,** *e.g.,* for at least two weeks or longer, the inventors have identified four optimization parameters, namely (1) substrate surface treatment (*e.g.*, PDMS surface treatment); (2) extracellular matrix (ECM) coating; (3) ECM overlay on top of liver-associated cells such as hepatocytes; and (4) culture medium composition **(****Fig. 14A****).**

***Substrate surface treatment (e.g., PDMS surface treatment):*** When a surface in contact with cells and/or culture medium is hydrophobic, the surface is desired to be treated such that it becomes more hydrophilic. In some embodiments, a hydrophobic surface (*e.g.,* PDMS surface) can be subjected to APTES treatment and/or plasma treatment. APTES (3-Aminopropyl)triethoxysilane is an aminosilane frequently used in the process of silanization, the functionalization of surfaces with alkoxysilane molecules. Plasma oxidation alters the surface chemistry of a hydrophobic surface (*e.g.,* PDMS), adding silanol (SiOH) groups to the surface, and renders the hydrophobic surface (*e.g.,* PDMS) hydrophilic (Lab Chip, 2010, 10, 548-552).

***ECM coating.*** To determine optimum condition for cell attachment, the surface-treated material (*e.g.,* APTES-treated or plasma-treated PDMS) can be coated with an ECM coating of different extracellular matrix molecules at varying concentrations (based on the resulting cell morphology and attachment). For example, the ECM coating can comprise one of the following conditions shown in Table 1.

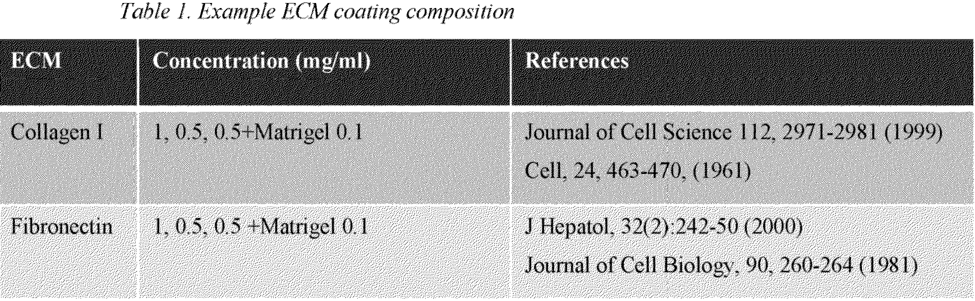

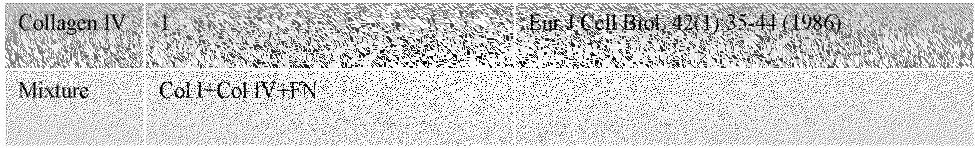

Hepatocytes were then cultured on the ECM coated surface for a period of time (*e.g.,* 5 days) and cell attachment was evaluated. It was determined that a plasma-treated PDMS surface coated with (i) an ECM mixture coating comprising fibronectin (~0.5 mg/mL), collagen IV (~0.4 mg/mL), and collagen I (~0.1 mg/mL); or (ii) an ECM coating of fibronectin at a concentration of about 0.5 mg/mL or higher, provided an optimum condition for hepatocyte attachment.

***ECM overlay:*** Similarly, it was determined that an ECM overlay mixture of Matrigel^{®} and fibronectin provide better cell morphology of hepatocytes than Matrigel^{®} overlay alone. In some embodiments, the ECM overlay mixture can have Matrigel^{®} at a concentration of about 0.25 mg/mL, and fibronectin at a concentration of about 0.02 mg/mL. In these embodiments, the ECM coating can have fibronectin at about 1 mg/mL.

***Culture medium composition:*** To determine optimum culture medium composition for rat hepatocytes, the cells were maintained in various culture medium compositions as shown in Table 2 and cell growth after a certain period of time were evaluated. In the experiment, the cells were adhered on an ECM coating of fibronectin at about 1 mg/mL.

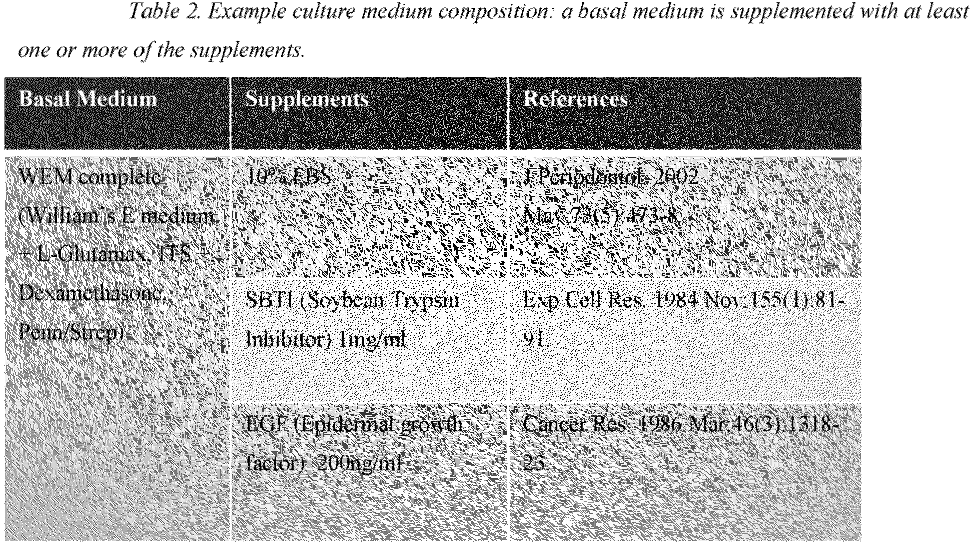

After 7 days of culture, it was determined that WEM complete basal medium supplemented with about 10% FBS provided a more confluent monolayer of hepatocytes and better cell morphology.

**Figs. 15A-15B** are images showing viable co-culture of rat hepatocytes on one side of the membrane facing the top chamber of the device (*e.g.*, as shown in Fig. 7B) and rLSECs on another side of membrane facing the bottom chamber of the device (in WEM plus supplements and 10% FBS). After two weeks, the co-culture remained viable. Prior to culturing hepatocytes, the PDMS surface was plasma-treated, followed by an ECM coating of fibronectin (higher than 0.5 mg/mL) or an ECM coating mixture of fibronectin, collagen IV and collagen I. The ECM coating was further overlaid with a mixture of Matrigel^{®} and fibronectin. The WEM complete basal medium supplemented with about 10% FBS was introduced into the chamber comprising the rat hepatocytes.

The inventors have also showed that rat hepatocytes and rLSECs maintained viable and functional in the rat liver chip for at least about 2 weeks or longer. Cell viability of rat hepatocytes and rLSECs over time were determined by measuring lactate dehydrogenase (LDH) levels over a period of time. As discussed in Example 1, when a cell is damaged and the membrane is compromised or becomes non-intact (*e.g.*, with a break in the membrane), LDH is released into the culture media and thus the culture media can be collected and measured for LDH content. **Fig. 16A** shows that unlike static culture (*e.g.*, plate culture), both rat hepatocytes and rLSECs maintained low LDH release levels over a course of at least about 2 weeks, indicating viability and health of the hepatocytes and rLSEC monolayer being maintained in the rat liver chip over a period of at least 2 weeks. **Fig. 16B** showed measurement of albumin production over time, indicating that albumin secretion in the rat liver chip was maintained for at least 2 weeks or longer, or even increased after two weeks.

Similar to **Fig. 16A****,** **Fig. 17C** showed improved viability of rat hepatocytes and rLSECs in the liver chip for a period of at least 4 weeks or longer than in a static culture, as evidenced by lower LDH release levels measured in the liver chip.

F-actin staining of the 4-week cultures shows cuboidal morphology of rat hepatocytes and pericanalicular distribution in rat liver chips but cell deterioration and detachment in static cultures (*e.g.,* plates) (data not shown). In addition, multidrug resistance-associated protein 2 (MRP2) staining correctly expressed at the canalicular wall of rat hepatocytes in liver chips whereas static cultures (*e.g.*, plates) show only background staining of dead cells (data not shown).

Next, to determine effects of serum on mRNA expression and CYP450 enzyme activity, rat hepatocytes were cultured in plate cultures with different concentrations of serum. **Fig. 18A** shows that serum inhibited mRNA expression of cyp1a1, but no significant effect on cyp2b1 and cyp3a1. **Fig. 18B** shows that the activity of CYP3A and CYP2B are serum-independent, as different concentrations of serum showed no effect on CYP3A and CYP2B activity.

### Comparison of the rat liver chips described herein to static cultures and/or in vivo models

One of the limitations of the static cultures (*e.g.*, plates) of hepatocytes is that CYP mRNA expression (*e.g.*, cyp2b1, cyp3a1, cyp1a1) in rat hepatocytes declined over time **(****Figs. 19A-19C****).** In addition, CYP3A1/A2 enzyme activity in rat hepatocytes also declined over time in static cultures (*e.g.,* conventional sandwich plate culture) **(****Fig. 20A****,** **Fig. 20B****).** In contrast, rat liver chip shows improved CYP3A4 activity over static culture and the CYP3A4 activity was maintained in the liver chip for at least two weeks or longer **(****Fig. 20B****).**

In addition, the inventors measured one of the major functions of the liver - albumin secretion and other markers of liver injury such as liver transaminases (*e.g.*, ALT and AST) in the rat liver chips. A low level of albumin is indicative of liver injury. **Fig. 21A** shows high levels of albumin secretion in the rat liver chip, whereas the static plate shows low levels of albumin, indicating that the rat liver chips had improved hepatocyte viability over the static cultures. In addition, the rat liver chips produced at least 5 times higher albumin than in a co-cultured liver bioreactor as described in Tsang et al. FASEB journal (2007) 21: 3790-3801. The Tsang reference shows that the co-cultured liver bioreactor produced around 8 µg of albumin per a million cells at Day 12 of culture, while the rat liver chips, as shown in **Fig. 21A****,** produced about 40 µg of albumin per a million cells by Day 14 of culture.

Levels of alanine transaminase (ALT) and aspartate transaminase (AST) - indicators of severe liver injury- were also measured over a period of 22 days in the rat liver chip and the levels (**Figs. 21B-21C****,** and **Table 3**) were within the normal range of ALT and AST in rat and human *in vivo* as shown in Table 3 below. Thus, the rat liver chips can be used to evaluate hepatotoxic compounds by measuring the levels of albumin, ALT and/or AST.

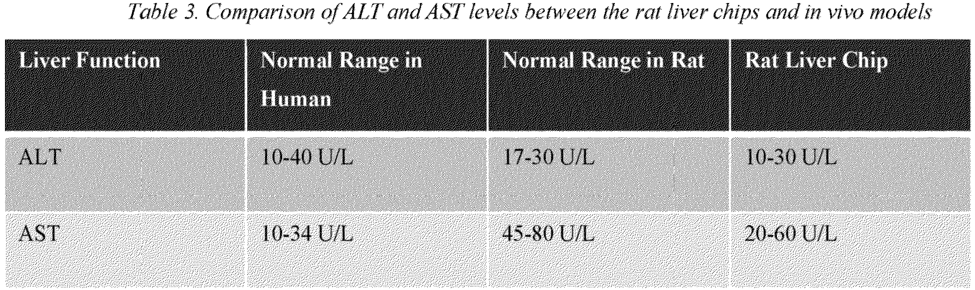

Another specific function of the liver is urea synthesis. If the urea level is high, this indicates healthy liver cells, whereas decreased levels indicate unhealthy liver cells. **Fig. 22** shows that urea levels were maintained in the rat liver chips for at least 2 weeks and the levels were higher than static cultures (*e.g.,* plates) and other existing *in vitro* liver models (*e.g.*, Hepregen).

### Example 4. Validation of Liver-on-Chip Model Species Differences and Predicting Toxicity in Vivo

To validate use of the liver chips to model species differences as well as to predict drug toxicity *in vivo,* aflatoxin B1 was selected as a validation compound. Aflatoxin B1 is a known liver toxicant derived from fungi. It is known to cause acute and/or chronic liver toxicity and some toxicity mechanism is metabolite dependent (*e.g.*, CYP1A2 and CYP3A4 in humans). In addition, aflatoxin B1 is known to have interspecies differences in toxicity with low IC50 in dogs. Based on static plate cultures of hepatocytes from multiple species, it was determined that the IC50 values of cell viability for dogs were about 10 nM (most potency), about 22 mM for rats, and about 520 nM for humans (least potency).

**Figs. 24A-24C** are dose response curves generated using the liver cells of different species: dog **(****Fig. 24A****),** rat **(****Fig. 24B****),** and human **(****Fig. 24C****).** The figures show similar trends of potency of aflatoxin B1 on different species as determined in the static plate cultures noted above. ATP was used as a viability end point.

### Example 5. Dog liver-on-a-chip device design and characterization

Similar to development of human and rat liver-on-a-chip devices as described in Examples 1-3, suitable cell sources of dog hepatocytes and dog liver sinusoidal endothelial cells (dLSECs) were first identified. Cryopreserved vials were evaluated for their adhesion ability, their ability to retain their *in vivo* like morphology, and/or their ability to form bile canaliculi network (for biliary clearance determination).

Like in Example 3, in order to achieve long-term maintenance (*e.g.*, viability and function) of a co-culture model of dog hepatocytes and dLSECs in a device as shown in **Fig. 7B****,** *e.g.,* for at least two weeks or longer, the inventors have optimized an ECM coating of the membrane, on which dog hepatocytes adhered. **Figs. 25A-25D** are images showing cell morphology of dog hepatocytes growing on an ECM coating of collagen I (e.g., at concentration of about 100 µg/ml) or an ECM mixture coating comprising fibronectin (e.g., at concentration of about 500 µg/ml), collagen IV (e.g., at concentration of about 400 µg/ml) and laminin (e.g., at concentration of about 100 µg/ml). It was determined that an ECM coating of collagen I provided improved viability and growth of dog hepatocytes.

In some embodiments, dog hepatocytes had an ECM overlay comprising Matrigel^{™} (e.g., at a concentration of about 250 µg/ml). In some embodiments, dog hepatocyte cultures can use the same culture medium as used in human hepatocyte cultures.

The inventors have also showed that dog hepatocytes and dLSECs maintained viable and functional in the dog liver chip for at least about 2 weeks or longer **(****Figs. 26A-26C****).** Cell viability of dog hepatocytes and dLSECs over time were determined by measuring lactate dehydrogenase (LDH) levels over a period of time. As discussed in Example 1, when a cell is damaged and the membrane is compromised or becomes non-intact (*e.g.*, with a break in the membrane), LDH is released into the culture media and thus the culture media can be collected and measured for LDH content. **Fig. 26B** shows that dog hepatocytes maintained low LDH release levels over a course of at least about 2 weeks, indicating viability and health of the hepatocytes monolayer being maintained in the dog liver chip over a period of at least 2 weeks. **Fig. 26C** showed measurement of albumin production over time, indicating that the dog liver chip remained viable and maintained a high level of albumin secretion for at least about 2 weeks or longer.

F-actin staining of the 2-week cultures shows cuboidal morphology of dog hepatocytes and pericanalicular distribution in dog liver chips **(****Figs. 27A-27B****).**

### Example 6. Universal endothelial cell culture medium for long-term culture

The inventors have developed a universal endothelial cell culture medium for long-term culture and this allows endothelial and epithelial cells to maintain functionality and/or viability for at least about 2 weeks or longer (including, *e.g.,* at least about 4 weeks or longer) in cell culture devices (*e.g*., but not limited to Transwell and microfluidic chip systems).

In some embodiments, the composition of the universal endothelial cell culture medium comprises, essentially consists of, or consist of the following:
- Basal medium : DMEM/F12
- Supplement: hEGF, Hydrocortisone, VEGF, hFGF-B, R3-IGF-1, Ascorbic Acid, Heparin, 0.5% of fetal bovine serum (FBS), optionally 1% of Penicillin Streptomycin (PS)
- Phenol red: optional (sometimes phenol red can interfere with the sensitivity of assays)

The universal endothelial cell culture medium was tested in human umbilical vein endothelial cells (HUVEC) and human primary glomerular microvascular endothelial cell for the co-culture model with kidney epithelial cell culture. The universal endothelial cell culture medium was also tested in HUVEC with Caco-2 cells (Gut system), human primary liver sinusoidal endothelial cells (hLSECs) with human primary hepatocyte (Liver system), and HUVEC with lung epithelial cell (lung system). In all these systems, the co-cultures maintained their functionality and viability for at least about 2 weeks or longer, including, e.g., at least about 1 month or longer. **Figs. 28A-28B** are images showing endothelial cells under existing commercial culture medium **(****Fig. 28A****)** and the novel endothelial cell culture medium described above **(****Fig. 28B****)** after 2 weeks of culture.

### Example 7

Conversin of nicotine to continine in liver cells is a measure of liver cell function and is catalyzed by CYP2A6. When grown in the chips and/or devices described herein, liver cells express CYP2A6 (Fig. 31). It is demonstrated herein that cotinine production is much higher under flow conditions (Fig. 32). Without wishing to be limited by theory, it is contemplated that this could be due to cells being exposed to more nicotine molecules during flow rather than a fixed number in static conditions, or more mixing of the nicotine solution under flow than static conditions. Additionally, chips treated basally had over twice the concentration of nicotine delivered, but still produced less cotinine than chips treated apically & basally (Fig. 33). Without wishing to be bound by theory, it is contemplated here in that this could indicate that only about half of the nicotine interacts with hepatocytes. Chips given basal media with no FGF had practically identical cotinine production as chips given complete basal media (Fig. 34). As depicted in Fig. 35, the presence of media flow permits higher cotinine production as compared to static conditions.

## Claims

1. A method of culturing cells, comprising: a) providing a microfluidic device comprising a membrane, said membrane comprising a first surface and a second surface; b) seeding viable human hepatocytes on said first surface and viable human liver sinusoidal endothelial cells on said second surface; and c) culturing said seeded cells under flow conditions such that said cells remain viable for at least 14 days,
wherein, prior to step b), said first surface of said membrane is coated with a layer of fibronectin, collagen I, and collagen IV.

2. The method of Claim 1, wherein said human hepatocytes are primary human hepatocytes that were previously cryopreserved.

3. The method of Claim 1, further comprising d) assessing the level of activity of one or more cellular enzymes.

4. The method of Claim 1, further comprising d) assessing the level of one or more cellular proteins.

5. The method of Claim 3 or 4, wherein, prior to step d), said seeded viable human hepatocytes are exposed to an agent.

6. The method of Claim 5, wherein said agent is a drug candidate.

7. The method of claim 1, wherein the microfluidic device further comprises cholangiocytes.

8. The method of claim 1, wherein the hepatocytes display at least one of the following characteristics:
cuboidal morphology;
formation of a bile canaliculus network;
albumin secretion over a period of time; and
at least a 2-fold increase in activity of CYP450 drug metabolizing enzyme in the presence of an agent that induces CYP450 drug metabolizing enzyme.

9. The method of claim 1, wherein said coating consists of a mixture of fibronectin, collagen I, and collagen IV.

## Patentansprüche

1. Verfahren zum Kultivieren von Zellen, umfassend: a) Bereitstellen einer mikrofluidischen Vorrichtung, die eine Membran umfasst, wobei die Membran eine erste Oberfläche und eine zweite Oberfläche umfasst; b) Aussäen lebensfähiger menschlicher Hepatozyten auf der ersten Oberfläche und lebensfähiger menschlicher Lebersinusoidendothelzellen auf der zweiten Oberfläche; und c) Kultivieren der ausgesäten Zellen unter Strömungsbedingungen, sodass die Zellen für mindestens 14 Tage lebensfähig bleiben, wobei vor Schritt b) die erste Oberfläche der Membran mit einer Schicht aus Fibronektin, Kollagen I und Kollagen IV beschichtet wird.

2. Verfahren nach Anspruch 1, wobei die menschlichen Hepatozyten primäre menschliche Hepatozyten sind, die zuvor kryokonserviert wurden.

3. Verfahren nach Anspruch 1, ferner umfassend d) die Bestimmung des Aktivitätsniveaus von einem oder mehreren zellulären Enzymen.

4. Verfahren nach Anspruch 1, ferner umfassend d) die Bestimmung des Gehalts eines oder mehrerer zellulärer Proteine.

5. Verfahren nach Anspruch 3 oder 4, wobei vor dem Schritt d) die ausgesäten lebensfähigen menschlichen Hepatozyten einem Agens ausgesetzt werden.

6. Verfahren nach Anspruch 5, wobei der Wirkstoff ein Arzneimittelkandidat ist.

7. Verfahren nach Anspruch 1, wobei die mikrofluidische Vorrichtung ferner Cholangiozyten umfasst.

8. Verfahren nach Anspruch 1, wobei die Hepatozyten mindestens eine der folgenden Eigenschaften aufweisen:
quaderförmige Morphologie;
Bildung eines Gallenkanalikulus-Netzwerks;
Albuminsekretion über einen bestimmten Zeitraum; und
mindestens eine 2-fache Erhöhung der Aktivität des CYP450-Arzneimittel metabolisierenden Enzyms in Gegenwart eines Agens, das das CYP450-Arzneimittel metabolisierende Enzym induziert.

9. Verfahren nach Anspruch 1, wobei die Beschichtung aus einer Mischung aus Fibronectin, Kollagen I und Kollagen IV besteht.

## Revendications

1. Procédé de culture de cellules, comprenant : a) la fourniture d'un dispositif microfluidique comprenant une membrane, ladite membrane comprenant une première surface et une seconde surface ; b) l'ensemencement d'hépatocytes humains viables sur ladite première surface et de cellules endothéliales sinusoïdales hépatiques humaines viables sur ladite seconde surface ; et c) la culture desdites cellules ensemencées dans des conditions d'écoulement telles que lesdites cellules restent viables pendant au moins 14 jours,
dans lequel, avant l'étape b), ladite première surface de ladite membrane est revêtue d'une couche de fibronectine, de collagène I et de collagène IV.

2. Procédé selon la revendication 1, dans lequel lesdits hépatocytes humains sont des hépatocytes humains primaires qui ont été préalablement cryoconservés.

3. Procédé selon la revendication 1, comprenant également d) l'évaluation du niveau d'activité d'une ou plusieurs enzymes cellulaires.

4. Procédé selon la revendication 1, comprenant également d) l'évaluation du niveau d'une ou plusieurs protéines cellulaires.

5. Procédé selon la revendication 3 ou 4, dans lequel, avant l'étape d), lesdits hépatocytes humains viables ensemencés sont exposés à un agent.

6. Procédé selon la revendication 5, dans lequel ledit agent est un candidat médicament.

7. Procédé selon la revendication 1, dans lequel le dispositif microfluidique comprend également des cholangiocytes.

8. Procédé selon la revendication 1, dans lequel les hépatocytes présentent au moins l'une des caractéristiques suivantes :
morphologie cuboïde ;
formation d'un réseau de canalicules biliaires ;
sécrétion d'albumine sur une période donnée ; et
augmentation d'au moins 2 fois de l'activité de l'enzyme CYP450 qui métabolise les médicaments en présence d'un agent inducteur de l'enzyme CYP450 qui métabolise les médicaments.

9. Procédé selon la revendication 1, dans lequel ledit revêtement est constitué d'un mélange de fibronectine, de collagène I et de collagène IV.
